(19) Europäisches Patentamt
European Patent Office
Office européen des brevets

(11) Numéro de publication: **0 055 170**
**B1**

(12) # FASCICULE DE BREVET EUROPEEN

(45) Date de publication du fascicule du brevet: **24.10.84**

(21) Numéro de dépôt: **81401994.9**

(22) Date de dépôt: **14.12.81**

(51) Int. Cl.³: **C 07 J 21/00**, C 07 J 1/00, C 07 J 41/00, C 07 J 51/00, A 61 K 31/565, A 61 K 31/585

(54) **Nouveaux dérivés stéroides 3-céto delta 4 ou delta 1-4 substitués en position 7, leur préparation, leur application comme médicaments et les compositions les renfermant.**

(30) Priorité: **23.12.80 FR 8027272**

(43) Date de publication de la demande:
**30.06.82 Bulletin 82/26**

(45) Mention de la délivrance du brevet:
**24.10.84 Bulletin 84/43**

(84) Etats contractants désignés:
**AT BE CH DE FR GB IT LI LU NL SE**

(56) Documents cités:
**EP - A - 0 018 245**
**FR - A - 2 150 852**
**FR - A - 2 255 914**

**JOURNAL OF ORGANIC CHEMISTRY, vol. 26, no. 9, 12 septembre 1961 WASHINGTON DC (US) N.W. ATWATER et al.: "Steroid aldosterone antagonists" pages 3077-3083**
**JOURNAL OF MEDICINAL CHEMISTRY, vol. 18, no. 8, août 1975 WASHINGTON DC (US) RICHARD M. WEIER et al.: "7 Carboalkoxy Steroidal Spirolactones as Aldosterone Antagonists" pages 817-821**

***Le dossier contient des informations techniques présentées postérieurement au dépôt de la demande et ne figurant pas dans le présent fascicule.***

(73) Titulaire: **ROUSSEL-UCLAF, 35, boulevard des Invalides, F-75007 Paris (FR)**

(72) Inventeur: **Nedelec, Lucien, 45, boulevard de l'Ouest, F-93340 Le Raincy (FR)**
Inventeur: **Torelli, Vesperto, 5, rue de la Convention, F-94700 Maisons Alfort (FR)**
Inventeur: **Philibert, Daniel, 16, rue Chevalier, F-94210 La Varenne Saint Hilaire (FR)**

(74) Mandataire: **Bourgouin, André et al, ROUSSEL-UCLAF 111, route de Noisy Boîte postale no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne de nouveaux dérivés stéroïdes 3-céto $\Delta_4$ ou $\Delta_{1\text{-}4}$ substitués en position 7, leur procédé de préparation, leur application comme médicaments et les compositions les renfermant.

Des γ-lactones d'acides 7-halométhyl 17-hydroxy 3-oxo 17α-pregn-4-ène 21-carboxyliques sont décrites dans le brevet français FR-A N° 2255914. Cependant, ces produits sont présentés comme ayant une activité antiprogestative et étant dépourvus de l'activité antiminéralocorticoïde.

Dans la demande de brevet européen EP-A N° 0018245 sont décrits des stéroïdes 7-alkylés saturés en position 1(2). Dans la publication du «Journal of Med. Chem.», 18 (8), 1975, pp. 817-819, sont décrits des produits comportant en position 7 un radical carboxy, alkoxycarbonyle ou alkylthiocarbonyle et étant saturés ou non en postion 1(2). Il en est de même du brevet français FR-A N° 2150852.

Enfin, dans la publication du «Journal of Org. Chem.», 26 (9), 1961, pp. 3077-3083, sont décrits des produits comportant une γ-lactone en position 17.

L'invention a pour objet les produits répondant à la formule générale (I'):

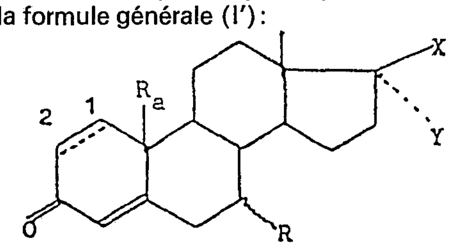

(I')

dans laquelle R représente soit un radical $R_1$, $R_1$ étant un radical alkyle, alkényle ou alkynyle ayant au plus 8 atomes de carbone, soit un radical $R_2$, $R_2$ étant un radical $-(CH_2)_n-R_3$ dans lequel n représente un entier de 1 à 8 et $R_3$ représente un radical hydroxyle libre ou protégé, un radical amino, amino protégé, mono- ou dialkylamino, un radical carboxy libre, estérifié ou salifié ou un atome d'halogène, X et Y représentent ensemble le groupement 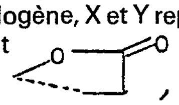 ,

ou bien X représente un radical hydroxyle et Y représente le radical $-CH_2-CH_2-CO_2M$, dans lequel M représente un atome d'hydrogène, un atome de métal alcalin ou un radical ammonium, Ra représente un atome d'hydrogène ou un radical méthyle,

le pointillé en position 1(2) indique la présence éventuelle d'une seconde liaison entre les carbones 1 et 2, et

le trait ondulé signifie que le substituant R peut se trouver dans l'une des deux positions possibles α ou β,

étant entendu que R ne peut pas représenter $R_1$ ou $R_2$ dans lequel n est égal à 1 et $R_3$ représente un atome d'halogène, lorsque la liaison 1(2) est une simple liaison, et que Ra représente un radical méthyle, que $R_3$ ne peut pas représentenr un radical carboxy libre lorsque Y représente le radical $-CH_2-CH_2-CO_2M$, dans lequel M représente un atome de métal alcalin ou un radical ammonium, et que $R_3$ ne peut pas représenter un radical carboxy salifié lorsque Y représente le radical $-CH_2-CH_2-CO_2-H$.

L'invention a notamment pour objet, parmi les produits de formule (I'), ceux répondant à la formule (I):

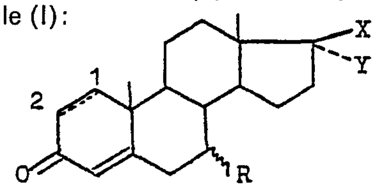

(I)

dans laquelle R, X, Y, le trait pointillé et le trait ondulé ont la signification indiquée précédemment, étant entendu que R ne peut pas représenter $R_1$ ou $R_2$ dans lequel n est égal à 1 et $R_3$ représente un atome d'halogène, lorsque la liaison 1(2) est une simple liaison, que $R_3$ ne peut pas représenter un radical carboxy libre lorsque Y représente le radical $-CH_2-CH_2-CO_2M$ dans lequel M représente un atome de métal alcalin ou un radical ammonium, et que $R_3$ ne peut pas représenter un radical carboxy salifié lorsque Y représente le radical $-CH_2-CH_2-CO_2H$.

Parmi les valeurs de R, on peut citer les radicaux méthyle, éthyle, propyle, isopropyle, butyle, tert.-butyle, isobutyle, sec.-butyle, pentyle, isopentyle, sec.-pentyle, tert.-pentyle, néopentyle, hexyle, heptyle ou octyle linéaires ou ramifiés.

On peut également citer les radicaux vinyle, allyle, isopropényle, butényle, isobutényle, penténylé, hexényle, hepténylé, ou octényle linéaires ou ramifiés.

On peut également citer les radicaux éthynyle, propargyle ou butynyle.

Lorsque R représente $R_2$, c'est-à-dire le radical $-(CH_2)_nR_3$, on peut citer parmi les valeurs de $R_3$ les valeurs tétrahydropyrannyloxy, t.-butyloxy ou benzyloxy.

On peut également citer les valeurs mono- ou dialkylamino dans lesquelles les radicaux alkyles ont de préférence 1 à 3 atomes de carbone, par exemple méthylamino, éthylamino, n-propylamino, diméthylamino, diéthylamino, di-n-propylamino, diisopropylamino, méthyléthylamino.

Lorsque $R_3$ représente un radical amino protégé, ce radical est de préférence le radical tritylamino, benzylamino, t.-butylamino ou t.-pentylamino.

Le radical carboxy que peut représenter $R_3$ peut être estérifié, par exemple, par l'un des radicaux suivants: un radical alkyle ayant de 1 à 4 atomes de carbone, à savoir méthyle, éthyle, propyle, isopropyle, butyle, isobutyle, sec.-butyle ou tert.-butyle; un radical aralkyle tel qu'un radical benzyle; un radical haloalkyle tel que 2-chloroéthyle, 2-bromoéthyle, 2-iodoéthyle ou βββ-trichloroéthyle.

Le radical carboxy peut être salifié, par exemple, par un équivalent d'un métal alcalin, alcalino-terreux ou d'une base organique aminée. On peut citer, par exemple, les sels de sodium, de potassium, de lithium, de calcium, de magnésium ou d'ammonium. Parmi les bases aminées, on peut citer, par exemple, la méthylamine, la propylamine, la triméthylamine, la N,N-diméthyléthanolamine, le tris(hydroxyméthyl)aminométhane.

Parmi les atomes d'halogènes que peut représenter $R_3$, on peut citer notamment le chlore ou le brome.

L'atome de métal alcalin que peut représenter M est de préférence le sodium, le potassium ou le lithium.

Parmi les composés de l'invention, on peut citer les produits de formule (I'') correspondant aux produits de formule (I'), dans laquelle Ra représente un radical méthyle, R représente $R_2$ et le trait pointillé en position 1(2) une simple liaison, étant entendu que n ne représente pas le nombre 1 lorsque $R_3$ représente un atome d'halogène, ou ceux dans lesquels Ra représente un atome d'hydrogène, R est défini comme dans la formule (I') et le trait pointillé en position 1(2) représente une simple liaison.

On peut également citer les produits de formule (I''') correspondant aux produits de formule (I'), dans laquelle le trait pointillé en position 1(2) indique la présence d'une double liaison.

On peut citer de même les produits de formule (I') dans laquelle X et Y représentent le groupement:

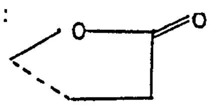

ainsi que les produits de formule (I') dans laquelle X représente un radical hydroxyle et Y représente un radical $-CH_2-CH_2-CO_2K$.

Bien que le radical R puisse se trouver dans l'une ou l'autre des deux positions possibles, on préfère les produits de formule (I') dans laquelle le radical R est dans la position 7α.

Parmi les produits préférés de l'invention, on peut citer les produits de formule (I') dans laquelle R représente un radical propyle, butyle, isobutyle ou butényle, étant entendu que Ra ne représente pas un radical méthyle lorsque la liaison 1(2) est une simple liaison, ainsi que les produits de ladite formule (I') dans laquelle R représente un radical chloropropyle, hydroxypropyle, carboxyéthyle, méthoxycarbonyléthyle.

Les produits décrits dans les exemples et, plus particulièrement, ceux qui comportent une double liaison en 1(2) constituent les produits préférés de l'invention.

Il en est spécialement ainsi de la γ-lactone de l'acide 17β-hydroxy 7α-propyl 3-oxo 17α-pregna 1,4-diène 21-carboxylique et du 17β-hydroxy 3-oxo 7α-propyl 17α-pregna 1,4-diène 21-carboxylate de potassium.

L'invention a également pour objet un procédé de préparation des produits de formule ($I''_1$), correspondant aux produits de formule (I'') telle que définie ci-dessus dans laquelle Ra représente un radical méthyle, caractérisé en ce que l'on soumet un produit de formule ($II_1$):

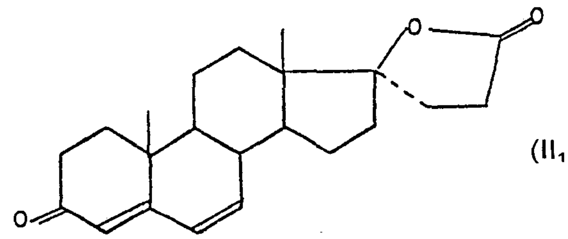

($II_1$)

soit à l'action d'un produit de formule ($III_1$):

$$R_4-(CH_2)_n-MgX' \qquad (III_1)$$

en présence d'un sel de cuivre, formule ($III_1$) dans laquelle X' représente un atome d'halogène, $R_4$ représente un radical hydroxyle protégé, un radical mono- ou dialkylamino ou un radical amino protégé et n a la signification indiquée ci-dessus, soit à l'action d'un produit de formule ($III'_1$):

$$[R_4-(CH_2)_n]_2CuLi \qquad (III'_1)$$

dans laquelle $R_4$ et n conservent la même signification, pour obtenir, après action d'un acide, un produit de formule ($I''_A$):

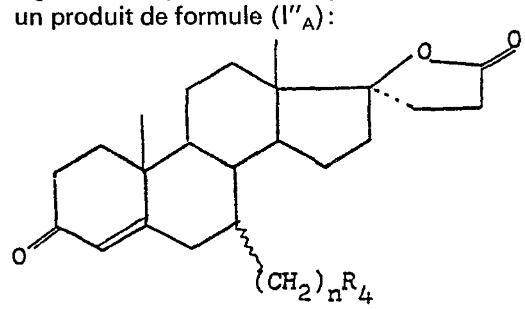

($I''_A$)

sous la forme, éventuellement, d'un mélange d'isomères 7α et 7β, mélange que, si désiré, l'on sépare, et, si désiré, soumet chaque isomère ou le mélange des produits dans lesquels $R_4$ représente un radical hydroxyle protégé ou un radical amino protégé, à l'action d'un agent d'hydrolyse, pour obtenir un produit de formule ($I''_B$):

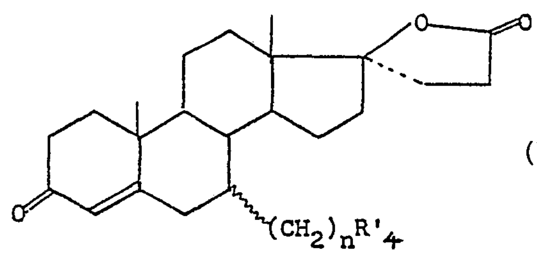

($I''_B$)

dans laquelle $R'_4$ représente un radical hydroxyle ou amino, et produit de formule ($I''_B$) dans laquelle $R'_4$ représente un radical hydroxyle que l'on traite, le cas échéant,

soit, lorsque n est un entier supérieur à 1, par un réactif d'oxydation, pour obtenir un produit de formule ($I''_C$):

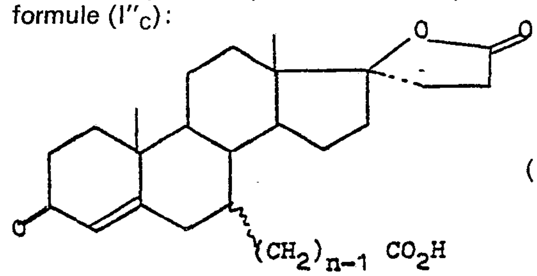

($I''_C$)

produit que, le cas échéant, l'on estérifie ou salifie selon les méthodes usuelles,

soit, à l'exception du cas où n est égal à 1, par un réactif d'halogénation, pour obtenir un produit de formule ($I''_D$):

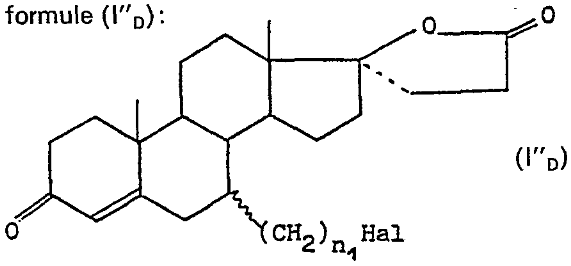

($I''_D$)

dans laquelle $n_1$ représente un entier de 2 à 8 et Hal représente un atome d'halogène et que, si désiré, l'on soumet les produits de formule ($I''_A$), ($I''_B$), ($I''_C$) et ($I''_D$) sous forme d'isomères 7α ou 7β ou de leur mélange, à l'action d'un hydroxyde ou de l'ammoniaque, pour obtenir les composés de formule ($I''_T$):

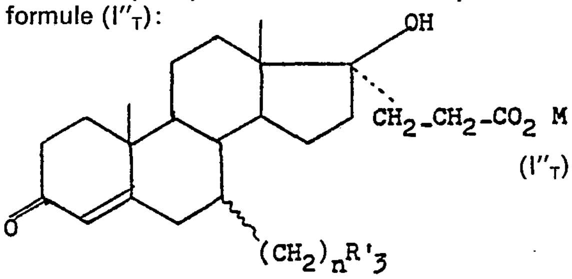

($I''_T$)

dans laquelle $R'_3$ a la signification de $R_3$ indiquée ci-dessus à l'exception de la valeur carboxy libre et M' représente un atome de métal alcalin ou un radical ammonium, sous forme d'isomères 7α ou tβ ou de leur mélange, que l'on sépare, si désiré, en chacun de ses isomères, puis soumet, si désiré, soit chacun des isomères, soit leur mélange, à l'action d'un agent acide, pour obtenir un composé de formule ($I''_U$):

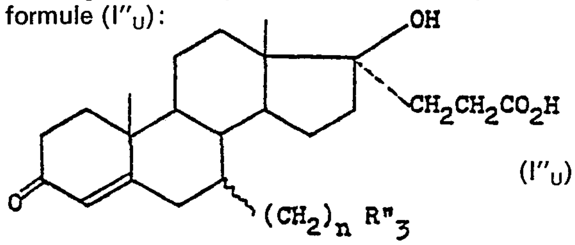

($I''_U$)

dans laquelle $R''_3$ a la signification de $R_3$ indiquée ci-dessus, à l'exception de la valeur carboxy salifié.

L'invention a également pour objet un procédé de préparation des produits de formule ($I''_2$), correspondant aux produits de formule (I'') telle que définie précédemment, dans laquelle Ra représente un atome d'hydrogène, caractérisé en ce que l'on soumet un produit de formule ($II_2$):

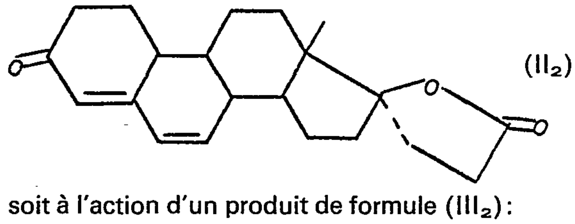

($II_2$)

soit à l'action d'un produit de formule ($III_2$):

$R_5-MgX'$ ($III_2$)

en présence d'un sel de cuivre, formule ($III_2$) dans laquelle $R_5$ représente $R_1$ qui est défini comme ci-dessus ou le radical $R_4-(CH_2)_n-$ défini comme ci-dessus, et X' représente un atome d'halogène, soit à l'action d'un produit de formule ($III'_2$):

$(R_5)_2CuLi$ ($III'_2$)

dans laquelle $R_5$ est défini comme ci-dessus, pour obtenir, après l'action d'un acide, un produit de formule ($I''_{A1}$):

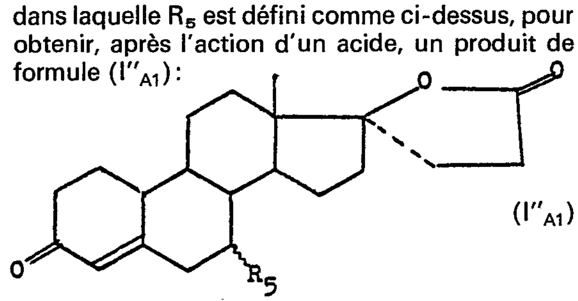

($I''_{A1}$)

sous la forme, éventuellement, d'un mélange d'isomères 7α et 7β, mélange que, si désiré, l'on sépare, et, si désiré, soumet chaque isomère ou le mélange des produits dans lesquels $R_5$ représente un radical $-(CH_2)_n-R_4$, dans lequel $R_4$ représente un radical hydroxyle protégé, ou un radical amino protégé, à l'action d'un agent d'hydrolyse pour obtenir un produit de formule ($I''_{B1}$):

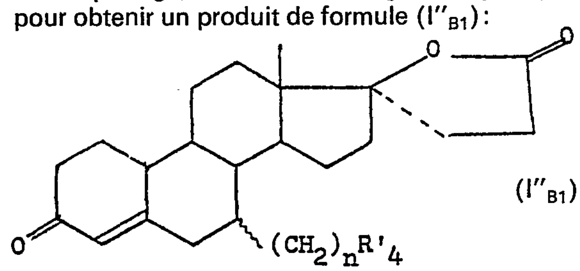

($I''_{B1}$)

dans laquelle $R'_4$ représente un radical hydroxyle ou amino, et produit de formule ($I''_{B1}$) dans laquelle $R'_4$ représente un radical hydroxyle que l'on traite, le cas échéant,

soit, lorsque n est un entier supérieur à 1, par réactif d'oxydation, pour obtenir un produit de formule ($I''_{C1}$):

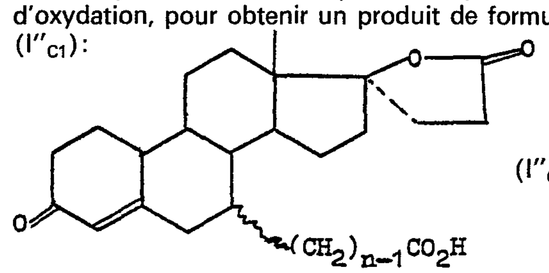

($I''_{C1}$)

produit que, le cas échéant, l'on estérifie ou salifie selon les méthodes usuelles,

soit par un réactif d'halogénation, pour obtenir un produit de formule ($I''_{D1}$):

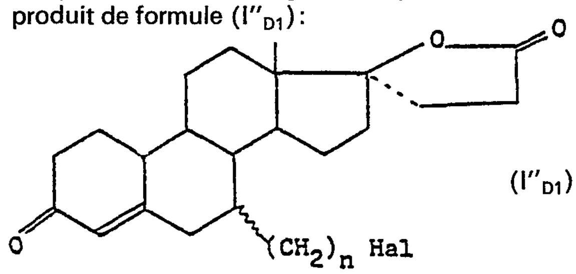

($I''_{D1}$)

dans laquelle Hal représente un atome d'halogène et que, si désiré, l'on soumet les produits de formules ($I''_{A1}$), ($I''_{B1}$), ($I''_{C1}$) et ($I''_{D1}$), sous forme d'isomères 7α ou 7β ou de leur mélange, à l'action

d'un hydroxyde alcalin ou de l'ammoniaque, pour obtenir les composés de formule ($I''_{T1}$) :

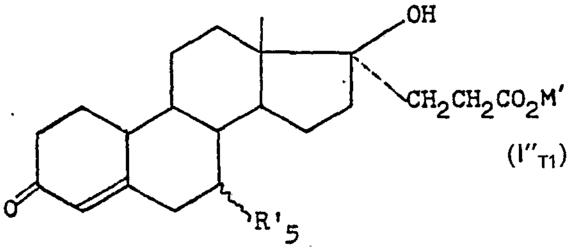

($I''_{T1}$)

dans laquelle $R'_5$ représente $R_1$ qui est défini comme ci-dessus, ou le radical $-(CH_2)_n-R'_3$, dans lequel $R'_3$ a la signification de $R_3$ indiquée ci-dessus, à l'exception de la valeur carboxy libre et M' représente un atome de métal alcalin ou un radical ammonium, sous forme d'isomères 7α ou 7β ou de leur mélange, que l'on sépare, si désiré, en chacun de ses isomères, puis soumet, si désiré, soit chacun des isomères, soit leur mélange, à l'action d'un agent acide, pour obtenir un composé de formule ($I''_{U1}$) :

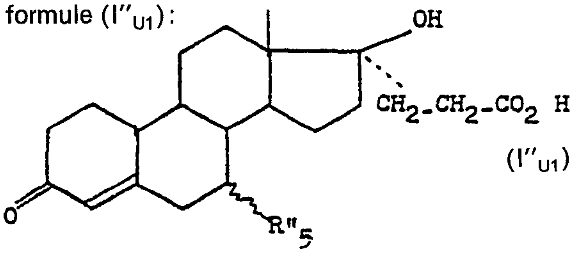

($I''_{U1}$)

dans laquelle $R''_5$ représente $R_1$ qui est défini comme ci-dessus, ou le radical $-(CH_2)_n-R''_3$, dans lequel $R''_3$ a la signification de $R_3$ indiquée ci-dessus, à l'exception de la valeur carboxy salifié.

Dans un mode de réalisation préféré du procédé de l'invention, on utilise un produit de formule $R_4(CH_2)_nMgX'$ ou $R_5MgX'$ dans laquelle X' représente un atome de chlore, de brome ou d'iode.

Le sel de cuivre que l'on utilise est un sel cuivrique, tel que le chlorure, le bromure ou l'acétate cuivrique, ou un sel cuivreux, tel que le chlorure, le bromure ou l'iodure cuivreux.

L'acide que l'on utilise pour obtenir le produit de formule ($I''_A$) ou ($I''_{A1}$) est l'acide chlorhydrique, nitrique ou sulfurique.

La séparation éventuelle des différents isomères est réalisée par chromatographie ou par cristallisation fractionnée.

L'hydrolyse permettant de transformer les produits de formule ($I''_A$) ou ($I''_{A1}$) en produits de formule ($I''_B$) ou ($I''_{B1}$) est de préférence une hydrolyse acide. On utilise alors préférentiellement l'acide chlorhydrique, paratoluènesulfonique ou trifluoroacétique.

L'oxydation éventuelle des produits de formule ($I''_B$) ou ($I''_{B1}$) en produits de formule ($I''_C$) ou ($I''_{C1}$) est effectuée préférentiellement à l'aide d'oxyde chromique. On utilise alors, par exemple, le réactif de Heilbron (oxyde chromique et acide sulfurique en solution dans l'eau), mais on peut également utiliser les réactifs chromiques en général.

L'estérification éventuelle des acides de formule ($I''_C$) ou ($I''_{C1}$) est réalisée dans les conditions usuelles. On peut par exemple utiliser un alcool en présence d'un acide tel que l'acide paratoluènesulfonique ou un diazoalcane.

La salification éventuelle des acides de formule ($I''_C$) ou ($I''_{C1}$) est réalisée dans des conditions usuelles, par exemple par action d'une base organique ou minérale ou d'un sel d'acide organique ou minéral.

La préparation des produits de formule ($I''_D$) ou ($I''_{D1}$) peut être effectuée par action d'un acide halohydrique en présence, par exemple, d'acide sulfurique ou à sec. On peut également utiliser un mélange de chlorure de zinc et d'acide chlorhydrique ou le chlorure de thionyle pour préparer le dérivé chloré. On préfère cependant l'action de la triphénylphosphine en présence de tétrachlorure de carbone selon le procédé décrit dans le «Canadian Journal of Chemistry», vol. 76 (1968), p. 86.

L'hydroxyde alcalin à l'action duquel on soumet les produits de formules ($I''_A$), ($I''_B$), ($I''_C$), ($I''_D$), ($I''_{A1}$), ($I''_{B1}$), ($I''_{C1}$) et ($I''_{D1}$) est de préférence la soude ou la potasse.

L'acide auquel on soumet éventuellement les composés de formule ($I''_T$) ou ($I''_{T1}$) est l'acide chlorhydrique, sulfurique, nitrique ou acétique.

L'invention a également pour objet un procédé de préparation des produits de formule (I''') telle que définie ci-dessus, caractérisé en ce que l'on soumet un composé de formule (IV) :

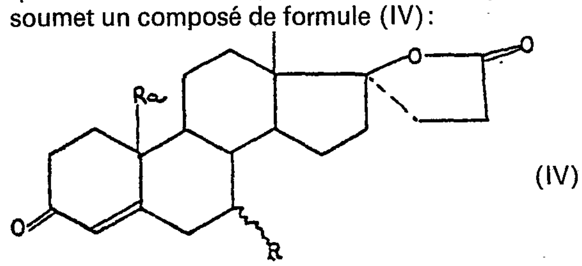

(IV)

dans laquelle R représente soit un radical $R_1$, $R_1$ étant un radical alkyle, alkényle ou alkynyle ayant au plus 8 atomes de carbone, soit un radical $R_2$, $R_2$ étant un radical $-(CH_2)_n-R_3$ dans lequel n représente un entier de 1 à 8 et $R_3$ représente un radical hydroxyle libre ou protégé, un radical amino, amino protégé, mono- ou dialkylamino, un radical carboxy libre, estérifié ou salifié ou un atome d'halogène et Ra a la significatin indiquée ci-dessus, sous forme éventuellement d'un mélange d'isomères 7α et 7β, à l'action d'un agent de déshydrogénation, pour obtenir un produit de formule ($I'''_A$) :

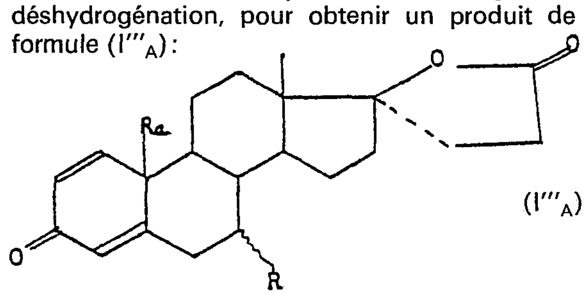

($I'''_A$)

dans laquelle Ra et R ont la signification précédente, sous la forme, éventuellement, d'un mélange d'isomères 7α et 7β que l'on sépare, si désiré, puis soumet, si désiré, soit chacun des isomères, soit leur mélange, à l'action d'un

hydroxyde alcalin ou de l'ammoniaque, pour obtenir les composés de formule ($I'''_B$) :

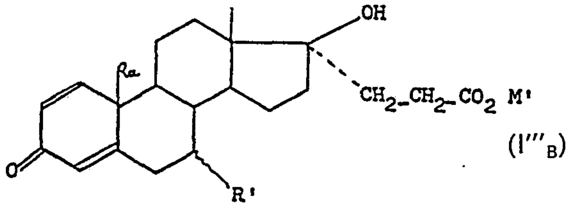

($I'''_B$)

dans laquelle Ra est défini comme précédemment, M' représente un atome de métal alcalin ou le groupement $NH_4$ et R' a la signification de R à l'exception de la valeur $(CH_2)_nR_3$ dans laquelle $R_3$ représente un radical carboxy libre, produit de formule ($I'''_B$) sous forme d'isomères 7α ou 7β ou de leur mélange, que l'on sépare, si désiré, en chacun des isomères, puis soumet, si désiré, soit chacun des isomères, soit leur mélange, à l'action d'un agent acide, pour obtenir un produit de formule ($I'''_C$) :

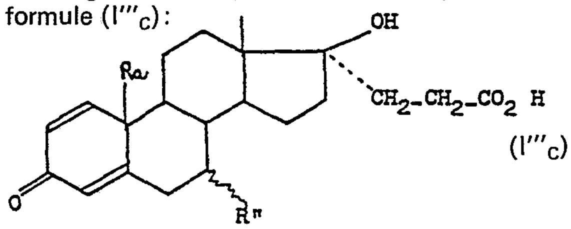

($I'''_C$)

dans laquelle Ra est défini comme précédemment et R'' a la signification de R à l'exception de la valeur $(CH_2)_n-R_3$ dans laquelle $R_3$ représente un radical carboxy salifié.

La transformation des produits de formule (IV) en produits de formule ($I'''_A$) est effectuée de préférence en faisant agir, par voie biochimique, la bactérie *Arthrobacter simplex*. On peut également utiliser un autre micro-organisme. Dans ce cas, la réaction est effectuée de préférence en milieu aqueux tamponné. Pour des raisons de solubilité, on utilise de préférence un sel d'un produit de formule (IV) préparé en faisant agir une base telle que la soude ou la potasse sur le produit de formule (IV). Lorsque la réaction avec le micro-organisme est effectuée, on obtient le produit de formule ($I'''_A$) en acidifiant la solution de sel de produit de formule ($I'''_A$) obtenue intermédiairement. On peut utiliser, par exemple, un acide tel que l'acide chlorhydrique, sulfurique ou acétique.

On peut également utiliser la voie chimique en soumettant les produits de formule (IV) à l'action d'un dérivé de la p-benzoquinone ou du chloranile; la réaction a lieu au sein, par exemple, d'une solution dans un solvant organique tel que le dioxanne, l'acétone, le benzène ou l'alcool tert.-butylique.

On peut également utiliser, comme réactif de déshydrogénation, l'anhydride sélénieux ou le benzène sélénique.

La transformation des produits ($I'''_A$) en produits ($I'''_B$) ainsi que la séparation éventuelle des isomères et l'acidification conduisant aux produits ($I'''_C$) sont réalisées dans les conditions décrites précédemment.

Les produits de formule (I') présentent de très intéressantes propriétés pharmacologiques; ils sont en particulier des antagonistes de l'aldostérone et augmentent la diurèse hydrosodée avec conservation du potassium organique.

Les produits de formule (I') offrent, de plus, l'avantage de ne présenter que très peu d'effets hormonaux secondaires. Des tests effectués sur récepteur et *in vivo* ont, en particulier, montré que les produits de formule (I') sont moins antiandrogènes que des produits apparentés décrits précédemment.

Les produits de formule (I') peuvent donc être utilisés avantageusement pour lutter, notamment, contre l'hypertension artérielle et les insuffisances cardiaques.

L'invention a donc pour objet les produits de formule (I') à titre de médicaments.

L'invention a notamment pour objet, à titre de médicaments, les produits de formule (I'') correspondant aux produits de formule (I') dans laquelle Ra représente un radical méthyle, R représente $R_2$ et le trait pointillé en position 1(2) une simple liaison, ou dans laquelle Ra représente un atome d'hydrogène, R est défini comme dans la formule (I') et le trait pointillé en position 1(2) représente une simple liaison, étant entendu que n ne représente par le nombre 1 lorsque $R_3$ représente un atome d'halogène.

Parmi ces produits, l'invention a plus particulièrement pour objet, à titre de médicaments, les produits de formule (I'') dans laquelle X et Y représentent ensemble le groupement:

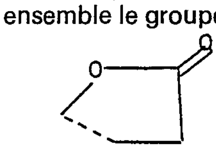

ou dans laquelle X représente un radical hydroxyle et Y représente un radical $-CH_2-CH_2-CO_2K$.

De même, on peut citer notamment ceux dans lesquels le radical R est dans la position 7α et représente plus particulièrement un radical chlorpropyle, hydroxypropyle, carboxyéthyle, méthoxycarbonyléthyle.

L'invention a notamment pour objet, à titre de médicaments, les produits de formule (I''') correspondant aux produits de formule (I') dans laquelle le trait pointillé en position 1(2) indique la présence d'une double liaison. Parmi ces produits, l'invention a plus particulièrement pour objet, à titre de médicaments, les produits de formule (I''') dans laquelle X et Y représentent ensemble le groupement:

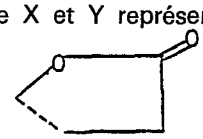

et ceux dans lesquels X représente un radical hydroxyle et Y représente un radical $-CH_2-Ch_2-CO_2K$.

De même, on peut citer notamment ceux dans lesquels le radical R est dans la position 7α et représente un radical propyle, butyle, isobutyle ou buténlyle, étant entendu que Ra ne représente pas un radical méthyle lorsque la liaison 1(2) est une simple liaison, ou encore un radical chloropropyle, hydroxypropyle, carboxyéthyle, méthoxycarbonyléthyle.

L'invention a plus spécialement pour objet, à titre de médicaments, la γ-lactone de l'acide de l'acide 17β-hydroxy 7α-propyl 3-oxo 17α-pregna 1,4-diène 21-carboxylique et le 17β-hydroxy 3-oxo 7α-propyl 17α-prégna 1,4-diène 21-carboxylate de potassium.

La posologie utile varie en fonction de l'affection à traiter et de la voie d'administration; elle peut aller par exemple de 10 à 500 mg/d chez l'adulte par voie orale pour le produit de l'exemple 10.

Les composés de formule (I') sont utilisés par voie buccale, rectale, transcutanée ou intraveineuse. Ils peuvent être prescrits sous forme de comprimés, de comprimés enrobés, de cachets, de capsules, de granulés, d'émulsions, de sirops, de suppositoires et de préparations injectables.

L'invention a donc également pour objet les compositions pharmaceutiques renfermant comme principe actif au moins un composé de formule (I'). Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (II) utilisés comme produits de départ du procédé de l'invention sont des produits connnus; ils peuvent être préparés selon les procédés décrits dans le brevet américain USP N° 3194803 ou dans le brevet français N° 2216276.

Les produits de formule (IV) dans lesquels Ra est un radical méthyle et R est un radical alkyle saturé ou instauré, différent de méthyle, peuvent être préparés selon le procédé décrit dans la demande de brevet européen publiée sous le N° 0018245.

Le produit de formule (IV) dans lequel R et Ra représentent un radical méthyle est décrit dans «J. Org. Chem. Soc.», *26*, 3077-83 (1961).

Les autres produits de formule (IV) sont constitués par les produits de formule (I'') dont un procédé de préparation est indiqué ci-dessus.

Les exemples suivants illustrent l'invention sans toutefois la limiter.

*Exemple 1:*

*γ-Lactone de l'acide 17β-hydroxy 7α-propyl 3-oxo 17α-pregna-1,4-diène 21-carboxylique*

On met en solution sous atmosphère inerte 2 g de la γ-lactone de l'acide 17β-hydroxy 7α-propyl 3-oxo 17α-pregn-4-ène 21-carboxylique et 1,44 g de 2,3-dichloro 5,6-dicyano 1,4-benzoquinone dans 10 $cm^3$ de dioxanne. On agite au reflux pendant 1¾ h. On glace 10 min, essore le précipité, le rince au dioxanne.

La phase organique est lavée avec une solution aqueuse de thiosulfate de sodium à 10%, puis à l'ammoniaque N. On réextrait les lavages à l'acétate d'éthyle, sèche les phases organiques réunies, et distille à sec sous pression réduite. On obtient 2,8 g de produit brut que l'on chromatographie sur silice en éluant avec un mélange benzène/acétate d'éthyle (8/2). On recueille 1,4 g d'un produit que l'on cristallise dans 5 $cm^3$ d'éther éthylique et essore.

On obtient 1,3 g de produit que l'on peut recristalliser dans l'isopropanol, F = 202° C.

*Analyse* pour $C_{25}H_{34}O_3$=382,52:

| | | |
|---|---|---|
| Calculé: | C 78,49 | H 8,96% |
| Trouvé: | C 78,6 | H 9,1 % |

Pouvoir rotatoire: $/\alpha/_D$ = +3 ±2° (c = 0,8% dans le chloroforme).

*Exemple 2:*

*γ-Lactone de l'acide 17β-hydroxy 7α-/(1,1-diméthyléthoxy)propyl/3-oxopregn-4-ène 21-carboxylique* et *γ-lactone de l'acide 17β-hydroxy 7β-/(1,1-diméthyléthoxy)propyl/3-oxopreg-4-ène 21-carboxylique*

On mélange sous atmosphère inerte 1,36 g de chlorure cuivrique, 0,860 g de chlorure de lithium, 150 $cm^3$ d'éther anhydre et 150 $cm^3$ de tétrahydrofuranne anhydre. On ajoute ensuite 17 g de γ-lactone de l'acide 17β-hydroxy 3-oxopregna 4,6-diène 21-carboxylique (canrénone).

On refroidit à −30° C et ajoute goutte à goutte sous agitation en 1 h 105 $cm^3$ d'une solution de chlorure de 1,1-diméthyléthyléthoxypropylmagnésium dans le tétrahydrofuranne (0,68N).

On agite 30 min à −30° C et ajoute 100 $cm^3$ d'acide chlorhydrique 5N. On agite 5 min en laissant remonter la température à température ambiante. On verse dans 250 $cm^3$ d'ammoniaque 2N, décante, réextrait à l'éther, lave à l'ammoniaque 0,5N, puis à l'eau. On sèche, filtre, distille à sec et obtient 23 g de produit brut.

Le produit obtenu est chromatographié sur silice (éluant: éther éthylique/benzène 85/15).

On sépare 13,7 g d'isomère 7α (Rf = 0,45) et 5,1 g d'isomère 7β (Rf = 0,35).

*Isomère 7α:* il est cristallisé dans 10 $cm^3$ d'éther isopropylique. Après essorage, on obtient 11,1 g de produit, F = 118° C.

Après recristallisation de 5 g de ce produit dans un mélange éther éthylique/éther isopropylique (1/1), on obtient 4,2 g de produit pur.

*Analyse* pour $C_{29}H_{44}O_4$ = 456,64:

| | | |
|---|---|---|
| Calculé: | C 76,27 | H 9,71% |
| Trouvé: | C 76,4 | H 9,8 % |

Pouvoir rotatoire: $/\alpha/_D$ = +55 ±1,5° (c = 1,3% du $CHCl_3$)

RMN: 60 MHz $CDCl_3$ ppm
1: méthyl en 18
1,19: t.-butyl sur la chaîne propyl en 7α
1,225: méthyl en 19
3,3 (t): méthylène adjacent du t.-butyl en 7α (J=6 Hz)
5,74: proton en 4.

*Isomère 7β:*
RMN: 60 MHz $CDCl_3$ ppm
1,18: méthyl en 19
5,71: proton en 4
3,3 (t): méthylène adjacent au t.-butyl en 7β.

*Exemple 3:*

*γ-Lactone de l'acide 17β-hydroxy 7α-/3-hydroxypropyl/3-oxo 17α-pregn-4-ène 21-carboxylique* et *γ-lactone de l'acide 17β-hydroxy 7β-/3-hydroxypropyl/3-oxo 17α-pregn-4-ène 21-carboxylique*

*Isomère 7α:* on met en solution sous atmosphère inerte 9,5 g de l'isomère 7α obtenu à l'exemple 2 dans 19 $cm^3$ de dioxanne et 28,5 $cm^3$ d'acide chlorhydrique concentré 22° B. On agite 1½ h à température ambiante et verse sur 150 $cm^3$ d'eau. On extrait à l'acétate d'éthyle, lave à l'aide d'une solution aqueuse de carbonate acide de sodium, puis à l'eau. On sèche, filtre et distille à sec sous pression réduite.

On obtient 8,3 g de produit cristallisé que l'on chromatographie sur silice (éluant: benzène/méthyléthylcétone 5/5). On obtient 7 g de produit attendu, F = 208° C, que l'on peut cristalliser dans l'acétate d'éthyle, F = 208° C.

*Analyse* pour $C_{25}H_{36}O_4$ = 400,54:

| | | |
|---|---|---|
| Calculé: | C 74,96 | H 9,06% |
| Trouvé: | C 75,0 | H 9,1 % |

Pouvoir rotatoire: $/\alpha/_D$ = +61,5 ±1,5° (c = 1% dans $CHCl_3$).

*Isomère 7β:* on opère dans des conditions identiques à celles utilisées pour l'isomère 7α. En partant de 2,7 g de l'isomère 7β du produit de l'exemple 2, on obtient 1,7 g du produit attendu.

Après recristallisation dans l'acétate d'éthyle, on obtient un produit pur, F = 192° C.

*Analyse* pour $C_{25}H_{36}O_4$ = 400,54:

| | | |
|---|---|---|
| Calculé: | C 74,96 | H 9,06% |
| Trouvé: | C 74,9 | H 9,1 % |

Pouvoir rotatoire: $/\alpha/_D$ = +59 ±1,5° (c = 1% dans $CDCl_3$).

*Exemple 4:*

*γ-Lactone de l'acide 17β-hydroxy- 7α-carboxyéthyl-3-oxo 17α-pregn-4-ène 21-carboxylique*

On dissout sous atmosphère inerte 1,5 g d'isomère 7α obtenu à l'exemple 3 dans 45 $cm^3$ d'acétone. On refroidit à +5° C et ajoute 1,5 g de réactif d'Heilbron 8N. On agite 30 min à 5° C, verse sur 50 $cm^3$ d'eau, extrait à l'acétate d'éthyle et lave à l'eau. On extrait à l'aide d'une solution aqueuse de carbonate acide de sodium et acidifie par de l'acide chlorhydrique concentré.

On glace, essore, sèche sous pression réduite et obtient 1,45 g de produit attendu, F = 205° C.

Après recristallisation dans 20 $cm^3$ de méthanol, on obtient 1,05 g de produit pur, F = 205° C.

*Analyse* pour $C_{25}H_{34}O_5$ = 414,52:

| | | |
|---|---|---|
| Calculé: | C 72,43 | H 8,27% |
| Trouvé: | C 72,7 | H 8,4 % |

Pouvoir rotatoire: $/\alpha/_D$ = +43,5 ±1° (c = 1% dans $CHCl_3$).

Le réactif d'Heilbron peut être préparé de la manière suivante: pour 1 l de réactif d'Heilbron, on mélange 270 g d'oxyde chromique, 400 $cm^3$ d'eau, 230 $cm^3$ d'acide sulfurique et on complète à 1 l par de l'eau.

*Exemple 5:*

*γ-Lactone de l'acide 17β-hydroxy 7α-isopropoxycarbonyléthyl-3-oxo 17α-pregn-4-ène 21-carboxylique*

On agite à 80° C sous atmosphère inerte 2 g d'acide tel qu'obtenu à l'exemple 4 et 1,5 g d'O-isopropyl N,N'-dicyclohexyliso-urée dans 25 $cm^3$ de dioxanne. Après 46 h d'agitation, on glace, essore la dicyclohexylurée formée, la lave au chlorure de méthylène et distille le filtrat à sec sous pression réduite. On obtient 2,8 g de produit brut que l'on chromatographie sur silice (éluant: chlorure de méthylène/dioxanne 95/5). On recueille 1,85 g de produit attendu. Après recristallisation dans l'éther isopropylique, on obtient 1,7 g de produit pur, F = 130° C.

*Analyse* pour $C_{28}H_{40}O_5$ = 456,60:

| | | |
|---|---|---|
| Calculé: | C 73,65 | H 8,83% |
| Trouvé: | C 73,6 | H 8,8 % |

Pouvoir rotatoire: $/\alpha/_D$ = +31,5 ±1,5° (c = 1% dans $CHCl_3$).

L'O-isopropyl N,N'-dicyclohexyliso-urée à été préparée comme suit: on agite à 55° C pendant 6 h sous atmosphère inerte 3,09 g de dicyclohexylcarbodiimide, 30 mg de chlorure cuivreux et 994 mg d'isopropanol.

On dilue avec de l'acétate d'éthyle, lave à l'eau, puis à l'ammoniaque N. On sèche, filtre et distille à sec sous pression réduite. On obtient 3,2 g de produit utilisé tel quel.

*Exemple 6:*

*γ-Lactone de l'acide 17β-hydroxy 7α-méthoxycarbonyléthyl 3-oxo 17α-pregn-4-ène 21-carboxylique*

On dissout sous atmosphère inerte 800 mg d'acide obtenu à l'exemple 4 dans 6 $cm^3$ de chloroforme. On ajoute du diazométhane en solution dans le chlorure de méthylène et agite 15 min à température ambiante. On distille à sec sous pression réduite et purifie par chromatographie sur silice (éluant: éther éthylique/benzène 85/15).

On recueille 780 mg de produit, F = 147° C. On recristallise dans 2 $cm^3$ d'acétate d'éthyle pour obtenir 610 mg d'ester pur, F = 147° C.

*Analyse* pour $C_{26}H_{36}O_5$ = 428,55:

| | | |
|---|---|---|
| Calculé: | C 72,86 | H 8,47% |
| Trouvé: | C 72,9 | H 8,6 % |

Pouvoir rotatoire: $/\alpha/_D$ = +44 ±1° (c = 1% dans $CHCl_3$).

*Exemple 7:*

*γ-Lactone de l'acide 7α-(3-chloropropyl) 17β-hydroxy 3-oxo 17α-pregn-4-ène 21-carboxylique*

On met en solution sous atmosphère inerte 4 g de γ-lactone de l'acide 17β-hydroxy 7α-(3-hydroxypropyl) 3-oxo 17α-pregn-4-ène 21-carboxylique décrite à l'exemple 3 et 3,8 g de triphénylphosphine dans 40 $cm^3$ de tétrachlorure de carbone anhydre et 15 $cm^3$ de chloroforme. On agite 3 h au reflux, filtre l'insoluble et lave au chloroforme. On distille à sec sous pression réduite et obtient 8,99 g d'un produit brut que l'on purifie par chromatographie sur silice (éluant: benzène/méthyléthylcétone 5/5). On isole 3,5 g de produit que l'on recristallise dans l'acétate d'éthyle. On obtient 3,2 g de produit pur, F = 202° C.

*Analyse* pour $C_{25}H_{35}ClO_3$ = 418,99:

| | | | |
|---|---|---|---|
| Calculé: | C 71,66 | H 8,42 | Cl 8,46% |
| Trouvé: | C 71,7 | H 8,5 | Cl 8,5 % |

Pouvoir rotatoire:/α/$_D$ = +60 ±1,5° (c = 1% dans le $CHCl_3$).

*Exemple 8:*

*γ-Lactone de l'acide 7α-(3-chloropropyl) 17β-hydroxy 3-oxo 17α-pregna-1,4-diène 21-carboxylique*

On met en solution sous atmosphère inerte 3,5 g de γ-lactone de l'acide 7α-(3-chloropropyl) 17β-hyroxy 3-oxo 17α-pregn-4-ène 21-carboxylique préparé à l'exemple 7 et 2,3 g de 2,3-dichloro 5,6-dicyano 1,4-benzoquinone dans 17,5 $cm^3$ de dioxanne.

On agite au reflux pendant 3 h. On glace ensuite pendant 10 min, essore le précipité, rince au dioxanne et dilue le filtrat avec 30 $cm^3$ d'acétate d'éthyle.

On lave la phase organique à l'aide d'une solution aqueuse de thiosulfate de sodium à 10%, puis à l'ammoniaque 0,5N.

On réextrait à l'acétate d'éthyle, sèche, filtre et distille à sec sous pression réduite. On obtient 4,7 g de produit que l'on chromatographie sur silice (éluant: benzène/acétate d'éthyle 8/2). On recueille 2,4 g d'une huile que l'on cristallise dans 5 $cm^3$ d'éther éthylique.

On obtient 2,3 g de produit F = 196° C que l'on peut recristalliser dans l'isopropanol pour obtenir 1,9 g de produit pur, F = 196° C.

*Analyse* pour $C_{25}H_{33}ClO_3$ = 416,97:

| | | | |
|---|---|---|---|
| Calculé: | C 72,01 | H 7,98 | Cl 8,50% |
| Trouvé: | C 72,0 | H 8,1 | Cl 8,7 % |

Pouvoir rotatoire:/α/$_D$ = +4 ±1,5° (c = 0,8% dans $CHCl_3$).

*Exemple 9:*

*γ-Lactone de l'acide 7α-(but-3-ényl) 17β-hydroxy 3-oxo 17α-pregna-1,4-diène 21-carboxylique*

On met en solution sous atmosphère inerte 4,7 g de γ-lactone de l'acide 7α-(but-3-ényl) 17β-hydroxy 3-oxo 17α-pregn-4-ène 21-carboxylique et 3,5 g de 2,3-dichloro 5,6-dicyano 1,4-benzoquinone dans 23,5 $cm^3$ de dioxanne. On agite au reflux pendant 2½ h, glace, essore le précipité, le rince au dioxanne.

On dilue le filtrat avec 30 $cm^3$ d'acétate d'éthyle, lave à l'aide d'une solution aqueuse de thiosulfate de sodium à 10%, puis à l'ammoniaque 0,5N, puis enfin à l'eau. On extrait à l'acétate d'éthyle, sèche, filtre et distille sous pression réduite. On obtient 5,2 g d'une huile que l'on chromatographie sur silice (éluant: benzène/acétate d'éthyle 8/2). On recueille 1,8 g d'un produit que l'on cristallise dans l'éther . On obtient 1,7 g de produit attendu, F = 197° C.

*Analyse* pour $C_{26}H_{34}O_3$ = 394,53:

| | | |
|---|---|---|
| Calculé: | C 79,15 | H 8,69% |
| Trouvé: | C 79,3 | H 8,8 % |

Pouvoir rotatoire:/α/$_D$ = −7 ±1° (c = 0,9% dans $CHCl_3$).

*Exemple 10:*

*17β-Hydroxy-3-oxo 7α-propyl 17α-pregna-1,4-diène 21-carboxylate de potassium*

On met en suspension sous atmosphère inerte 2 g de γ-lactone de l'acide 17β-hydroxy 7α-propyl 3-oxo 17α-pregna-1,4-diène 21-carboxylique préparé à l'exemple 1 dans 11,2 $cm^3$ de potasse éthanolique 0,45N et 11,2 $cm^3$ d'eau. On porte au reflux 15 min, distille à sec sous pression réduite et ajoute 50 $cm^3$ d'acétone. On glace et essore le produit attendu. On obtient 2 g de produit, F = 280° C.

Ce produit est dissous dans un mélange eau/acétone (1/1) tiédi. On filtre, ajoute 70 $cm^3$ d'aétone, glace et essore. On obtient 1,7 g de sel de potassium attendu sous forme de monohydrate, F = 280° C.

*Analyse* pour $C_{25}H_{35}KO_4$ = 438,63 (produit séché à 100° C):

| | | |
|---|---|---|
| Calculé: | C 68,45 | H 8,04% |
| Trouvé: | C 68,2 | H 8,0 % |

*Analyse* produit non séché (1 $H_2O$):

| | | |
|---|---|---|
| Calculé: | C 65,75 | H 8,17% |
| Trouvé: | C 65,5 | H 8,2 % |

Pouvoir rotatoire:/α/$_D$ = −20 ±1° (c = 1% dans l'eau).

*Exemple 11:*

*7α/1,1-(Diméthyléthoxy)propyl/ 17β-hydroxy-3-oxo 17α-pregn-4-ène 21-carboxylate de potassium*

En opérant comme indiqué à l'exemple 10 au départ de 1,5 g de produit 7α obtenu a l'exemple 2, on obtient 0,9 g de produit attendu, F > 300° C.

*Analyse* pour $C_{25}H_{45}KO_5$ = 512,75 (pertes sous vide 7,8% à 150° C):

| | | |
|---|---|---|
| Calculé: | C 67,93 | H 8,85% |
| Trouvé: | C 67,6 | H 8,8 % |

K. Fischer: 7,2 à 7,3% d'eau

Pouvoir rotatoire:/α/$_D$ = +29,5 ±1,5° (c = 1% dans l'eau).

*Exemple 12:*

*17β-Hydroxy 7α-(3-hydroxypropyl) 3-oxopregn-4-ène 21-carboxylate de potassium*

En opérant comme indiqué à l'exemple 10, au départ de 1,4 g de produit obtenu à l'exemple 3, on obtient 0,95 g de produit attendu, F > 300° C.

*Analyse* pour $C_{25}H_{37}KO_5$ = 456,65 (pertes sous vide 11,8% à 150° C):

| | | |
|---|---|---|
| Calculé: | C 65,75 | H 8,17% |
| Trouvé: | C 65,7 | H 8,2 % |

K. Fischer: 11,3 à 11,7% d'eau

Pouvoir rotatoire: $/\alpha/_D$ = +36,5 ±1,5° (c = 1% dans l'eau).

*Exemple 13:*

*7α-(3-Chloropropyl) 17β-hydroxy 3-oxo 17α-pregn-4-ène 21-carboxylate de potassium*

En opérant comme indiqué à l'exemple 10, au départ de 1,5 g de produit obtenu à l'exemple 7, on obtient 1,25 g de produit attendu, F = 235° C.

*Analyse* pour $C_{25}H_{36}KO_4Cl$ = 475,11 (pertes sous vide 2,8% à 150° C):

| | | | |
|---|---|---|---|
| Calculé: | C 63,20 | H 7,64 | Cl 7,46% |
| Trouvé: | C 63,2 | H 7,6 | Cl 7,4 % |

K. Fischer: 4,7% d'eau

Pouvoir rotatoire: $/\alpha/_D$ = +38 ±1,5° (c = 1% d'eau).

*Exemple 14:*

*7α-(3-Chloropropyl) 17β-hydroxy 3-oxo 17α-pregna-1,4-diène 21-carboxylate de potassium*

En opérant comme indiqué à l'exemple 10, au départ de 0,84 g de produit obtenu à l'exemple 8, on obtient 0,62 g de produit attendu, F = 230° C.

*Analyse* pour $C_{25}H_{34}ClKO_4$ = 473,094 (solvaté à 5% d'eau):

| | | | |
|---|---|---|---|
| Calculé: | C 60,29 | H 7,43 | Cl 7,12% |
| Trouvé: | C 60,4 | H 7,0 | Cl 7,2 % |

K. Fischer: 4,7 à 5,2 d'eau

Pouvoir rotatoire: $/\alpha/_D$ = 22,5 ±1,5° (c = 1,1% dans l'eau).

*Exemple 15:*

*7α-(But-3-ényl) 17β-hydroxy 3-oxo 17α-pregna-1,4-diène 21-carboxylate de potassium*

En opérant comme indiqué à l'exemple 10 au départ de 1,16 g de produit obtenu à l'exemple 9, on obtient 0,634 g de produit attendu, F ≃ 270° C.

*Analyse* pour $C_{26}H_{35}KO_4 1/2H_2O$ = 459,65:

| | | |
|---|---|---|
| Calculé: | C 67,93 | H 7,89% |
| Trouvé: | C 67,6 | H 7,8 % |

K. Fischer: 1,9 à 2,1% d'eau

Pouvoir rotatoire: $/\alpha/_D$ = −32 ±1° (c = 1% dans l'eau).

*Exemple 16:*

*γ-Lactone de l'acide 17β-hydroxy 7α-propyl 3-oxo 17α-pregna-1,4-diène 21-carboxylique*

On met en suspension sous atmosphère inerte 10 g de la γ-lactone de l'acide 17α-hydroxy 7α-propyl 3-oxo 17α-pregn-4-ène 21-carboxylique dans 60,5 cm³ de potasse méthanolique et 60,5 cm³ d'eau. On porte au reflux pendant 15 min et obtient une solution de sel de potassium (solution A). On prépare séparément une solution-tampon comprenant 13,6 g de phosphate monopotassique dans 1,9 l d'eau. On ajoute 60 cm³ de soude N et ajoute le pH à 7,2 par addition de 6,1 cm³ de soude normale (solution B).

On mélange 1,5 l de solution B à la solution A ci-dessus, puis ajoute 50 mg de ménadione, 100 cm³ de crème enzymatique d'*Arthrobacter simplex* et 10 g d'hyflosupercel.

On agite la suspension sous barbotage d'air pendant 24 h à 33 ±1° C.

On acidifie ensuite avec 100 cm³ d'acide chlorhydrique 5N et agite 30 min à température ambiante. On ajoute 400 cm³ d'acétate d'éthyle, agite 15 min, filtre sur hyflosupercel et rince à l'acétate d'éthyle.

On décante le filtrat, lave à l'aide d'une solution aqueuse de thiosulfate de sodium à 10%, puis d'une solution aqueuse de carbonate acide de sodium, puis par de l'eau. On réextrait à l'acétate d'éthyle, sèche, filtre et distille à sec sous pression réduite. On obtient 9,5 g de produit attendu.

Le gâteau d'hyflosupercel est remis en suspension dans 150 cm³ de méthanol pendant 30 min sous agitation. On filtre, concentre le filtrat sous pression réduite, dilue à l'eau et extrait à l'acétate d'éthyle. On sèche, filtre et obtient 0,6 g de produit attendu. Une nouvelle extraction de l'hyflosupercel par un mélange de chlorure de méthylène/méthanol (1/1) permet de récupérer 140 mg de produit.

On réunit les trois lots (soit 10,2 g), les dissout dans 300 cm³ d'acétate d'éthyle à environ 50° C et ajoute 41,5 g de charbon actif. On agite 5 min et filtre sur hyflosupercel. On distille le filtrat à sec sous pression réduite.

On reprend les cristaux dans 25 cm³ d'éther isopropylique, essore et recueille 9,6 g de produit pur, F = 202° C.

Le produit est identique à celui obtenu à l'exemple 1.

*Exemple 17:*

*γ-Lactone de l'acide 17β-hydroxy 7α-propyl 3-oxo 17α-pregna-1,4-diène 21-carboxylique*

On chauffe à 100° C et sous atmosphère inerte 1 g de la γ-lactone de l'acide 17β-hydroxy 7α-propyl 3-oxo 17α-pregn-4-ène 21-carboxylique dans 10 cm³ de toluène anhydre et 1,1 g d'anhydride benzènesélénique. Après 1 h d'agitation, on dilue la solution obtenue avec 30 cm³ d'acétate d'éthyle, lave à l'eau et avec une solution aqueuse de carbonate acide de sodium. On

réextrait les phases aqueuses à l'acétate d'éthyle, sèche, filtre et distille à sec sous pression réduite.

On obtient 1,25 g d'huile. Par chromatographie sur silice (éluant: benzène/acétate d'éthyle 8/2), on recueille 850 mg de produit attendu, F = 202° C.

Le produit est identique à celui obtenu aux exemples 1 et 16.

*Exemple 18:*

*γ-Lactone de l'acide 7α-butyl 17β-hydroxy 3-oxo 17α-pregna-1,4-diène 21-carboxylique*

On opère de la manière indiquée à l'exemple 16, à partir de 2 g de γ-lactone de l'acide 7α-butyl 17β-hydroxy 3-oxo 17α-pregna-4-ène 21-carboxylique et obtient 1,6 g de produit attendu, F = 183° C.

*Analyse* pour $C_{26}H_{36}O_3$ = 396,5:

| | | |
|---|---|---|
| Calculé: | C 78,74 | H 9,15% |
| Trouvé: | C 79,0 | H 9,3 % |

Pouvoir rotatoire:/α/$_D$ = −9,5 ±2° (c = 0,7% dans $CHCl_3$).

*Exemple 19:*

*γ-Lactone de l'acide 7α-(2-méthylpropyl) 17β-hydroxy 3-oxo 17α-pregna-1,4-diène 21-carboxylique*

On opère de la manière indiquée à l'exemple 16, à partir de 1 g de γ-lactone de l'acide 7α-(2-méthoxypropyl) 17β-hydroxy 3-oxo 17α-pregn-4-ène 21-carboxylique et obtient un premier lot de 0,9 g de produit attendu.

On reprend le gâteau d'hyflosupercel par du chlorure de méthylène, filtre, lave le filtrat à l'aide d'une solution aqueuse de carbonate acide de sodium, sèche, filtre et évapore à sec pour obtenir 0,2 g de produit.

Les deux lots sont réunis (soit 1,1 g), purifiés par chromatographie sur silice (éluant: benzène/acétate d'éthyle 8/2). On recueille 0,925 g de produit que l'on peut recristalliser dans l'isopropanol, F = 245° C.

*Analyse* pour $C_{26}H_{36}O_3$ = 396,5:

| | | |
|---|---|---|
| Calculé: | C 78,74 | H 9,15% |
| Trouvé: | C 78,9 | H 9,4 % |

Pouvoir rotatoire:/α/$_D$ = +6,5 ±2° (c = 0,5% dans $CHCl_3$).

*Exemple 20:*

*γ-Lactone de l'acide 7α-diméthylaminopropyl 17β-hydroxy 3-oxo 17α-pregn-4-ène 21-carboxylique*

On dissout à température ambiante sous atmosphère inerte 0,810 g de chlorure cuivrique et 0,510 g de chlorure de lithium dans 210 cm³ de tétrahydrofuranne et ajoute 10,2 g de γ-lactone de l'acide 17β-hydroxy 3-oxopregna 4,6-diène 21-carboxylique (canrénone). On refroidit la solution à −30° C et ajoute en 1½ h 65 cm³ d'une solution de chlorure de N-diméthylaminopropylmagnésium dans le tétrahydrofuranne (0,85 mM/cm³). On maintient 30 min à −30° C, puis ajoute 100 cm³ d'acide chlorhydrique 5N.

Après 1 h à température ambiante, on alcalinise le mélange réactionnel par addition d'une solution d'ammoniaque 2N, décante et extrait à l'acétate d'éthyle. La phase organique est lavée à l'eau, séchée et évaporée à sec. On recueille 12 g de produit brut que l'on chromatographie sur silice (éluant: chlorure de méthylène/isopropanol/ammoniaque 93/7/0,5). On obtient 4,8 g de produit que l'on recristallise dans l'acétate d'éthyle et recueille 2 g de produit pur, F = 166° C.

*Analyse* pour $C_{27}H_{11}NO_3$ = 427,608:

| | | | |
|---|---|---|---|
| Calculé: | C 75,83 | H 9,67 | N 3,28% |
| Trouvé: | C 76,1 | H 9,8 | N 3,3 % |

Pouvoir rotatoire:/α/$_D$ = +56,5 ±1,5° (c = 1% dans $CHCl_3$).

*Exemple 21:*

*γ-Lactone de l'acide 17β-hydroxy 3-oxo 7α-propyl 19-norpregn-4-ène 21-carboxylique*

On mélange 75 cm³ de tétrahydrofuranne, 101 mg de chlorure cuivrique et 33 mg de chlorure de lithium, agite à 20° C pendant 30 min et ajoute 2,5 g de γ-lactone de l'acide 17β-hydroxy 3-oxo 19-norpregna-4,6-diène 21-carboxylique (obtenu selon le brevet français N° 2216276). Après 30 min sous agitation, on ajoute à +10° C en 30 min 12 cm³ de bromure de n-propylmagnésium. On agite à +15° C pendant 15 min, puis ajoute 15 cm³ d'acide chlorhydrique 1 N, verse sur 300 cm³ d'eau et extrait à l'acétate d'éthyle. On sèche la phase organique, évapore le solvant et obtient un produit brut que l'on chromatographie sur silice en éluant au mélange benzène/acétate d'éthyle (7/3). On obtient 1,78 g de produit attendu (isomère 7α) et 0,38 g d'isomère 7β correspondant.

On dissout le 1,78 g d'isomère 7α dans 5 cm³ de chlorure de méthylène, concentre et reprend à l'éther éthylique. On essore les cristaux formés, les lave à l'éther et les sèche. On obtient 1,56 g de produit attendu, F = 177° C.

*Analyse* pour $C_{24}H_{34}O_3$ = 370,53:

| | | |
|---|---|---|
| Calculé: | C 77,80 | H 9,25% |
| Trouvé: | C 77,9 | H 9,3 % |

Spectre RMN ($CDCl_3$ 60 MHz ppm)
1,03: méthyl en 18
5,92: hydrogène en 4.
Pouvoir rotatoire:/α/$_D$ = +27 ±2° (c = 0,6% $CHCl_3$).

*Exemple 22:*

*Exemple de composition pharmaceutique*

On a préparé des comprimés à 50 mg de produit de l'exemple 10, comme principe actif:

| | |
|---|---|
| Produit de l'exemple 10 | 50 mg |
| Excipient (talc, amidon, stéarate de magnésium) | |

*Etude pharmacologique du produit de l'exemple 10:*

I. *Etude de l'activité antialdostérone*

L'étude a été effectuée au moyen d'un test inspiré de Kagawa C.M. («P.S.E.B.M.», 1958, *99*, 705) et de Marcus («Endocrinology», 1952, *50*, 286).

*La technique utilisée est la suivante:*

Des rats mâles Sprague Dawley SPF IFFA CREDO, de 180 g, sont surrénalectomisés 7 d avant la diurèse, sous anesthésie à l'Imalgène (Kétamine) par voie intrapéritonéale à raison de 100 mg/kg. Dès l'opération et jusqu'à la veille de l'expérience, ils reçoivent comme eau de boisson du sérum physiologique.

Les animaux sont mis à jeun 17 h avant la diurèse; le sérum physiologique est alors remplacé par de l'eau glucosée à 5%.

Les produits sont administrés par voie orale 1 h avant la mise en cage.

Au moment de la mise en diurèse, les animaux reçoivent, d'une part, une surcharge hydrosaline par voie intrapéritonéale, à raison de 5 ml par rat, de sérum physiologique à 9‰ et, d'autre part, 1 µg/kg de monoacétate d'aldostérone en solution alcoolique à 2,5%, par voie sous-cutanée.

Les rats sont alors placés par deux en cage à diurèse, sans nourriture ni boisson, pendant 4 h.

Au bout de ce temps, on effectue une miction forcée par pression sur la vessie et mesure le volume de l'urine recueillie.

Après rinçage soigneux des cages et de la verrerie, le volume des urines est amené à 50 $cm^3$. Sur cette solution, on effectue le dosage du sodium et du potassium urinaires par photométrie de flamme à l'autoanalyseur.

Les résultats obtenus, exprimés en pourcentage d'inhibition de l'activité de 1 µg/kg de monoacétate d'aldostérone injecté par voie sous-cutanée sur le log du rapport: concentration en sodium/concentration en potassium, selon la méthode de Kagawa, «Endocrinology», 1960, *67*, pp. 125-132, sont les suivants:

| | Dose de produit administré par voie orale (mg/kg) | % d'inhibition |
|---|---|---|
| Produit de l'exemple 10 | 2<br>0,4 | 80<br>30 |

II. *Activité androgène*

L'activité androgène du produit de l'exemple 10 a été étudiée par la méthode des récepteurs hormonaux décrits par J.P. Raynaud et coll. dans «J. Ster. Biochem.», 1975, 615-622.

*La technique est la suivante:*

La prostate prélevée sur des rats mâles castrés 24 h auparavant est homogénéisée dans un tampon trométhamine 10 mmol, saccharose 0,25M · HCl, pH 7,4.

L'homogénat est centrifugé à 105 000 g pendant 1 h. Le liquide surnageant, ou cytosol, est alors ajusté de façon à avoir une dilution 1/5 (poids/volume).

On incube à 0° C pendant 2 h le cytosol avec une concentration fixe de 17β-hydroxy 17α-méthyl-estra 4,9,11-triène 3-one tritié, désigné ci-après par produit R tritié, en présence ou non de concentration croissante de ce même produit froid, désigné ci-après produit R froid, de testostérone ou du produit à tester.

On détermine au bout de 2 h la radioactivité du produit tritié lié au récepteur par la technique d'adsorption au charbon/dextran (1,25/0,625%).

On trace ensuite:

– les courbes représentant les pourcentages de produit R tritié lié en fonction du log de la concentration du produit R froid, de la testostérone ou du produit à tester ajoutés, et

– la droite $I_{50}$ parallèle à l'axe des abscisses et d'ordonnée:

$$\frac{B}{T} = \frac{B/T\ Max. + B/T\ Min.}{2}$$

B/T Max. est le pourcentage de produit R tritié lié quand aucun produit n'est ajouté,

B/T Min. est le pourcentage de produit R tritié lié quand la quantité maximale de produit R froid est ajoutée.

Les intersections de cette droite $I_{50}$ et des courbes permettent de déterminer les valeurs CT et CX.

CT: concentration de la testostérone froide qui inhibe de 50% la fixation du produit R tritié.

CX: concentration du produit testé qui inhibe de 50% la fixation du produit R tritié.

L'affinité relative du produit testé ou ARL est donnée par la formule:

$$ARL = 100 \times \frac{CT}{CX}$$

*Les résultats sont les suivants:*

| Produits | ARL |
|---|---|
| Testostérone | 100 |
| Produit de l'exemple 10 | 0,02 |

**Revendications** pour les Etats contractants: BE, CH, DE, FR, GB. IT, LI, LU, NL, SE

1. Les produits de formule générale (I'):

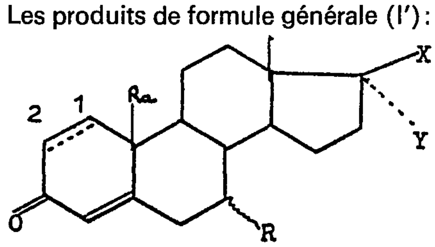

(I')

dans laquelle R représente soit un radical $R_1$, $R_1$ étant un radical alkyle, alkényle ou alkynyle ayant au plus 8 atomes de carbone, soit un radical $R_2$, $R_2$ étant un radical $-(CH_2)_nR_3$ dans lequel n représente un entier de 1 à 8, et $R_3$ repréente un radical hydroxyle libre ou protégé, un radical amino,

amino protégé, mono- ou dialkylamino, un radical carboxy libre, estérifié ou salifié ou un atome d'halogène,
X et Y représentent ensemble le groupement

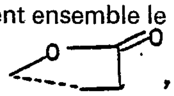

ou bien X représente un radical hydroxyle et Y représente le radical $-CH_2-CH_2-CO_2M$ dans lequel M représente un atome d'hydrogène, un atome de métal alcalin ou un radical ammonium, Ra représente un atome d'hydrogène ou un radical méthyle, le pointillé en position 1(2) indique la présence éventuelle d'une seconde liaison entre les carbones 1 et 2 et le trait ondulé signifie que le substituant R peut se trouver dans l'une des deux positions possibles α ou β, étant entendu que R ne peut pas représenter $R_1$ ou $R_2$ dans lequel n est égal à 1 et $R_3$ représente un atome d'halogène, lorsque la liaison 1(2) est une simple liaison et que Ra représente un radical méthyle, que $R_3$ ne peut pas représenter un radical carboxy libre lorsque Y représente le radical $-CH_2-CH_2-CO_2M$ dans lequel M représente un atome de métal alcalin ou un radical ammonium, et que $R_3$ ne peut pas représenter un radical carboxy salifié lorsque Y représente le radical $-CH_2-CH_2-CO_2H$.

2. Les produits de formule générale (I') telle que définie à la revendication 1, répondant à la formule générale (I):

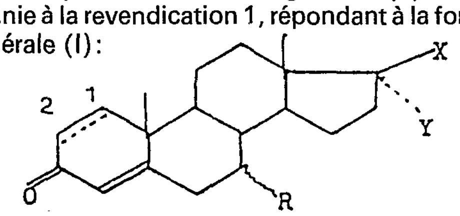

(I)

dans laquelle R, X, Y, le trait pointillé et le trait ondulé ont la signification indiquée à la revendication 1, étant entendu que R ne peut pas représenter $R_1$ ou $R_2$ dans lequel n est égal à 1 et $R_3$ représente un atome d'halogène, lorsque la liaison 1(2) est une simple liaison, que $R_3$ ne peut pas représenter un radical carboxy libre lorsque Y représente le radical $-CH_2-CH_2-CO_2M$ dans lequel M représente un atome de métal alcalin ou un radical ammonium, et que $R_3$ ne peut pas représenter un radical carboxy salifié lorsque Y représente le radical $-CH_2-CH_2-CO_2H$.

3. Les produits de formule (I'') correspondant aux produits de formule (I') telle que définie à la revendication 1, dans laquelle Ra représente un radical méthyle, R représente $R_2$ et le trait pointillé en position 1(2) une simple liaison, étant entendu que n ne représente pas le nombre 1 lorsque $R_3$ représente un atome d'halogène.

4. Les produits de formule (I'') correspondant aux produits de formule (I') telle que définie à la revendication 1, dans laquelle Ra représente un atome d'hydrogène, R a la signification indiquée à la revendication 1 et le trait pointillé en position 1(2) représente une simple liaison.

5. Les produits de formule (I''') correspondant aux produits de formule (I') telle que définie à la revendication 1, dans laquelle le trait pointillé en position 1(2) indique la présence d'une double liaison.

6. Les produits de formule (I') telle que définie à l'une quelconque des revendications 1 à 5, dans laquelle X et Y représentent ensemble le groupement

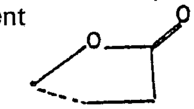

7. Les produits de formule (I') telle que définie à l'une des revendications 1 à 5, dans laquelle X représente un radical hydroxyle et Y représente un radical $-CH_2-CH_2-CO_2K$.

8. Les produits de formule (I') telle que définie à l'une des revendications 1 à 7, dans laquelle le radical R est dans la position 7α.

9. Les produits de formule (I') telle que définie à l'une des revendications 1, 2 et 4 à 8, dans laquelle R représente un radical propyle, butyle, isobutyle ou butényle, étant entendu que Ra ne représente pas un radical méthyle lorsque la liaison 1(2) est une simple liaison.

10. Les produits de formule (I') telle que définie à l'une des revendications 1 à 8, dans laquelle R représente un radical chloropropyle, hydroxypropyle, carboxyéthyle, méthoxycarbonyléthyle.

11. La γ-lactone de l'acide 17β-hydroxy 7α-propyl 3-oxo 17α-pregna-1,4-diène 21-carboxylique, et
le 17β-hydroxy 3-oxo 7α-propyl 17α-pregna-1,4-diène 21-carboxylique de potassium.

12. Procédé de préparation des produits de formule (I') telle que définie à l'une des revendications 1, 2 ou 3, dans laquelle Ra repréènte un radical méthyle, caractérisé en ce que l'on soumet un produit de formule ($II_1$):

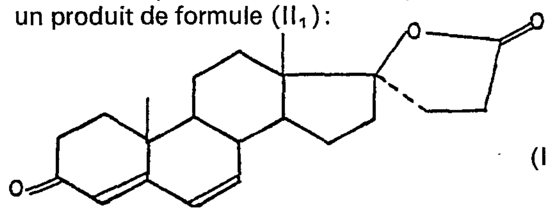

($II_1$)

soit à l'action d'un produit de formule ($III_1$):

$$R_4-(CH_2)_n-MgX' \qquad (III_1)$$

en présence d'un sel de cuivre, formule ($III_1$) dans laquelle X' représente un atome d'halogène, $R_4$ représente un radical hydroxyle protégé, un radical mono- ou dialkylamino ou un radical amino protégé et n a la signification indiquée à la revendication 1,
soit à l'action d'un produit de formule ($III'_1$):

$$[R_4-(CH_2)]_nCuLi \qquad (III'_1)$$

dans laquelle $R_4$ et n conservent la même signification, pour obtenir, après action d'un acide, un produit de formule ($I''_A$):

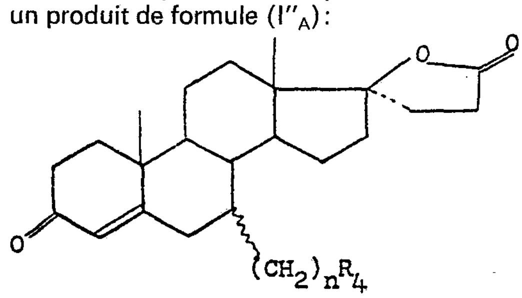

($I''_A$)

sous la forme, éventuellement, d'un mélange d'isomères 7α et 7β, mélange que, si désiré, l'on sépare, et, si désiré, soumet chaque isomère, ou le mélange des produits dans lesquels $R_4$ représente un radical hydroxyle protégé ou un radical amino protégé, à l'action d'un agent d'hydrolyse, pour obtenir un produit de formule (I"$_B$):

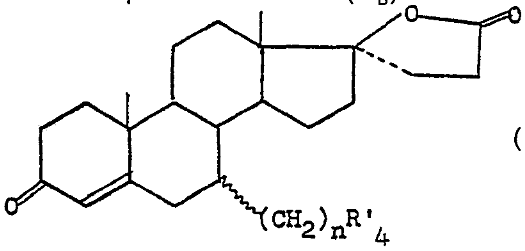

(I"$_B$)

dans laquelle R'$_4$ représente un radical hydroxyle ou amino, et produit de formule (I"$_B$) dans laquelle R'$_4$ représente un radical hydroxyle que l'on traite, le cas échéant,

soit, lorsque n est un entier supérieur à 1, par un réactif d'oxydation, pour obtenir un produit de formule (I"$_C$):

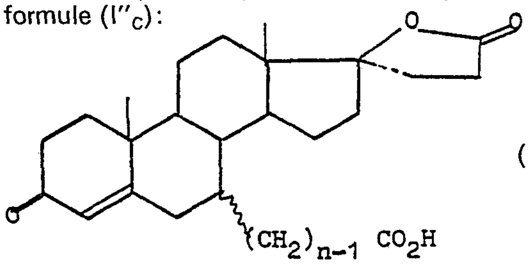

(I"$_C$)

produit que, le cas échéant, l'on estérifie ou salifie selon les méthodes usuelles,

soit, à l'exception du cas où n est égal à 1, par un réactif d'halogénation, pour obtenir un produit de formule (I"$_D$):

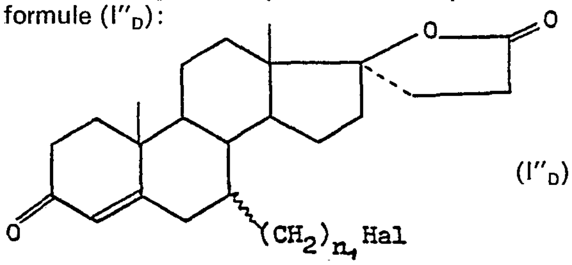

(I"$_D$)

dans laquelle $n_1$ représente un entier de 2 à 8 et Hal représente un atome d'halogène et que, si désiré, l'on soumet les produits de formules (I"$_A$), (I"$_B$), (I"$_C$) et (I"$_D$), sous forme d'isomères 7α ou 7β ou de leur mélange, à l'action d'un hydroxyde alcalin ou de l'ammoniaque, pour obtenir les composés de formule (I"$_T$):

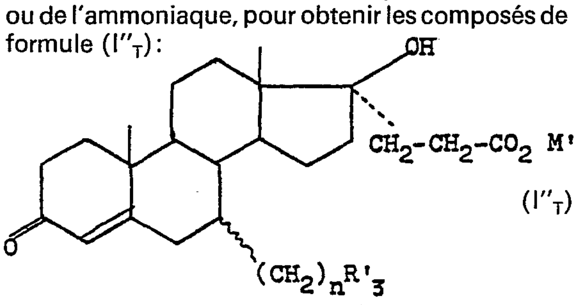

(I"$_T$)

dans laquelle R'$_3$ a la signification de $R_3$ indiquée à la revendication 1, à l'exception de la valeur carboxy libre et M' représente un atome de métal alcalin ou un radical ammonium, sous forme d'isomères 7α ou 7β ou de leur mélange, que l'on sépare, si désiré, en chacun de ses isomères, puis soumet, si désiré, soit chacun des isomères, soit leur mélange, à l'action d'un agent acide, pour obtenir un composé de formule (I"$_U$):

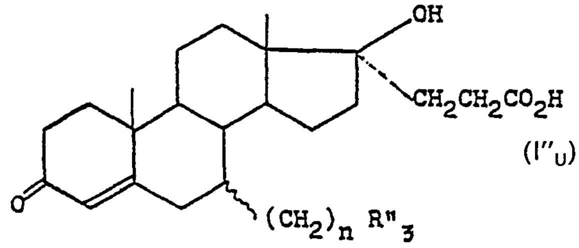

(I"$_U$)

dans laquelle R"$_3$ a la signification de $R_3$ indiquée à la revendication 1, à l'exception de la valeur carboxy salifié.

13. Procédé de préparation des produits de formule (I') telle que définie à l'une des revendications 1 ou 4, dans laquelle Ra représente un atome d'hydrogène, caractérisé en ce que l'on soumet un produit de formule ($II_2$):

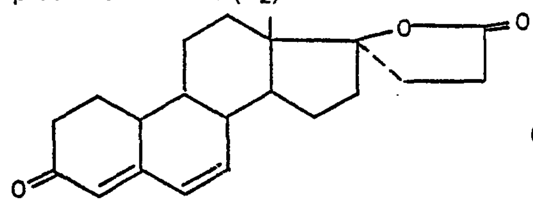

($II_2$)

soit à l'action d'un produit de formule ($III_2$):

$R_5-MgX'$ ($III_2$)

en présence d'un sel de cuivre, formule ($III_2$) dans laquelle $R_5$ représente $R_1$ qui est défini comme à la revendication 1, ou le radical $R_4-(CH_2)_n-$ défini comme à la revendication 12, et X' représente un atome d'halogène,

soit à l'action d'un produit de formule (III'$_2$):

$(R_5)_2CuLi$ (III'$_2$)

dans laquelle $R_5$ est défini comme ci-dessus, pour obtenir, après l'action d'un acide, un produit de formule (I"$_{A1}$):

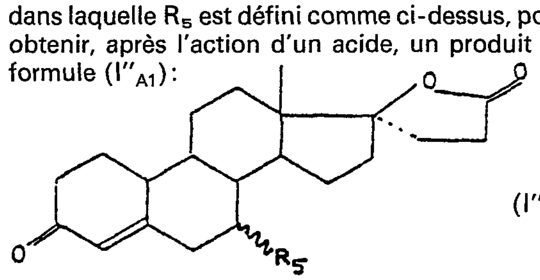

(I"$_{A1}$)

sous la forme, éventuellement, d'un mélange d'isomères 7α et 7β, mélange que, si désiré, l'on sépare, et, si désiré, soumet chaque isomère, ou le mélange des produits dans lesquels $R_5$ représente un radical $-(CH_2)_n-R_4$, dans lequel $R_4$ représente un radical hydroxyle protégé ou un radical amino protégé, à l'action d'un agent d'hydrolyse pour obtenir un produit de formule (I"$_{B1}$):

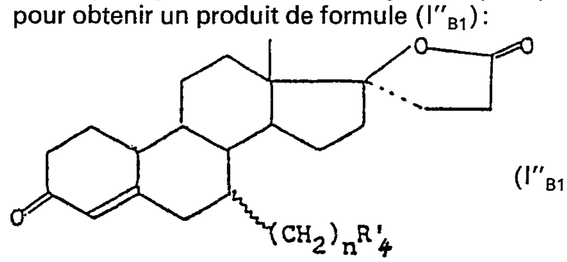

(I"$_{B1}$)

dans laquelle $R'_4$ représente un radical hydroxyle ou amino, et produit de formule $(I''_{B1})$ dans laquelle $R'_4$ représente un radical hydroxyle que l'on traite, le cas échéant,

soit, lrsque n est un entier supérieur à 1, par un réactif d'oxydation, pour obtenir un produit de formule $(I''_{C1})$ :

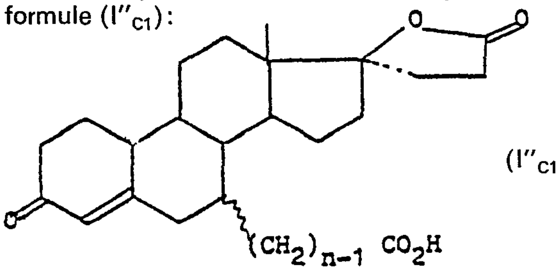

$(I''_{C1})$

produit que, le cas échéant, l'on estérifie ou salifie selon les méthodes usuelles,

soit par un réactif d'halogénation, pour obtenir un produit de formule $(I''_{D1})$ :

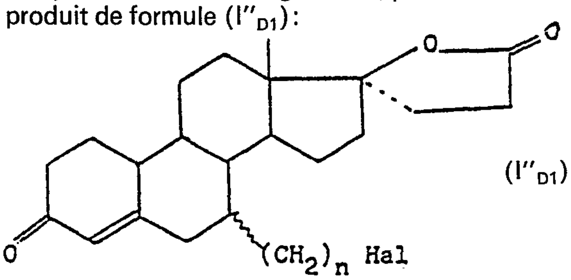

$(I''_{D1})$

dans laquelle Hal représente un atome d'halogène et que, si désiré, l'on soumet les produits de formules $(I''_{A1})$, $(I''_{B1})$, $(I''_{C1})$ et $(I''_{D1})$ sous forme d'isomères 7α ou 7β ou de leur mélange, à l'action d'un hydroxyde alcalin ou de l'ammoniaque, pour obtenir les composés de formule $(I''_{T1})$ :

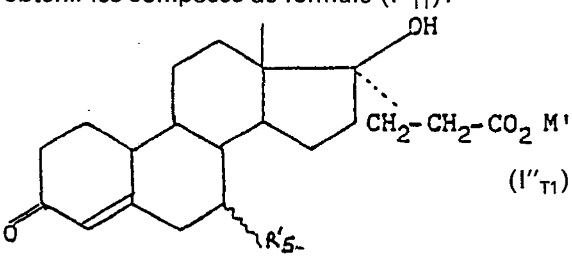

$(I''_{T1})$

dans laquelle $R'_5$ représente $R_1$ qui est défini comme ci-dessus, ou le radical $-(CH_2)_n-R'_3$, dans lequel $R'_3$ a la signification de $R_3$ indiquée ci-dessus à la revendication 1, à l'exception de la valeur carboxy libre, et M' représente un atome de métal alcalin ou un radical ammonium, sous forme d'isomères 7α ou 7β ou de leur mélange, que l'on sépare, si désiré, en chacun de ses isomères, puis soumet, si désiré, soit chacun des isomères, soit leur mélange, à l'action d'un agent acide, pour obtenir un composé de formule $(I''_{U1})$ :

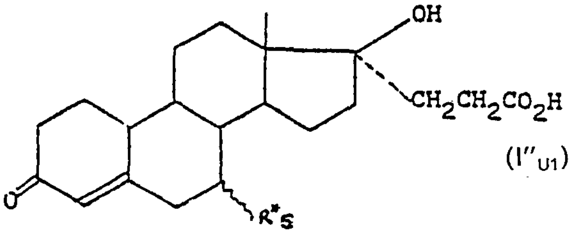

$(I''_{U1})$

dans laquelle $R''_5$ représente $R_1$ qui est défini comme ci-dessus, ou le radical $-(CH_2)_n-R''_3$, dans lequel $R''_3$ a la signification de $R_3$ indiquée à la revendication 1, à l'exception de la valeur carboxy salifié.

14. Procédé de préparation des produits de formule (I') telle que définie à l'une des revendications 1 ou 5, caractérisé en ce que l'on soumet un composé de formule (IV) :

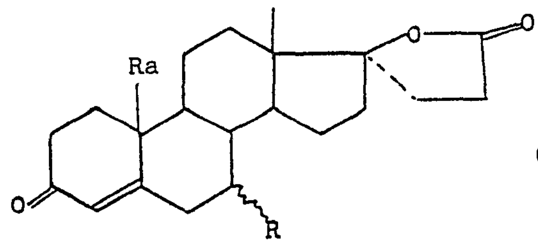

(IV)

dans laquelle R représente soit un radical $R_1$, $R_1$ étant un radical alkyle, alkényle ou alkynyle ayant au plus 8 atomes de carbone, soit un radical $R_2$, $R_2$ étant un radical $-(CH_2)_n-R_3$ dans lequel n représente un entier de 1 à 8, et $R_3$ représente un radical hydroxyle libre ou protégé, un radical amino, amino protégé, mono- ou dialkylamino, un radical carboxy libre, estérifié ou salifié ou un atome d'halogène, et Ra a la signification indiquée à la revendication 1, sous la forme, éventuellement, d'un mélange d'isomères 7α et 7β, à l'action d'un agent de déshydrogénation, pour obtenir un produit de formule $(I'''_A)$ :

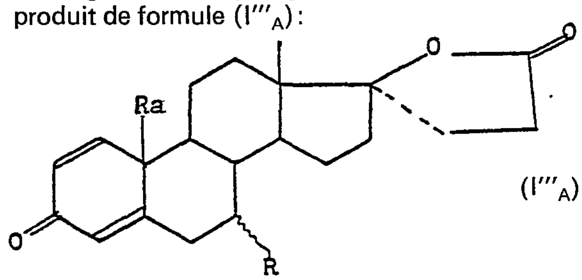

$(I'''_A)$

dans laquelle Ra et R ont la signification précédente, sous la forme, éventuellement, d'un mélange d'isomères 7α et 7β que l'on sépare, si désiré, puis soumet, si désiré, soit chacun des isomères, soit leur mélange, à l'action d'un hydroxyde alcalin ou de l'ammoniaque, pour obtenir les composés de formule $(I'''_B)$ :

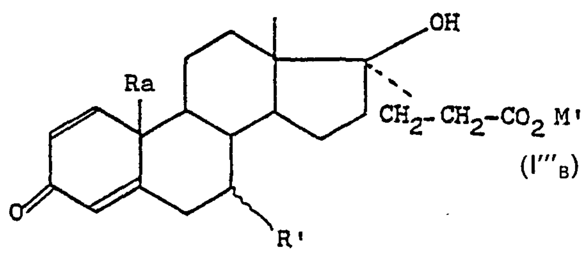

$(I'''_B)$

dans laquelle Ra a la signification précédente, M' représente un atome de métal alcalin ou le groupement $NH_4$ et R' a la signification de R à l'exception de la valeur $(CH_2)_n-R_3$ dans laquelle $R_3$ représente un radical carboxy libre, produit de formule $(I'''_B)$ sous forme d'ismères 7α ou 7β ou de leur mélange, que l'on sépare, si désiré, en chacun des isomères, puis soumet, si désiré, soit chacun des isomères, soit leur mélange, à l'action d'un agent acide, pour obtenir un produit de formule $(I'''_C)$ :

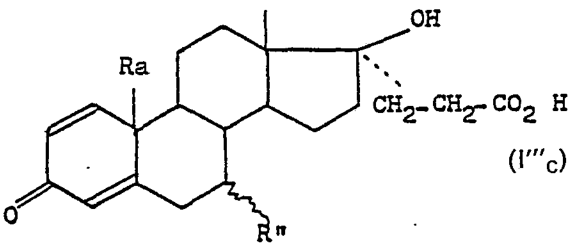

(I'''$_C$)

dans laquelle Ra a la signification précédente et R'' a la signification de R à l'exception de la valeur $(CH_2)_n-R_3$ dans laquelle $R_3$ représente un radical carboxy salifié.

15. A titre de médicaments, les produits de formule (I') telle que définie à la revendication 1.

16. A titre de médicaments, les produits de formule (I) telle que définie à la revendication 2.

17. A titre de médicaments, les produits de formule (I'') selon l'une des revendications 3, 4, 6, 7, 8 ou 10.

18. A titre de médicaments, les produits de formule (I''') selon l'une des revendications 5 à 10.

19. A titre de médicaments, l'un des produits décrits à la revendication 11.

20. Les compositions pharmaceutiques renfermant comme principe actif au moins un médicament défini à l'une des revendications 15 à 19.

**Revendications** pour l'Etat contractant: AT

1. Procédé de préparation des produits de formule générale (I'):

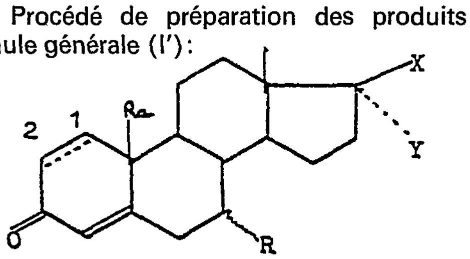

(I')

dans laquelle R représente soit un radical $R_1$, $R_1$ étant un radical alkyle, alkényle ou alkynyle ayant au plus 8 atomes de carbone, soit un radical $R_2$, $R_2$ étant un radical $-(CH_2)_nR_3$ dans lequel n représente un entier de 1 à 8 et $R_3$ représente un radical hydroxyle libre ou protégé, un radical amino, amino protégé, mono- ou dialkylamino, un radical carboxy libre, estérifié ou salifié ou un atome d'halogène,

X et Y représentent ensemble le groupement

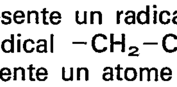

,

ou bien X représente un radical hydroxyle et Y représente le radical $-CH_2-CH_2-CO_2M$ dans lequel M représente un atome d'hydrogène, un atome de métal alcalin ou un radical ammonium, Ra représente un atome d'hydrogène ou un radical méthyle, le pointillé en position 1(2) indique la présence éventuelle d'une seconde liaison entre les carbones 1 et 2 et le trait ondulé signifie que le substituant R peut se trouver dans l'une des deux positions possibles α ou β, étant entendu que R ne peut pas représenter $R_1$ ou $R_2$ dans lequel n est égal à 1 et $R_3$ représente un atome d'halogène, lorsque la liaison 1(2) est une simple liaison et que Ra représente un radical méthyle, que $R_3$ ne peut pas représenter un radical carboxy libre lorsque Y représente le radical $-CH_2-CH_2-CO_2M$ dans lequel M représente un atome de métal alcalin ou un radical ammonium et que $R_3$ ne peut pas représenter un radical carboxy salifié lorsque Y représente le radical $-CH_2-CH_2-CO_2H$, caractérisé en ce que:

a) pour préparer les produits de formule (I'), telle que définie ci-dessus, dans laquelle Ra représente un radical méthyle, R représente $R_2$ et le trait pointillé en position 1(2) représente une simple liaison, l'on soumet un produit de formule ($II_1$):

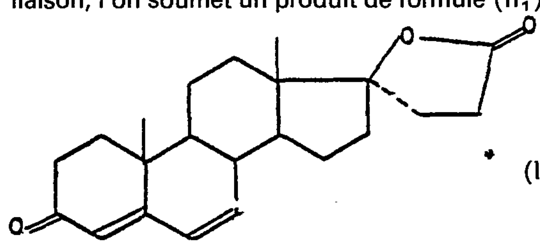

($II_1$)

soit à l'action d'un produit de formule ($III_1$):

$$R_4-(CH_2)_n-MgX' \qquad (III_1)$$

en présence d'un sel de cuivre, formule ($III_1$) dans laquelle X' représente un atome d'halogène, $R_4$ représente un radical hydroxyle protégé, un radical mono- ou dialkylamino ou un radical amino protégé et n a la signification indiquée précédemment,

soit, à l'action d'un produit de formule ($III'_1$):

$$[R_4(CH_2)_n]_2CuLi \qquad (III'_1)$$

dans laquelle $R_4$ et n conservent la même signification, pour obtenir, après action d'un acide, un produit de formule ($I''_A$):

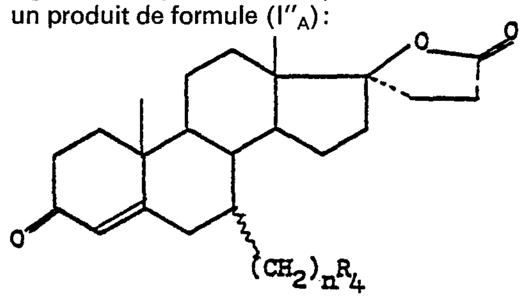

($I''_A$)

sous la forme, éventuellement, d'un mélange d'isomères 7α et 7β, mélange que, si désiré, l'on sépare, et, si désiré, soumet chaque isomère ou le mélange des produits dans lesquels $R_4$ représente un radical hydroxyle protégé ou un radical amino protégé, à l'action d'un agent d'hydrolyse, pour obtenir un produit de formule ($I''_B$):

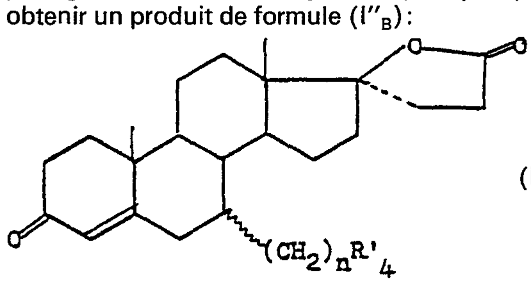

($I''_B$)

dans laquelle $R_4$ représente un radical hydroxyle ou amino, et un produit de formule ($I''_B$) dans

laquelle $R'_4$ représente un radical hydroxyle que l'on traite, le cas échéant,

soit, lorsque n est un entier supérieur à 1 par un réactif d'oxydation, pour obtenir un produit de formule ($I''_C$):

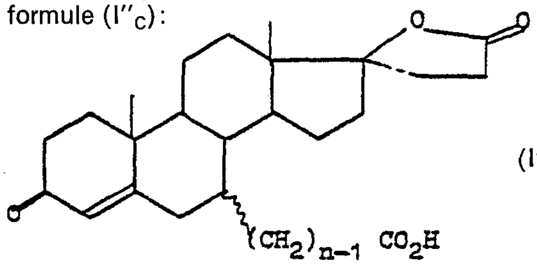

($I''_C$)

produit que, le cas échéant, l'on estérifie ou salifie selon les méthodes usuelles,

soit, à l'exception du cas où n est égal à 1, par un réactif d'halogénation, pour obtenir un produit de formule ($I''_D$):

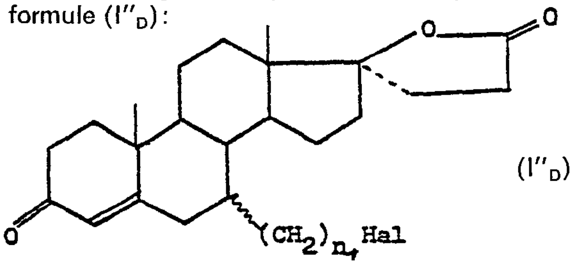

($I''_D$)

dans laquelle $n_1$ représente un entier de 2 à 8 et Hal représente un atome d'halogène et que, si désiré, l'on soumet les produits de formules ($I''_A$), ($I''_B$), ($I''_C$) et ($I''_D$) sous forme d'isomères 7α ou 7β ou de leur mélange, à l'action d'un hydroxyde alcalin ou de l'ammoniaque, pour obtenir les composés de formule($I''_T$):

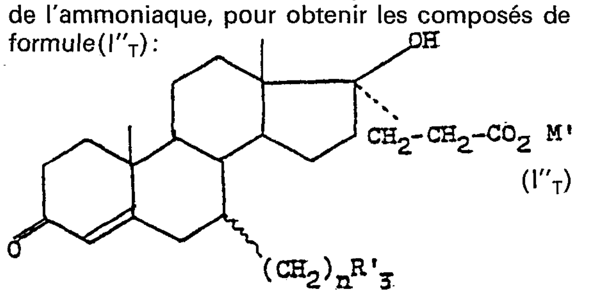

($I''_T$)

dans laquelle $R'_3$ a la signification de $R_3$ indiquée précédemment à l'exception de la valeur carboxy libre et M' représente un atome de métal alcalin ou un radical ammonium, sous forme d'isomères 7α ou 7β ou de leur mélange, que l'on sépare, si désiré, en chacun de ses isomères, puis soumet, si désiré, soit chacun des isomères, soit leur mélange, à l'action d'un agent acide, pour obtenir un composé de formule ($I''_U$):

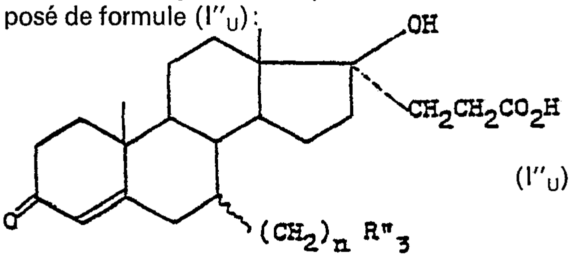

($I''_U$)

dans laquelle $R''_3$ a la signification de $R_3$ indiquée précédemment, à l'exception de la valeur carboxy salifié;

b) pour préparer les produits de formule (I'), telle que définie ci-dessus, dans laquelle Ra représente un atome d'hydrogène et le trait pointillé en position 1(2) représente une simple liaison, l'on soumet un produit de formule ($II_2$):

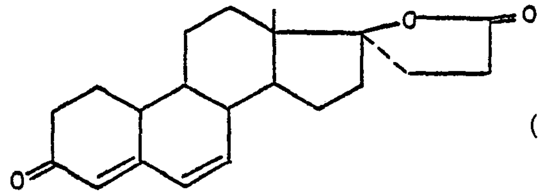

($II_2$)

soit à l'action d'un produit de formule ($III_2$):

$R_5-MgX'$ ($III_2$)

en présence d'un sel de cuivre, formule ($III_2$) dans laquelle $R_5$ représente $R_1$ qui est défini comme précédemment ou le radical $R_4-(CH_2)_n-$ défini comme précédemment et X' représente un atome d'halogène,

soit à l'action d'un produit de formule ($III'_2$):

$(R_5)_2CuLi$ ($III'_2$)

dans laquelle $R_5$ est défini comme ci-dessus, pour obtenir, après l'action d'un acide, un produit de formule ($I''_{A1}$):

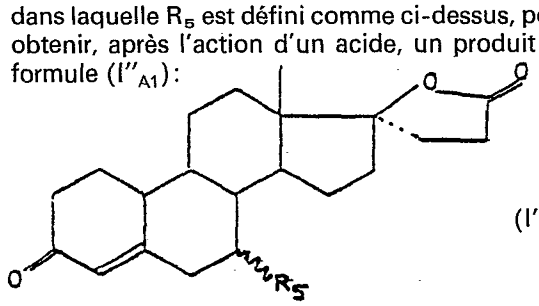

($I''_{A1}$)

sous la forme, éventuellement, d'un mélange d'isomères 7α et 7β, mélange que, si désiré, l'on sépare, et, si désiré, soumet chaque isomère ou le mélange des produits dans lesquels $R_5$ représente un radical $-(CH_2)_n-R_4$, dans lequel $R_4$ représente un radical hydroxyle protégé ou un radical amino protégé, à l'action d'un agent d'hydrolyse pour obtenir un produit de formule ($I''_{B1}$):

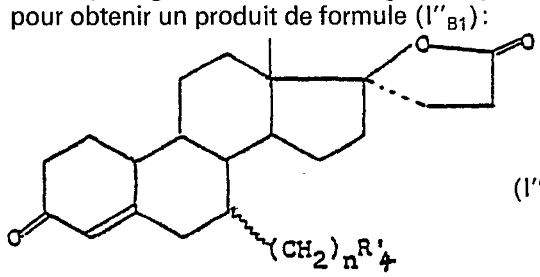

($I''_{B1}$)

dans laquelle $R'_4$ représente un radical hydroxyle ou amino, et produit de formule ($I''_{B1}$) dans laquelle $R'_4$ représente un radical hydroxyle que l'on traite, le cas échéant,

soit lorsque n est un entier supérieur à 1, par un réactif d'oxydation, pour obtenir un produit de formule ($I''_{C1}$):

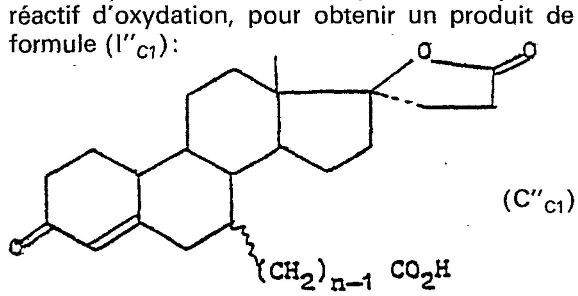

($C''_{C1}$)

produit que, le cas échéant, l'on estérifie ou salifie selon les méthodes usuelles,

soit par un réactif d'halogénation, pour obtenir un produit de formule ($I''_{D1}$):

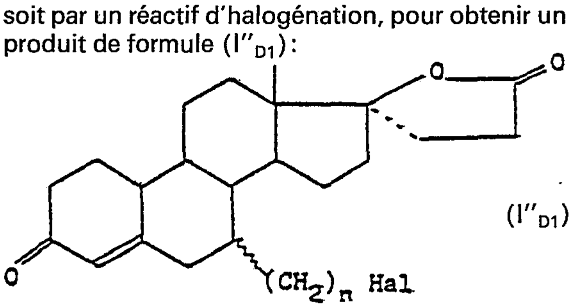

($I''_{D1}$)

dans laquelle Hal représente un atome d'halogène et que, si désiré, l'on soumet les produits de formules ($I''_{A1}$, ($I''_{B1}$), ($I''_{C1}$) et ($I''_{D1}$) sous forme d'isomères 7α ou 7β ou de leur mélange, à l'action d'un hydroxyde alcalin ou de l'ammoniaque, pour obtenir les composés de formule ($I''_{T1}$):

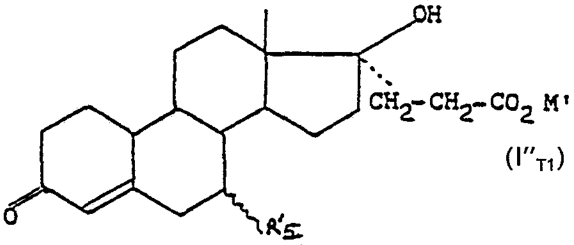

($I''_{T1}$)

dans laquelle $R'_5$ représente $R_1$ qui est défini comme ci-dessus, ou le radical $-(CH_2)_n-R'_3$, dans lequel $R'_3$ a la signification de $R_3$ indiquée précédemment à l'exception de la valeur carboxy libre et M' représente un atome de métal alcalin ou un radical ammonium, sous forme d'isomères 7α ou 7β ou de leur mélange, que l'on sépare, si désiré, en chacun de ses isomères, puis soumet, si désiré, soit chacun des isomères, soit leur mélange, à l'action d'un agent acide, pour obtenir un composé de formule ($I''_{U1}$):

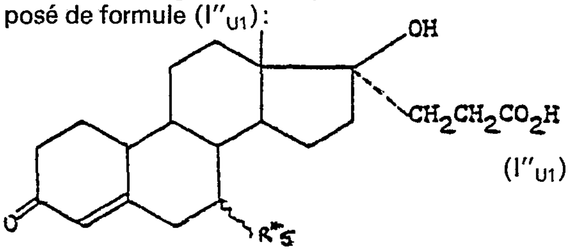

($I''_{U1}$)

dans laquelle $R''_5$ représente $R_1$ qui est défini comme ci-dessus, ou le radical $-(CH_2)_n-R''_3$, dans lequel $R''_3$ a la signification de $R_3$ indiquée précédemment à l'exception de la valeur carboxy salifié;

c) pour préparer les produits de formule (I'), telle que définie ci-dessus, dans laquelle le trait pointillé en 1(2) représente une double liaison, l'on soumet un composé de formule (IV):

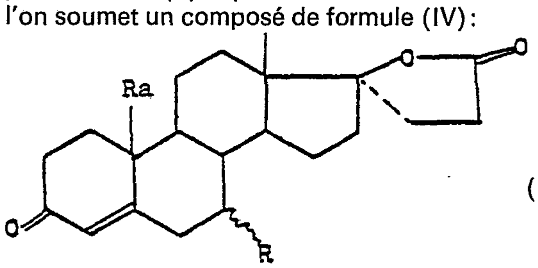

(IV)

dans laquelle R représente soit un radical $R_1$, $R_1$ étant un radical alkyle, alkényle ou alkynyle ayant au plus 8 atomes de carbone, soit un radical $R_2$, $R_2$ étant un radical $-(CH_2)_n-R_3$ dans lequel n représente un entier de 1 à 8 et $R_3$ représente un radical hydroxyle libre ou protégé, un radical amino, amino protégé, mono- ou dialkylamino, un radical carboxy libre, estérifié ou salifié ou un atome d'halogène et Ra a la signification indiquée précédemment sous forme, éventuellement, d'un mélange d'isomères 7α et 7β, à l'action d'un agent de déshydrogénation, pour obtenir un produit de formule ($I'''_A$):

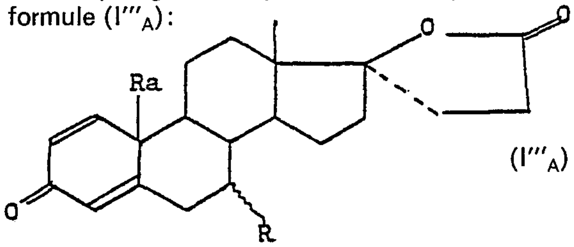

($I'''_A$)

dans laquelle Ra et R ont la signification précédente, sous la forme, éventuellement, d'un mélange d'isomères 7α et 7β que l'on sépare, si désiré, puis soumet, si désiré, soit chacun des isomères, soit leur mélange, à l'action d'un hydroxyde alcalin ou de l'ammoniaque, pour obtenir les composés de formule ($I'''_B$):

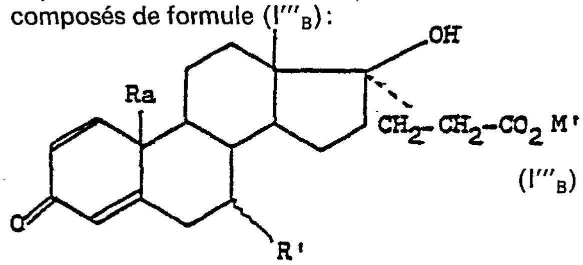

($I'''_B$)

dans laquelle Ra a la signification précédente, M' représente un atome de métal alcalin ou le groupement $NH_4$ et R' a la signification de R à l'exception de la valeur $(CH_2)_n-R_3$ dans laquelle $R_3$ représente un radical carboxy libre, produit de formule ($I'''_B$) sous forme d'isomères 7α ou 7β ou de leur mélange, que l'on sépare, si désiré, en chacun des isomères, puis soumet, si désiré, soit chacun des isomères soit leur mélange à l'action d'un agent acide, pour obtenir un produit de formule ($I'''_C$):

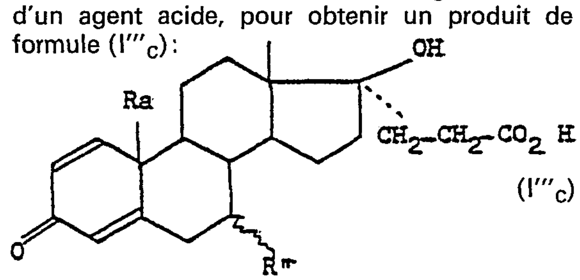

($I'''_C$)

dans laquelle Ra a la signification précédente et R'' a la signification de R à l'exception de la valeur $(CH_2)_n-R_3$ dans laquelle $R_3$ représente un radical carboxy salifié.

2. Procédé de préparation des produits de formule (I') telle que définie à la revendication 1, répondant à la formule (I):

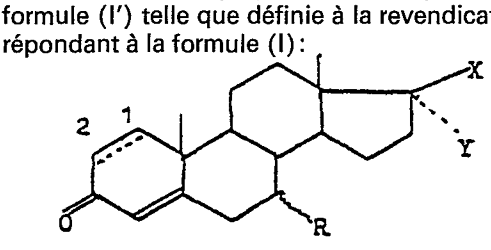

(I)

dans laquelle R, X, Y, le trait pointillé et le trait ondulé ont la signification indiquée à la revendication 1, étant entendu que R ne peut pas représenter $R_1$ ou $R_2$ dans lequel n est égal à 1 et $R_3$ représente un atome d'halogène, lorsque la liaison 1(2) est une simple liaison, que $R_3$ ne peut pas représenter un radical carboxy libre, lorsque Y représente le radical $-CH_2-CH_2-CO_2M$ dans lequel M représente un atome de métal alcalin ou un radical ammonium et que $R_3$ ne peut pas représenter un radical carboxy salifié lorsque Y représente le radical $-CH_2-CH_2-CO_2H$, caractérisé en ce que:

a) pour préparer les produits de formule (I), telle que définie ci-dessus, dans laquelle R représente $R_2$ et le trait pointillé en position 1(2) représente une simple liaison, l'on soumet un produit de formule ($II_1$):

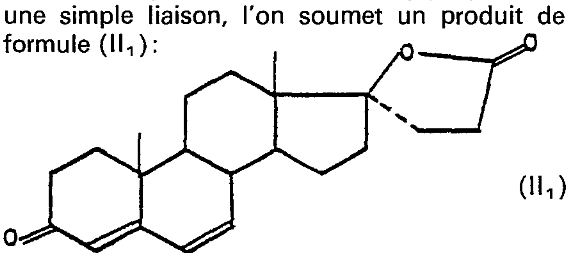

($II_1$)

soit à l'action d'un produit de formule ($III_1$):

$$R_4-(CH_2)_n-MgX' \qquad (III_1)$$

en présence d'un sel de cuivre, formule ($III_1$) dans laquelle X' représente un atome d'halogène, $R_4$ représente un radical hydroxyle protégé, un radical mono- ou dialkylamino ou un radical amino protégé et n a la signification indiquée précédemment,

soit à l'action d'un produit de formule ($III'_1$):

$$[R_4(CH_2)_n]_2CuLi \qquad (III'_1)$$

dans laquelle $R_4$ et n conservent la même signification pour obtenir, après action d'un acide, un produit de formule :

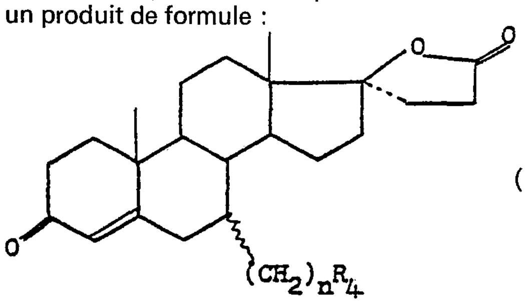

($I''_A$)

sous la forme, éventuellement, d'un mélange d'isomères 7α et 7β, mélange que, si désiré, l'on sépare, et, si désiré, soumet chaque isomère ou le mélange des produits dans lesquels $R_4$ représente un radical hydroxyle protégé ou un radical amino protégé, à l'action d'un agent d'hydrolyse, pour obtenir un produit de formule ($I''_B$):

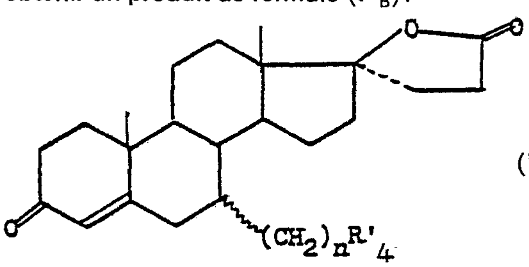

($I''_B$)

dans laquelle $R'_4$ représente un radical hydroxyle ou amino, et produit de formule ($I''_B$) dans laquelle $R'_4$ représente un radical hydroxyle que l'on traite, le cas échéant,

soit lorsque n est un entier supérieur à 1 par un réactif d'oxydation, pour obtenir un produit de formule ($I''_C$):

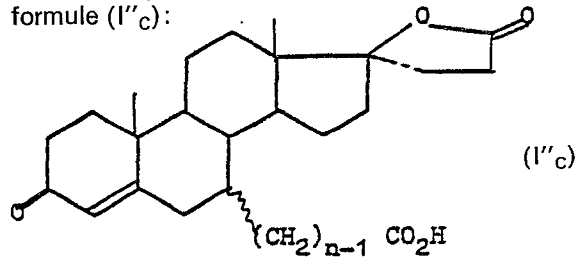

($I''_C$)

produit que, le cas échéant, l'on estérifie ou salifie selon les méthodes usuelles,

soit à l'exception du cas où n est égal à 1, par réactif d'halogénation, pour obtenir un produit de formule ($I''_D$):

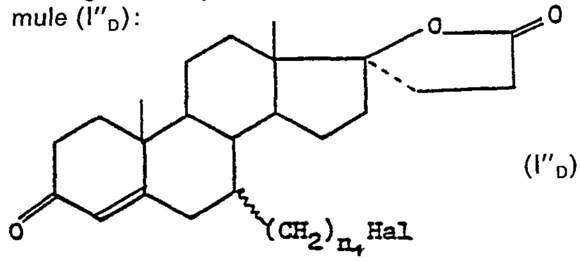

($I''_D$)

dans laquelle $n_1$ représente un entier de 2 à 8 et Hal représente un atome d'halogène et que, si désiré, l'on soumet les produits de formules ($I''_A$), ($I''_B$), ($I''_C$) et ($I''_D$) sous forme d'isomères 7α ou 7β ou de leur mélange, à l'action d'un hydroxyde alcalin ou de l'ammoniaque, pour obtenir les composés de formule ($I''_T$):

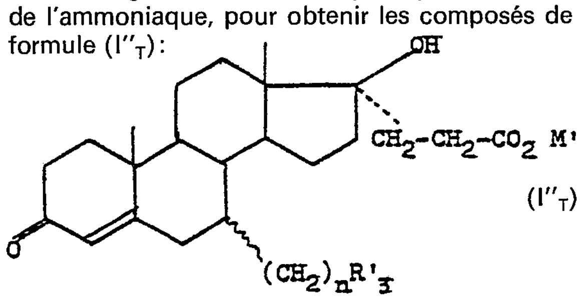

($I''_T$)

dans laquelle $R'_3$ a la signification de $R_3$ indiquée précédemment à l'exception de la valeur carboxy libre et M' représente un atome de métal alcalin ou un radical ammonium, sous forme d'isomères 7α ou 7β ou de leur mélange, que l'on sépare, si désiré, en chacun de ses isomères, puis soumet, si désiré, soit chacun des isomères, soit leur mélange, à l'action d'un agent acide, pour obtenir un composé de formule ($I''_U$):

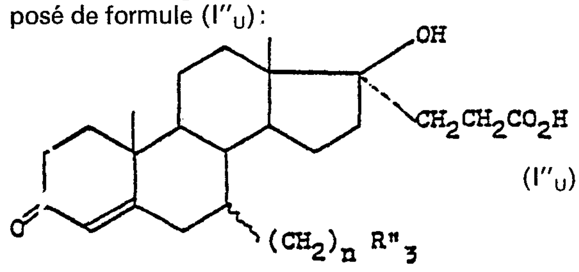

($I''_U$)

dans laquelle $R''_3$ a la signification de $R_3$ indiquée précédemment, à l'exception de la valeur carboxy salifié;

b) pour préparer les produits de formule (I), telle que définie ci-dessus, dans laquelle le trait pointillé en position 1(2) représente une double liaison, l'on soumet un composé de formule ($IV_1$):

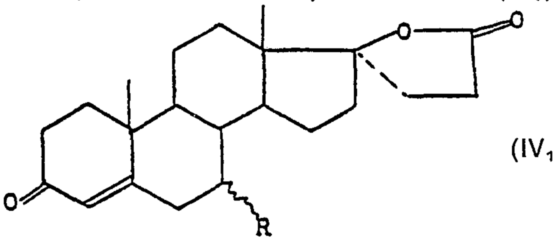

($IV_1$)

dans laquelle R a la signification indiquée à la revendication 1, sous la forme, éventuellement, d'un mélange d'isomères 7α et 7β, à l'action d'un agent de déshydrogénation, pour obtenir un produit de formule ($I'''_{A1}$):

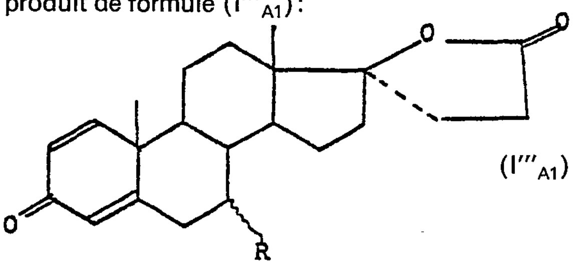

($I'''_{A1}$)

dans laquelle R a la signification précédente, sous la forme, éventuellement, d'un mélange d'isomères 7α et 7β que l'on sépare, si désiré, puis soumet soit chacun des isomères, soit leur mélange, à l'action d'un hydroxyde alcalin ou de l'ammoniaque, pour obtenir les composés de formule ($I'''_{B1}$):

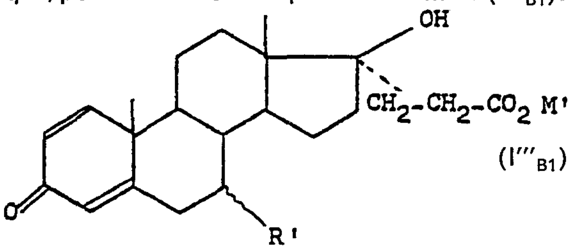

($I'''_{B1}$)

dans laquelle M' représente un atome de métal alcalin ou le groupement $NH_4$ et R' a la signification de R à l'exception de la valeur $(CH_2)_nR_3$ dans laquelle $R_3$ représente un radical carboxy libre, produit de formule ($I'''_{B1}$) sous forme d'isomères 7α ou 7β ou de leur mélange, que l'on sépare, si désiré, en chacun des isomères, puis soumet soit chacun des isomères, soit leur mélange, à l'action d'un agent acide, pour obtenir un produit de formule ($I'''_{C1}$):

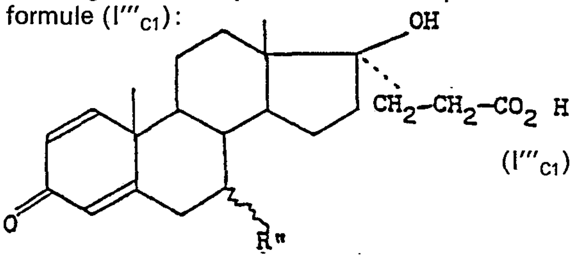

($I'''_{C1}$)

dans laquelle R" a la signification de R à l'exception de la valeur $(CH_2)_n-R_3$ dans laquelle $R_3$ représente un radical carboxy salifié.

3. Procédé selon la revendication 1, caractérisé en ce que l'on soumet les produits de formules ($I''_A$), ($I''_B$), ($I''_C$) et ($I''_D$) ou les produits de formules ($I''_{A1}$), ($I''_{B1}$), ($I''_{C1}$) et ($I''_{D1}$) ou les produits de formule ($I'''_A$) sous forme d'isomères 7α ou 7β ou de leur mélange, à l'action de l'hydroxyde de potassium, pour obtenir les sels de potassium correspondants.

4. Procédé selon la revendication 1, caractérisé en ce que le radical R est dans la position 7α.

5. Procédé selon la revendication 1, caractérisé en ce que l'on utilise au départ un produit de formules ($III_2$), ($III'_2$) ou (IV) dans laquelle $R_5$ ou R représente un radical propyle, butyle, isobutyle ou butényle, étant entendu que Ra ne représente pas un radical méthyle lorsque la liaison 1(2) est une simple liaison.

6. Procédé selon l'une des revendications 3, 4 ou 5, caractérisé en ce que l'on utilise au départ un produit de formule (II) ou (IV) substitué en position 10 par un radical méthyle.

7. Procédé selon la revendication 2, caractérisé en ce que l'on prépare la γ-lactone de l'acide 17β-hydroxy 7α-propyl 3-oxo 17α-pregna 1,4-dièn 21-carboxylique et le 17β-hydroxy 3-oxo 7α-propyl 17α-pregna 1,4-dièn 21-carboxylate de potassium.

**Patentansprüche** für die Vertragsstaaten BE, CH, DE, FR, GB, IT, LI, LU, NL, SE

1. Produkte der allgemeinen Formel (I'):

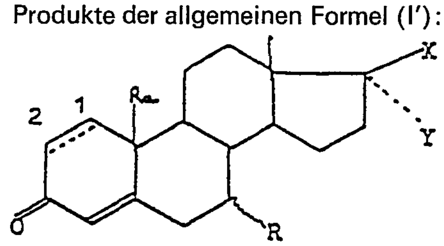

(I')

worin R entweder einen Rest $R_1$ bedeutet, wobei $R_1$ einen Alkyl-, Alkenyl- oder Alkinylrest mit höchstens 8 Kohlenstoffatomen darstellt, oder einen Rest $R_2$ bedeutet, wobei $R_2$ einen Rest $-(CH_2)_nR_3$ darstellt, worin n eine ganze Zahl von 1 bis 8 bedeutet und $R_3$ einen freien oder geschützten Hydroxylrest, einen Aminorest, einen geschützten Aminorest, einen Mono- oder Dialkylaminorest, einen freien, veresterten oder in ein Salz überführten Carboxyrest oder ein Halogenatom bedeutet, X und Y gemeinsam die gruppe

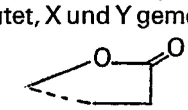

bedeuten oder X einen Hydroxylrest bedeutet und Y den Rest $-CH_2-CH_2-CO_2M$ darstellt, worin M ein Wasserstoffatom, ein Alkalimetallatom oder einen Ammoniumrest darstellt, Ra ein Wasserstoffatom oder einen Methylrest bedeutet, die gestrichelte Linie in 1(2)-Stellung die eventuelle Anwesenheit einer zweiten Bindung zwischen den 1 und 2 Kohlenstoffatomen anzeigt und die gewellte Linie anzeigt, dass der Substituent R sich in einer der beiden möglichen α- oder β-Positionen befinden kann, mit der Massgabe, das R nicht $R_1$ oder $R_2$, worin n für 1 steht und $R_3$ ein Halogenatom bedeutet, sein kann, wenn die 1(2)-Bindung eine

Einfachbindung darstellt, und wenn Ra einen Methylrest bedeutet, dass $R_3$ keine freie Carboxygruppe bedeuten kann, wenn Y den Rest $-CH_2-CH_2-CO_2M$ darstellt, worin M ein Alkalimetallatom oder einen Ammoniumrest bedeutet, und dass $R_3$ keine in ein Salz übergeführte Carboxygruppe bedeuten kann, wenn Y den Rest $-CH_2-CH_2-CO_2H$ darstellt.

2. Produkte der allgemeinen Formel (I') gemäss Anspruch 1 der allgemeinen Formel (I):

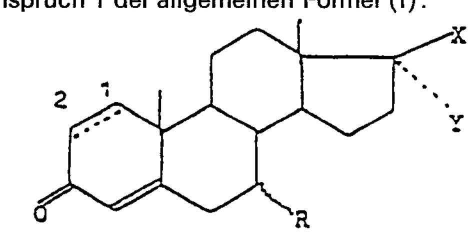

(I)

worin R, X, Y, die gestrichelte Linie und die gewellte Linie die in Anspruch 1 angegebene Bedeutung besitzen, mit der Massgabe, dass R nicht $R_1$ oder $R_2$, worin n für 1 steht und $R_3$ ein Halogenatom bedeutet, sein kann, wenn die 1(2)-Bindung eine Einfachbindung ist, dass $R_3$ keinen freien Carboxyrest bedeuten kann, wenn Y den Rest $-CH_2-CH_2-CO_2M$ bedeutet, worin M ein Alkalimetallatom oder einen Ammoniumrest darstellt, und dass $R_3$ nicht eine in ein Salz übergeführte Carboxygruppe bedeuten kann, wenn Y den Rest $-CH_2-CH_2-CO_2H$ darstellt.

3. Produkte der Formel (I'') entsprechend den Produkten der Formel (I') gemäss Anspruch 1, worin Ra einen Methylrest bedeutet, R für $R_2$ steht und die gestrichelte Linie in 1(2)-Stellung eine Einfachbindung ist, mit der Massgabe, dass n nicht die Zahl 1 bedeutet, wenn $R_3$ ein Halogenatom darstellt.

4. Produkte der Formel (I'') entsprechend den Produkten der Formel (I') gemäss Anspruch 1, worin Ra ein Wasserstoffatom bedeutet, R die in Anspruch 1 angegebene Bedeutung besitzt und die gestrichelte Linie in 1(2)-Stellung eine Einfachbindung darstellt.

5. Produkte der Formel (I''') entsprechend den Produkten der Formel (I') gemäss Anspruch 1, worin die gestrichelte Linie in 1(2)-Stellung die Anwesenheit einer Doppelbindung anzeigt.

6. Produkte der Formel (I') gemäss einem der Ansprüche 1 bis 5, worin X und Y gemeinsam die gruppe

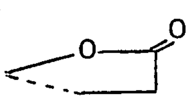 bedeuten.

7. Produkte der Formel (I') gemäss einem der Ansprüche 1 bis 5, worin X einen Hydroxylrest bedeutet und Y einen Rest $-CH_2-CH_2-CO_2K$ darstellt.

8. Produkte der Formel (I') gemäss einem der Ansprüche 1 bis 7, worin der Rest R sich in 7α-Stellung befindet.

9. Produkte der Formel (I') gemäss einem der Ansprüche 1, 2 und 4 bis 8, worin R einen Propyl-, Butyl-, Isobutyl- oder Butenylrest darstellt, mit der Massgabe, dass Ra keinen Methylrest darstellt, wenn die 1(2)-Bindung eine Einfachbindung ist.

10. Produkte der Formel (I') gemäss einem der Ansprüche 1 bis 8, worin R einen Chlorpropyl-, Hydroxypropyl-, Carboxyethyl-, Methoxycarbonylethylrest bedeutet.

11. γ-Lacton der 17β-Hydroxy-7α-propyl-3-oxo-17α-pregna-1,4-dien-21-carbonsäure;

Kalium-17β-hydroxy-3-oxo-7α-propyl-17α-pregna-1,4-dien-21-carboxylat.

12. Verfahren zur Herstellung der Produkte der Formel (I') gemäss einen der Ansprüche 1, 2 oder 3, worin Ra einen Methylrest bedeutet, dadurch gekennzeichnet, dass man ein Produkt der Formel ($II_1$):

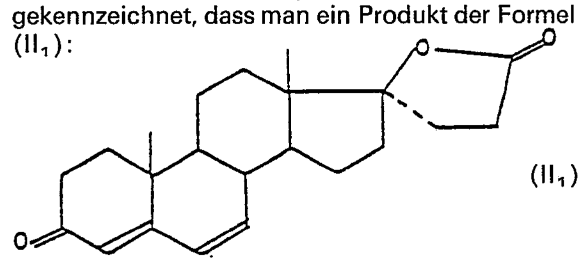

($II_1$)

entweder der Einwirkung eines Produktes der Formel ($III_1$):

$$R_4-(CH_2)_n-MgX' \qquad (III_1)$$

in Gegenwart eines Kupfersalzes unterzieht, wobei in der Fromel ($III_1$) X' ein Halogenatom bedeutet, $R_4$ einen geschützten Hydroxylrest, einen Mono- oder Dialkylaminorest oder einen geschützten Aminorest darstellt und n die in Anspruch 1 angegebene Bedeutung besitzt,
oder der Eiwirkung eines Produktes der Formel ($III'_1$):

$$[R_4(CH_2)_n]_2CuLi \qquad (III'_1)$$

unterzieht, worin $R_4$ und n die angegebene Bedeutung besitzen, um nach Einwirkung einer Säure ein Produkt der Formel ($I''_A$):

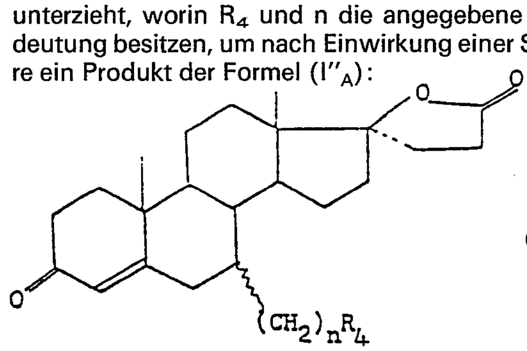

($I''_A$)

gegebenenfalls in Form eines Gemisches der 7α- und 7β-Isomeren, zu erhalten, welches Gemisch man gewünschtenfalls auftrennt und gewünschtenfalls ein jedes Isomeres oder das Gemisch der Produkte, worin $R_4$ einen geschützten Hydroxylrest oder einen geschützten Aminorest bedeutet, der Einwirkung eines Hydrolysemittels unterzieht, um ein Produkt der Formel ($I''_B$):

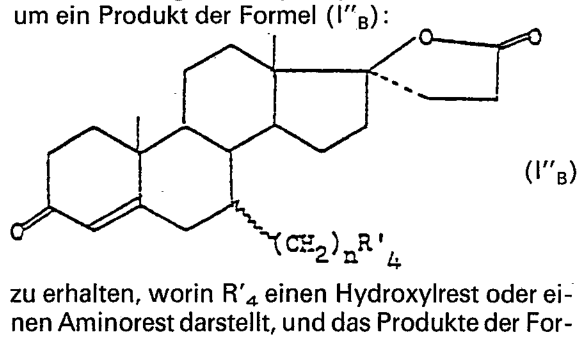

($I''_B$)

zu erhalten, worin $R'_4$ einen Hydroxylrest oder einen Aminorest darstellt, und das Produkte der Formel $(I''_B)$, worin $R'_4$ einen Hydroxylrest darstellt, man gegebenenfalls

entweder, wenn n eine ganze Zahl grösser als 1 ist, mit einem Oxidationsreagens behandelt, um ein Produkt der Formel $(I''_C)$:

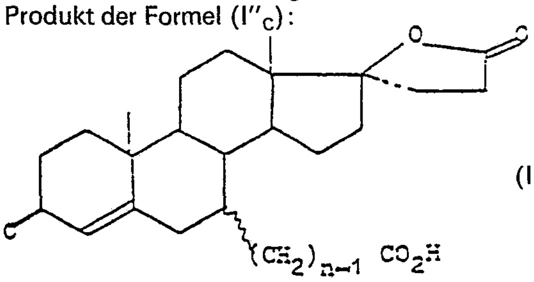

$(I''_C)$

zu erhalten, welches Produkt man gegebenenfalls nach üblichen Methoden verestert oder in ein Salz überführt,

oder mit Ausnahme des Falles, bei dem n gleich 1 ist, mit einem Halogenierungsreagens behandelt, um ein Produkt der Formel $(I''_D)$:

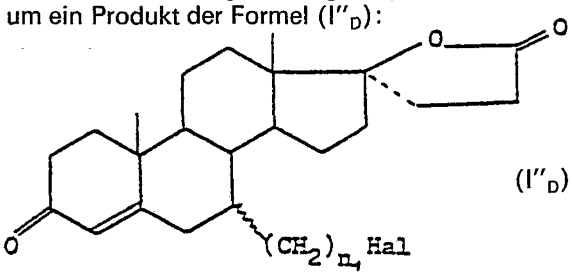

$(I''_D)$

zu erhalten, worin $n_1$ eine ganze Zahl von 2 bis 8 bedeutet und Hal ein halogenatom darstellt, und man gewünschtenfalls die Produkte der Formeln $(I''_A)$, $(I''_B)$, $(I''_C)$ und $(I''_D)$ in Form des 7α- oder 7β-Isomeren oder in Form von deren Gemisch der Einwirkung eines Alkalihydroxids oder von Ammoniak unterzieht, um die Verbindungen der Formel $(I''_T)$:

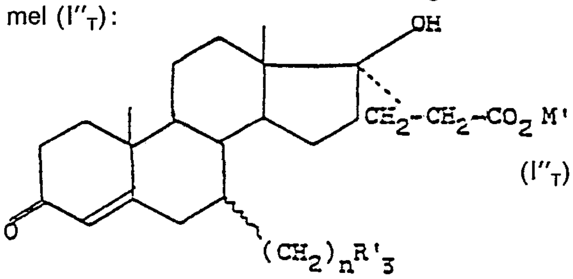

$(I''_T)$

worin $R'_3$ die in Anspruch 1 für $R_3$ angegebene Bedeutung mit Ausnahme derjenigen von freiem Carboxy besitzt und M' ein Alkalimetalltom oder einen Ammoniumrest bedeutet, in Form der 7α- oder 7β-Isomeren oder von deren Gemisch, das man gewünschtenfalls in ein jedes seiner Isomeren auftrennt, zu erhalten, danach gewünschtenfalls ein jedes der Isomeren oder deren Gemisch der Einwirkung eines sauren Mittels unterzieht, um eine Verbindung der Formel $(I''_U)$:

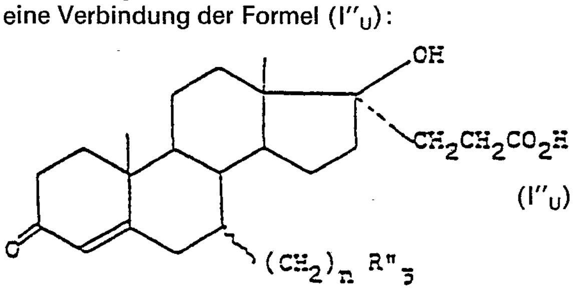

$(I''_U)$

zu erhalten, worin $R''_3$ die in Anspruch 1 für $R_3$ angegebene Bedeutung mit Ausnahme derjenigen von in ein Salz übergeführtem Carboxy besitzt.

13. Verfahren zur Herstellung der produkte der Formel (I') gemäss einem der beiden Ansprüche 1 oder 4, worin Ra ein Wasserstoffatom bedeutet, dadurch gekennzeichnet, dass man ein Produkt der Formel $(II_2)$:

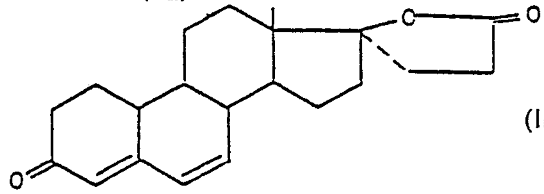

$(II_2)$

entweder der Einwirkung eines Produkts der Formel $(III_2)$:

$$R_5-MgX' \qquad (III_2)$$

in Gegenwart eines Kupfersalzes unterzieht, wobei in der Formel $(III_2)$ $R_5$ für $R_1$ steht, das wie in Anspruch 1 definiert ist, oder den Rest $R_4-(CH_2)_n-$, der wie in Anspruch 12 definiert ist, bedeutet und X' ein Halogenatom bedeutet,

oder der Einwirkung eines Produkts der Formel $(III'_2)$:

$$(R_5)_2CuLi \qquad (III'_2)$$

unterzieht, worin $R_5$ wie vorstehend definiert ist, um nach Einwirkung einer Säure ein Produkt der Formel $(I''_{A1})$:

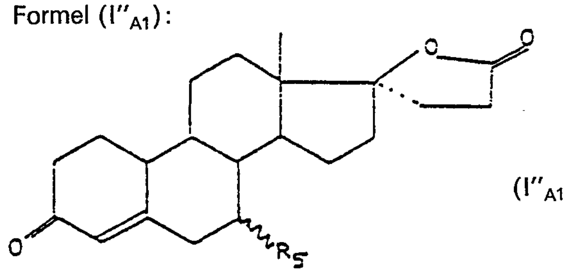

$(I''_{A1})$

gegebenenfalls in Form eines Gemisches der 7α- und 7β-Isomeren, das man gewünschtenfalls auftrennt, zu erhalten, und gewünschtenfalls ein jedes des Isomeres oder das Gemisch der Produkte, worin $R_5$ einen Rest $-(CH_2)_n-R_4$ darstellt, worin $R_4$ einen geschützten Hydroxylrest oder einen geschützten Aminorest bedeutet, der Einwirkung eines Hydrolysemittels unterzieht, um ein Produkt der Formel $(I''_{B1})$:

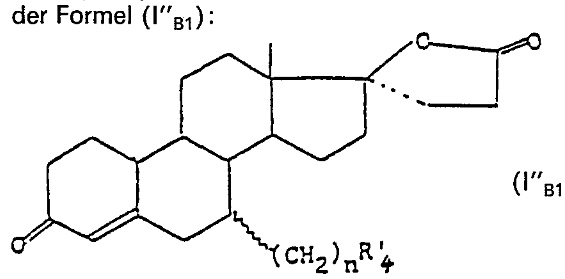

$(I''_{B1})$

zu erhalten, worin $R'_4$ einen Hydroxyl- oder Aminorest bedeutet, und das Produkt der Formel $(I''_{B1})$, worin $R'_4$ einen Hydroxylrest darstellt, man gegebenenfalls

entweder, wenn n eine ganze Zahl grösser als 1 ist, mit einem Oxidationsreagens behandelt, um eine Produkt der Formel $(I''_{C1})$:

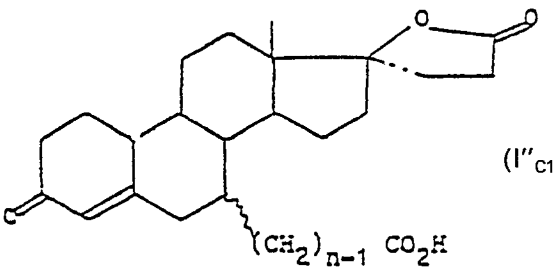

($I''_{C1}$)

zu erhalten, welches Produkt man gegebenenfalls nach üblichen Methoden verestert oder in ein Salz überführt,
oder mit einem halogenierungsreagens behandelt, um ein Produkt der Formel ($I''_{D1}$):

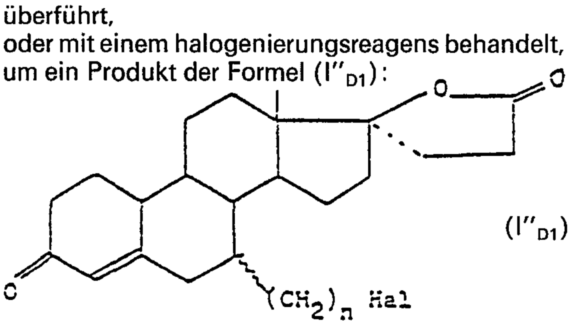

($I''_{D1}$)

zu erhalten, worin Hal ein Halogenatom bedeutet, und man gewünschtenfalls die Produkte der Formeln ($I''_{A1}$), ($I''_{B1}$), ($I''_{C1}$) und ($I''_{D1}$) in Form der 7α- oder 7β-Isomeren oder von deren Gemisch der Einwirkung eines Alkalihydroxids oder von Ammoniak unterzieht, um die Verbindungen der Formel ($I''_{T1}$):

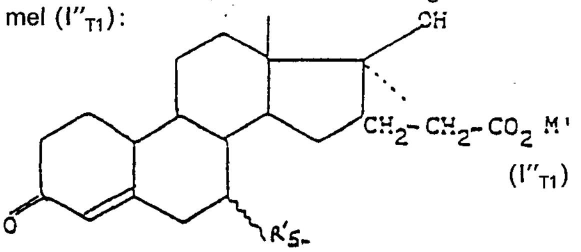

($I''_{T1}$)

worin $R'_5$ für $R_1$, das wie vorstehend definiert ist, oder den Rest $-(CH_2)_n-R'_3$ steht, worin $R'_3$ die vorstehend in Anspruch 1 für $R_3$ angegebene Bedeutung mit Ausnahme derjenigen von freiem Carboxy besitzt, und M' ein Alkalimetallatom oder einen Ammoniumrest bedeutet, in Form der 7α- oder 7β-Isomeren oder von deren Gemisch, das man gewünschtenfalls in ein jedes seiner Isomeren auftrennt, zu erhalten, danach gewünschtenfalls ein jedes der Isomeren oder deren Gemisch der Einwirkung eines sauren Mittels unterzieht, um eine Verbindung der Formel ($I''_{U1}$):

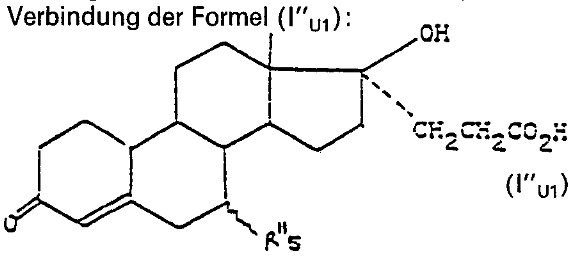

($I''_{U1}$)

zu erhalten, worin $R''_5$ für $R_1$, das wie vorstehend definiert ist, oder den Rest $-(CH_2)_n-R''_3$ steht, worin $R''_3$ die in Anspruch 1 angegebene Bedeutung von $R_3$ mit Ausnahme derjenigen von in ein Salz überführtem Carboxy besitzt.

14. Verfahren zur Herstellung der Produkte der Formel (I') gemäss einem der beiden Ansprüche 1 oder 5, dadurch gekennzeichnet, dass man eine Verbindung der Formel (IV):

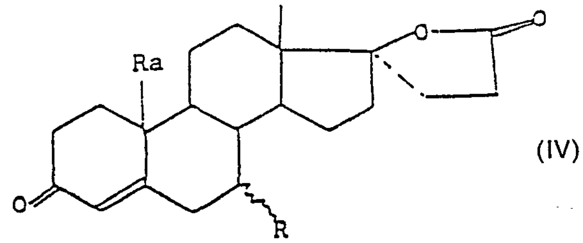

(IV)

worin R entweder einen Rest $R_1$, wobei $R_1$ einen Alkyl-, Alkenyl- oder Alkinylrest mit höchstens 8 Kohlenstoffatomen darstellt, oder einen Rest $R_2$ bedeutet, wobei $R_2$ einen Rest $-(CH_2)_n-R_3$ darstellt, worin n eine ganze Zahl von 1 bis 8 ist und $R_3$ einen freien oder geschützten Hydroxylrest, einen Aminorest, einen geschützten Aminorest, einen Mono- oder Dialkylaminorest oder einen freien, veresterten oder in ein Salz überführten Carboxyrest oder ein Halogenatom bedeutet, und Ra die in Anspruch 1 angegebene Bedeutung besitzt, gegebenenfalls in Form eines Gemisches der 7α- und 7β-Isomeren der Einwirkung eines Dehydrogenierungsmittels unterzieht, um ein Produkt der Formel ($I'''_A$):

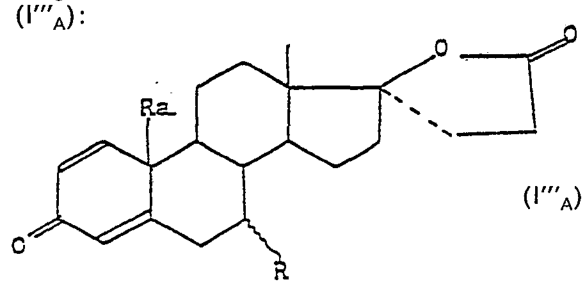

($I'''_A$)

worin Ra und R die vorstehend angegebene Bedeutung besitzen, gegebenenfalls in Form eines Gemisches der 7α- und 7β-Isomeren, das man gewünschtenfalls auftrennt, zu erhalten, danach gewünchtenfalls ein jedes der Isomeren oder deren Gemisch der Einwirkung eines Alkalihydroxids oder derjenigen von Ammoniak unterzieht, um die Verbindungen der Formel ($I'''_B$):

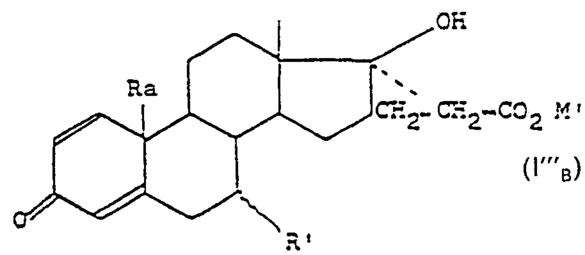

($I'''_B$)

worin Ra die vorstehende Bedeutung besitzt, M' ein Alkalimetallatom oder die gruppe $NH_4$ darstellt und R' die Bedeutung von R mit Ausnahme der Bedeutung $(CH_2)_n-R_3$ besitzt, worin $R_3$ einen freien Carboxyrest bedeutet, zu erhalten, das Produkt der Formel ($I'''_B$) in Form der 7α- oder 7β-Isomeren oder in Form von deren Gemisch, das man gewünschtenfalls in ein jedes der Isomeren auftrennt, danach gewünschtenfalls entweder ein jedes der Isomeren oder ein Gemisch der Einwirkung eines sauren Mittels unterzieht, um ein Produkt der Formel ($I'''_C$):

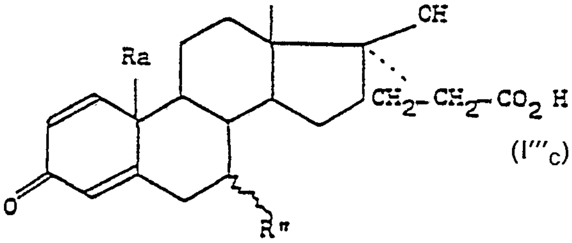

(I'''c)

zu erhalten, worin Ra die vorstehend angegebene Bedeutung besitzt und R'' die Bedeutung von R mit Ausnahme der Bedeutung $(CH_2)_n-R_3$ besitzt, worin $R_3$ einen in ein Salz überführten Carboxyrest darstellt.

15. Als Arzneimittel die Produkte der Formel (I') gemäss Anspruch 1.

16. Als Arzneimittel die Produkte der Formel (I) gemäss Anspruch 2.

17. Als Arzneimittel die Produkte der Formel (I'') gemäss einem der Ansprüche 3, 4, 6, 7, 8 oder 10.

18. Als Arzneimittel die Produkte der Formel (I''') gemäss einem der Ansprüche 5 bis 10.

19. Als Arzneimittel irgendeines der in Anspruch 11 beschriebenen Produkte.

20. Pharmazeutische Zusammensetzungen, enthaltend als Wirkstoff zumindest ein Arzneimittel gemäss einem der Ansprüche 15 bis 19.

**Patentansprüche** für den Vertragsstaat: AT

1. Verfahren zur Herstellung der Produkte der allgemeinen Formel (I'):

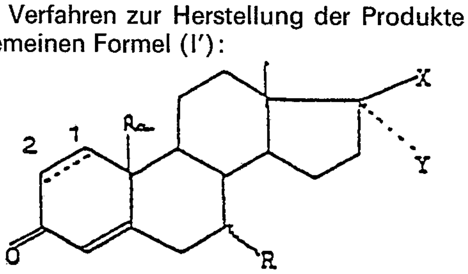

(I')

worin R entweder einen Rest $R_1$, wobei $R_1$ einen Alkyl-, Alkenyl- oder Alkinylrest mit höchstens 8 Kohlenstoffatomen bedeutet, oder einen Rest $R_2$ darstellt, wobei $R_2$ einen Rest $-(CH_2)_nR_3$ bedeutet, worin n eine ganze Zahl von 1 bis 8 ist und $R_3$ einen freien oder geschützten Hydroxylrest, einen Aminorest, einen geschützten Aminorest, einen Mono- oder Dialkylaminorest, einen freien, veresterten oder in ein Salz überführten Carboxyrest oder ein Halogenatom bedeutet, X und Y gemeinsam die Gruppe

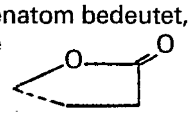

darstellen oder X einen Hydroxylrest bedeutet und Y den Rest $-CH_2-CH_2-CO_2M$ bedeutet, worin M ein Wasserstoffatom, ein Alkalimetallatom oder einen Ammoniumrest darstellt, Ra ein Wasserstoffatom oder einen Methylrest bedeutet, die gestrichelte Linie in 1(2)-Stellung die etwaige Anwesenheit einer zweiten Bindung zwischen den 1 und 2 Kohlenstoffatomen anzeigt und die gewellte Linie anzeigt, dass der Substituent R sich in einer der beiden möglichen α- oder β-Positionen befinden kann, mit der Massgabe, dass R nicht $R_1$ oder $R_2$, worin $n_1$ ist und $R_3$ ein Halogenatom bedeutet, ist, wenn die 1(2)-Bindung eine Einfachbindung ist, und R einen Methylrest bedeutet, dass $R_3$ nicht einen freien Carboxyrest bedeutet, wenn Y den Rest $-CH_2-CH_2-CO_2M$ darstellt, worin M ein Alkalimetallatom oder einen Ammoniumrest bedeutet, und dass $R_3$ nicht einen in ein Salz überführten Carboxyrest bedeutet, wenn Y den Rest $-CH_2-CH_2-CO_2H$ darstellt, dadurch gekennzeichnet, dass man

a) zur Herstellung der Produkte der Formel (I'), wie vorstehend definiert, worin Ra einen Methylrest bedeutet, R für $R_2$ steht und die gestrichelte Linie in 1(2)-Stellung eine Einfachbindung darstellt, ein Produkt der Formel ($II_1$):

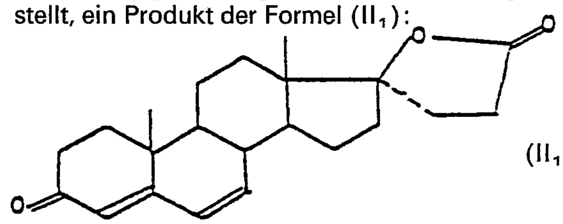

($II_1$)

entweder der Einwirkung eines Produktes der Formel ($III_1$):

$$R_4-(CH_2)_n-MgX' \qquad (III_1)$$

in Gegenwart eines Kupfersalzes unterzieht, wobei in der Formel ($III_1$) X' ein Halogenatom bedeutet, $R_4$ einen geschützten Hydroxylrest, einen Mono- oder Dialkylaminorest oder einen geschützten Aminorest bedeutet und n die vorstehend angegebene Bedeutung besitzt,
oder der Einwirkung eines Produktes der Formel ($III'_1$):

$$[R_4-(CH_2)_n]_2CuLi \qquad (III'_1)$$

unterzieht, worin $R_4$ und n die angegebene Bedeutung besitzen, um nach Einwirkung einer Säure ein Produkt der Formel ($I''_A$):

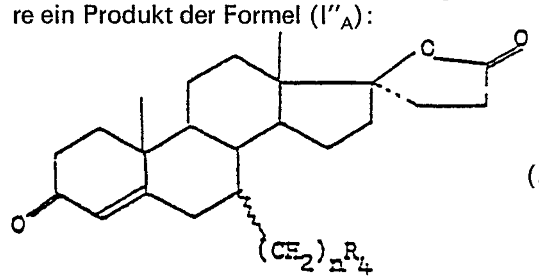

($I''_A$)

gegebenenfalls in Form eines Gemisches des 7α- und 7β-Isomeren zu erhalten, welches Gemisch man gewünschtenfalls auftrennt und gewünschtenfalls ein jedes der Isomeren oder das Gemisch der Produkte, worin $R_4$ einen geschützten Hydroxylrest oder einen geschützten Aminorest bedeutet, der Einwirkung eines Hydrolysemittels unterzieht, um ein Produkt der Formel ($I''_B$):

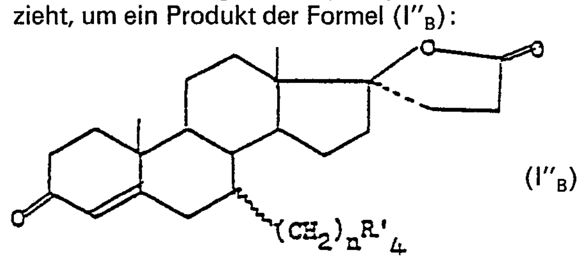

($I''_B$)

worin $R'_4$ einen Hydroxyl- oder Aminorest bedeutet, zu erhalten, und das Produkt der Formel ($I''_B$), worin $R'_4$ einen Hydroxylrest bedeutet, man gegebenenfalls

entweder, wenn n eine ganze Zahl grösser als 1 ist, mit einem Oxidationsreagens behandelt, um ein Produkt der Formel ($I''_C$):

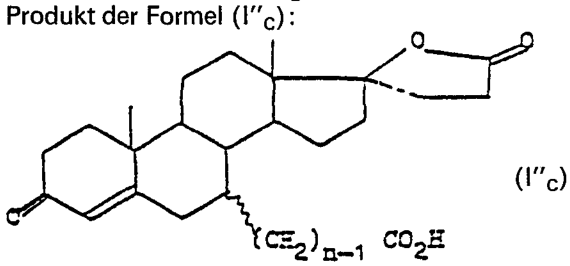

($I''_C$)

zu erhalten, welches Produkt man gegebenenfalls nach üblichen Methoden verestert oder in ein Salz überführt,

oder mit Ausnahme des Falls, bei dem n gleich 1 ist, mit einem Halogenierungsreagens behandelt, um ein Produkt der Formel ($I''_D$):

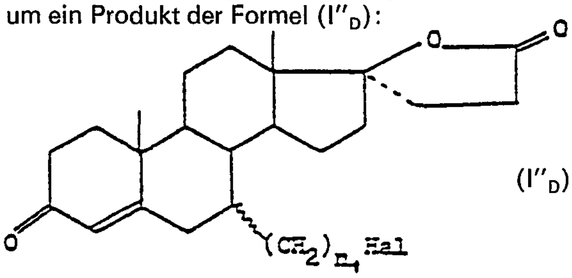

($I''_D$)

worin $n_1$ eine ganze Zahl von 2 bis 8 bedeutet und Hal ein Halogenatom darstellt, zu erhalten, und gewünschtenfalls die Produkte der Formeln ($I''_A$), ($I''_B$), ($I''_C$) und ($I''_D$) in Form des 7α- oder 7β-Isomeren oder von deren Gemisch der Einwirkung eins Alkalihydroxids oder von Ammoniak unterzieht, um die Verbindungen der Formel ($I''_T$):

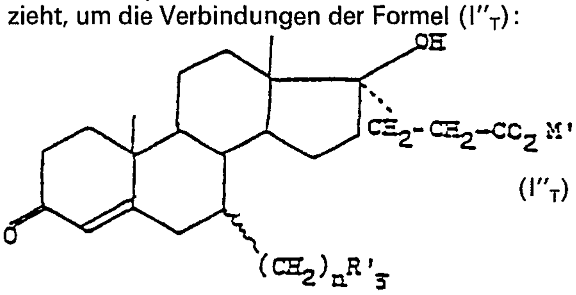

($I''_T$)

worin $R'_3$ die vorstehend für $R_3$ angegebene Bedeutung mit Ausnahme derjenigen von freien Carboxy besitzt und M' ein Alkalimetallatom oder einem Ammoniumrest bedeutet, in Form des 7α- oder 7β-Isomeren oder von deren Gemisch zu erhalten, das man gewünschtenfalls in ein jedes der Isomeren auftrennt, danach gewünschtenfalls ein jedes der Isomeren oder deren Gemisch der Einwirkung eines sauren Mittels unterzieht, um eine Verbindung der Formel ($I''_U$):

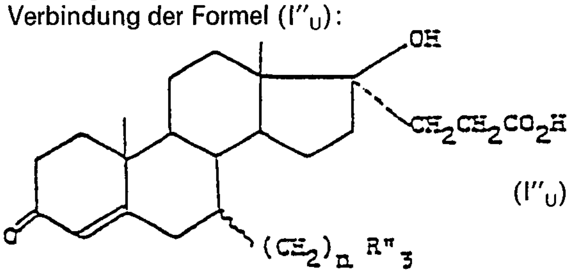

($I''_U$)

worin $R''_3$ die vorstehend für $R_3$ angegebene Bedeutung mit Ausnahme derjenigen von in ein Salz überführtem Carboxy besitzt, zu erhalten;

b) zur Herstellung der Produkte der Formel (I'), wie vorstehend definiert, worin Ra ein Wasserstoffatom bedeutet und die gestrichelte Linie in 1(2)-Stellung eine Einfachbindung anzeigt, ein Produkt der Formel ($II_2$):

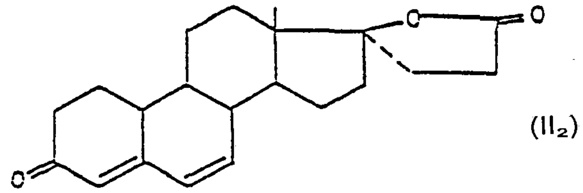

($II_2$)

entweder der Einwirkung eines Produkts der Formel ($III_2$):

$$R_5-MgX' \qquad (III_2)$$

in Gegenwart eines Kupfersalzes unterzieht, wobei in der Formel ($III_2$) $R_5$ für $R_1$, das wie vorstehend definiert ist, oder den Rest $R_4-(CH_2)_n-$, der wie vorstehend definiert ist, steht und X' ein Halogenatom darstellt,

oder der Einwirkung eines Produktes der Formel ($III'_2$):

$$(R_5)_2CuLi \qquad (III'_2)$$

unterzieht, worin $R_5$ wie vorstehend definiert ist, um nach Einwirkung einer Säure ein Produkt der Formel ($I''_{A1}$):

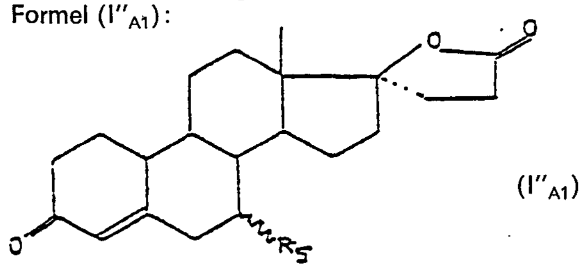

($I''_{A1}$)

gegebenenfalls in Form eines Gemisches des 7α- oder 7β-Isomeren, welches man gewünschtenfalls auftrennt, zu erhalten, und gewünschtenfalls ein jedes der Isomeren oder das Gemisch der Produkte, worin $R_5$ einen Rest $-(CH_2)_n-R_4$ darstellt, worin $R_4$ einen geschützten Hydroxylrest oder einen geschützten Aminorest bedeutet, der Einwirkung eines Hydrolysemittels unterzieht, um ein Produkt der Formel ($I''_{B1}$):

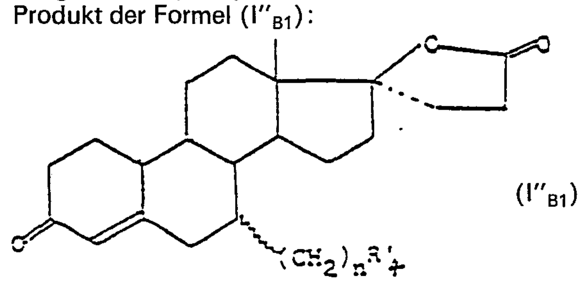

($I''_{B1}$)

worin $R'_4$ einen Hydroxylrest oder einen Aminorest bedeutet, zu erhalten, und das Produkt der Formel ($I''_{B1}$), worin $R'_4$ einen Hydroxylrest darstellt, gegebenenfalls

entweder, wenn n eine ganze Zahl grösser als 1 ist, mit einem Oxidationsreagens behandelt, um ein Produkt der Formel ($I''_{C1}$):

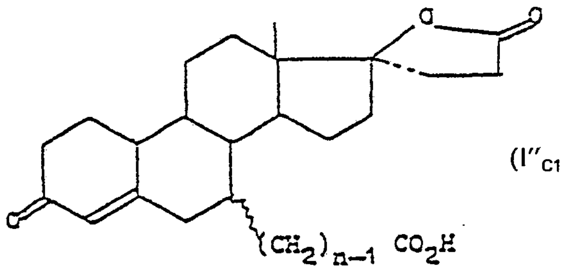

($I''_{C1}$)

zu erhalten, welches Produkt man gegebenenfalls nach üblichen methoden verestert oder in ein Salz überführt,

oder mit einem Halogenierungsreagens behandelt, um ein Produkt der Formel ($I''_{D1}$):

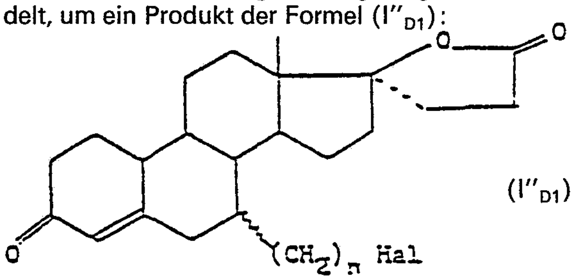

($I''_{D1}$)

worin Hal ein Halogenatom bedeutet, zu erhalten, und gewünschtenfalls die Produkte der Formeln ($I''_{A1}$), ($I''_{B1}$), ($I''_{C1}$) und ($I''_{D1}$) in Form der 7α- oder 7β-Isomeren oder von deren Gemisch der Einwirkung eines Alkalihydroxids oder von Ammoniak unterzieht, um die Verbindungen der Formel ($I''_{T1}$):

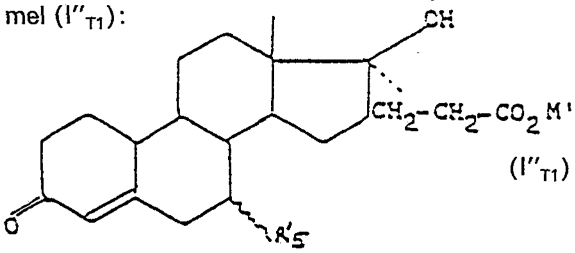

($I''_{T1}$)

worin $R'_5$ für $R_1$, das wie vorstehend definiert ist, oder den Rest $-(CH_2)_n-R'_3$ steht, worin $R'_3$ die vorstehend für $R_3$ angegebene Bedeutung mit Ausnahme derjenigen von freiem Carboxy besitzt, und M' ein Alkalimetallatom oder einen Ammoniumrest bedeutet, in Form des 7α- oder 7β-Isomeren oder von deren Gemisch zu erhalten, das man gewünschtenfalls in ein jedes seiner Isomeren auftrennt, danach gewünschtenfalls ein jedes der Isomeren oder deren Gemisch der Einwirkung eines sauren Mittels unterzieht, um eine Verbindung der Formel ($I''_{U1}$):

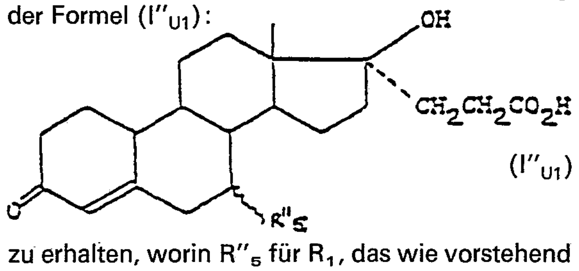

($I''_{U1}$)

zu erhalten, worin $R''_5$ für $R_1$, das wie vorstehend definiert ist, oder den Rest $-(CH_2)_n-R''_3$ steht, worin $R''_3$ die vorstehend für $R_3$ angegebene Bedeutung mit Ausnahme derjenigen von in ein Salz überführtem Carboxy besitzt, und

c) zur Herstellung der Produkte der Formel (I'), wie vorstehend definiert, worin die gestrichelte Linie in 1(2)-Stellung eine Doppelbindung bedeutet, man eine Verbindung der Formel (IV):

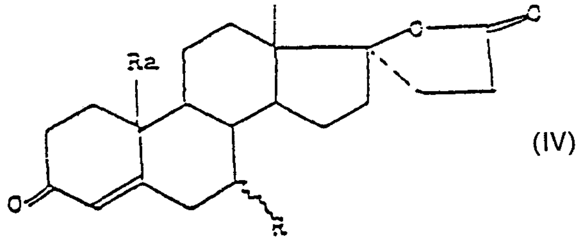

(IV)

worin R entweder einen Rest $R_1$, wobei $R_1$ einen Alkyl-, Alkenyl- oder Alkinylrest mit höchstens 8 Kohlenstoffatomen darstellt, oder einen Rest $R_2$ bedeutet, wobei $R_2$ einen Rest $-(CH_2)_n-R_3$ darstellt, worin n eine ganze Zahl von 1 bis 8 ist und $R_3$ einen freien oder geschützten Hydroxylrest, einen Aminorest, einen geschützten Aminorest, einen Mono- oder Dialkylaminorest, einen freien, in einen Ester überführten oder in ein Salz überführten Carboxyrest oder ein Halogenatom bedeutet und Ra die vorstehend angegebene Bedeutung besitzt, gegebenenfalls in Form eines Gemisches der 7α- und 7β-Isomeren der Einwirkung eines Dehydrogenierungsmittels unterzieht, um ein Produkt der Formel ($I'''_A$):

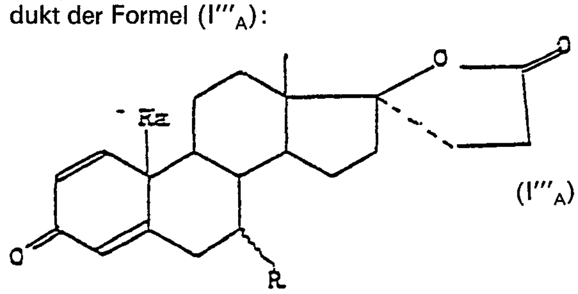

($I'''_A$)

worin Ra und R wie vorstehend definiert sind, gegebenenfalls in Form eines Gemisches der 7α- und 7β-Isomeren zu erhalten, das man gewünschtenfalls auftrennt, danach gewünschtenfalls ein jedes der Isomeren oder deren Gemisch der Einwirkung eines Alkalihydroxids oder von Ammoniak unterzieht, um die Verbindungen der Formel ($I'''_B$):

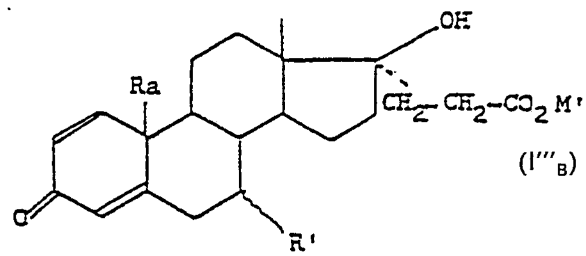

($I'''_B$)

worin Ra die vorstehend angegebene Bedeutung besitzt, M' ein Alkalimetallatom oder die Gruppe $NH_4$ bedeutet und R' die Bedeutung von R mit Ausnahme der Bedeutung $(CH_2)_n-R_3$ besitzt, worin $R_3$ einen freien Carboxyrest bedeutet, das produkt der Formel ($I'''_B$) in Form des 7α- oder 7β-Isomeren oder in Form von deren Gemisch, das man gewünschtenfalls in ein jedes der Isomeren trennt, danach gewünschtenfalls entweder ein jedes der Isomeren oder deren Gemisch der Einwirkung eines sauren Mittels unterzieht, um ein Produkt der Formel ($I'''_C$):

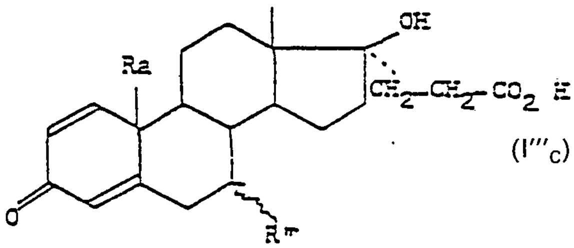

($I'''_C$)

zu erhalten, worin Ra die vorstehend angegebene Bedeutung besitzt und R'' die Bedeutung von R mit Ausnahme der Bedeutung $(CH_2)_n-R_3$ besitzt, worin $R_3$ einen in ein Salz überführten Carboxyrest bedeutet.

2. Verfahren zur Herstellung von Produkten der Formel (I'), wie in Anspruch 1 definiert, entsprechend der Formel (I):

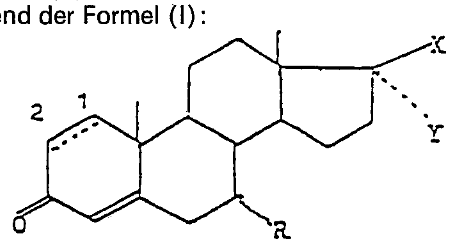

(I)

worin R, X, Y, die gestrichelte Linie und die gewellte Linie die in Anspruch 1 angegebene Bedeutung besitzen, mit der Massgabe, dass R nicht $R_1$ oder $R_2$, worin n für 1 steht und $R_3$ ein Halogenatom bedeutet, ist, wenn die 1(2)-Bindung eine Einfachbindung ist, dass $R_3$ nicht einen freien Carboxyrest bedeuten kann, wenn Y den Rest $-CH_2-CH_2-CO_2M$ darstellt, worin M ein Alkalimetallatom oder einen Ammoniumrest darstellt, und dass $R_3$ nicht einen in ein Salz überführten Carboxyrest bedeuten kann, wenn Y den Rest $-CH_2-CH_2-CO_2H$ darstellt, dadurch gekennzeichnet, dass man

a) zur Herstellung der Produkte der Formel (I), wie vorstehend definiert, worin R für $R_2$ steht und die gestrichelte Linie in 1(2)-Stellung eine Einfachbindung wiedergibt, ein Produkt der Formel ($II_1$):

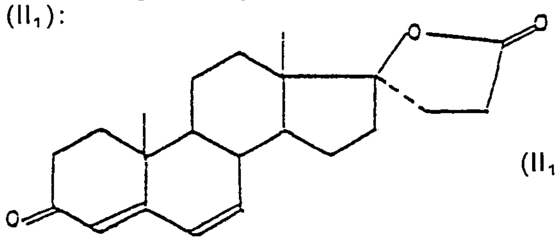

($II_1$)

entweder der Einwirkung eines Produktes der Formel ($III_1$):

$$R_4-(CH_2)_n-MgX' \qquad (III_1)$$

in Gegenwart eines Kupfersalzes unterzieht, wobei in der Formel ($III_1$) X' ein Halogenatom bedeutet, $R_4$ einen geschützten Hydroxylrest, einen Mono- oder Dialkylaminorest oder einen geschützten Aminorest darstellt und n die vorstehend angegebene Bedeutung besitzt,

oder der Einwirkung eines Produktes der Formel ($III'_1$):

$$[R_4-(CH_2)_n]_2CuLi \qquad (III'_1)$$

unterzieht, worin $R_4$ und n die angegebene Bedeutung besitzen, um nach Einwirkung einer Säure ein Produkt der Formel ($I''_A$):

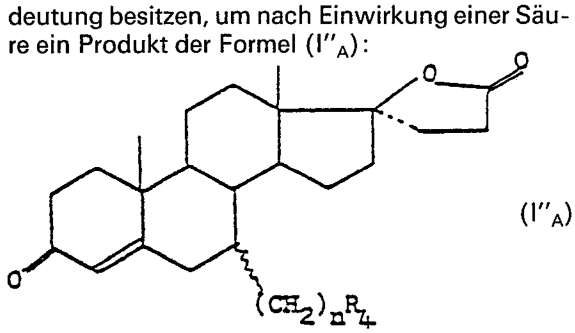

($I''_A$)

gegebenenfalls in Form eines Gemisches der 7α- und 7β-Isomeren zu erhlaten, welches Gemisch man gewünschtenfalls auftrennt und gewünschtenfalls ein jedes der Isomeren oder das Gemisch der Produkte, worin $R_4$ einen geschützten Hydroxylrest oder einen geschützten Aminorest darstellt, der Einwirkung eines Hydrolysemittels unterzieht, um ein Produkt der Formel ($I''_B$):

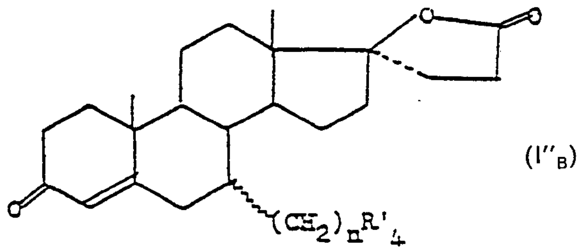

($I''_B$)

worin $R'_4$ einen Hydroxylrest oder einen Aminorest darstellt, zu erhalten, und das Produkt der Formel ($I''_B$), worin $R'_4$ einen Hydroxylrest bedeutet, man gegebenenfalls

entweder, wenn n eine ganze Zahl grösser als 1 ist, mit einem Oxidationsreagens behandelt, um ein Produkt der Formel ($I''_C$):

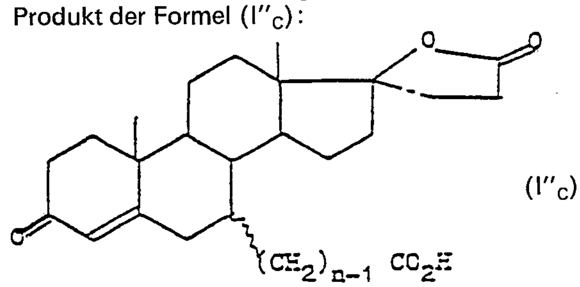

($I''_C$)

zu erhalten, welches Produkt man gewünschtenfalls nach üblichen Methoden verestert oder in ein Salz überführt,

oder mit Ausnahme des Falles, bei dem n gleich 1 ist, mit einem Halogenierungsreagens behandelt, um ein Produkt der Formel ($I''_D$):

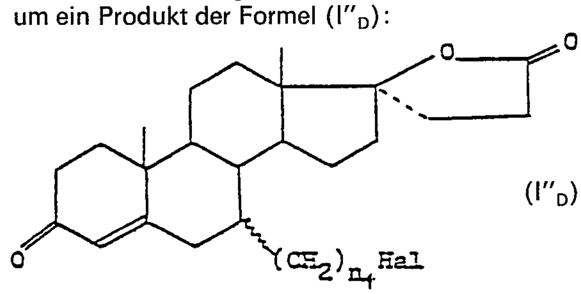

($I''_D$)

worin $n_1$ eine ganze Zahl von 2 bis 8 bedeutet und Hal ein Halogenatom darstellt, zu erhalten, und gewünschtenfalls die Produkte der Formeln ($I''_A$), ($I''_B$), ($I''_C$) und ($I''_D$) in Form der 7α- oder 7β-Isomeren oder von deren Gemisch der Einwirkung eines Alkalihydroxids oder von Ammoniak unterzieht, um die Verbindungen der Formel ($I''_T$):

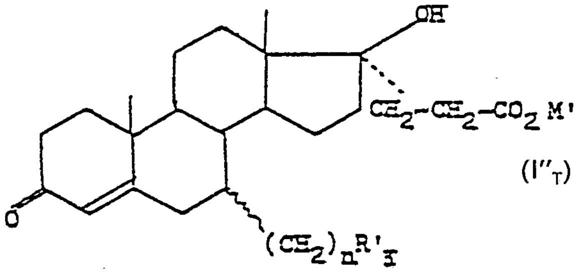

($I''_T$)

worin $R'_3$ die vorstehend angegebene Bedeutung von $R_3$ mit Ausnahme von freiem Carboxy besitzt und M' ein Alkalimetallatom oder einen Ammoniumrest darstellt, in Form der 7α- oder 7β-Isomeren oder von deren Gemisch zu erhalten, das man gewünschtenfalls in ein jedes seiner Isomeren auftrennt, danach gewünschtenfalls ein jedes der Isomeren oder deren Gemisch der Einwirkung eines sauren Mittels unterzieht, um eine Verbindung der Formel ($I''_U$):

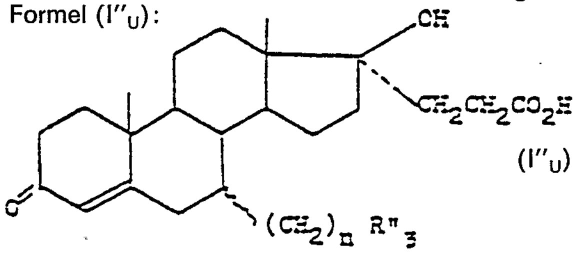

($I''_U$)

zu erhalten, worin $R''_3$ die vorstehend für $R_3$ angegebene Bedeutung mit Ausnahme derjenigen von in ein Salz überführtem Carboxy besitzt, und

b) zur Herstellung der Produkte der Formel (I), wie vorstehend definiert, worin die gestrichelte Linie in 1 (2)-Stellung eine Doppelbindung darstellt, man eine Verbindung der Formel ($IV_1$):

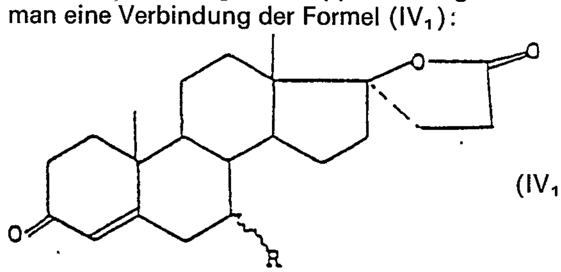

($IV_1$)

worin R die in Anspruch 1 angegebene Bedeutung besitzt, gegebenenfalls in Form eines Gemisches der 7α- und 7β-Isomeren der Einwirkung eines Dehydrogenierungsmittels unterzieht, um ein Produkt der Formel ($I'''_{A1}$):

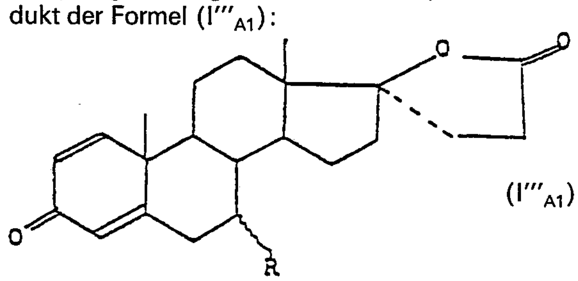

($I'''_{A1}$)

worin R die vorstehend angegebene Bedeutung besitzt, gegebenenfalls in Form eines Gemisches der 7α- und 7β-Isomeren zu erhalten, das man gewünschtenfalls auftrennt, danach ein jedes der Isomeren oder deren Gemisch der Einwirkung eines Alkalihydroxids oder derjenigen von Ammoniak unterzieht, um die Verbindungen der Formel ($I'''_{B1}$):

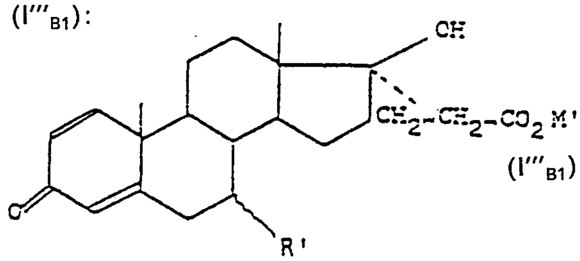

($I'''_{B1}$)

worin M' ein Alkalimetallatom oder die Gruppe $NH_4$ bedeutet und R' die Bedeutung von R mit Ausnahme der Bedeutung $(CH_2)_nR_3$ besitzt, worin $R_3$ einen freien Carboxyrest darstellt, zu erhalten, das Produkt der Formel ($I'''_{B1}$) in Form des 7α- oder 7β-Isomeren oder von deren Gemisch, das man gewünschtenfalls in ein jedes der Isomeren auftrennt, danach entweder ein jedes der Isomeren oder deren Gemisch der Einwirkung eines sauren Mittels unterzieht, um ein Produkt der Formel ($I'''_{C1}$):

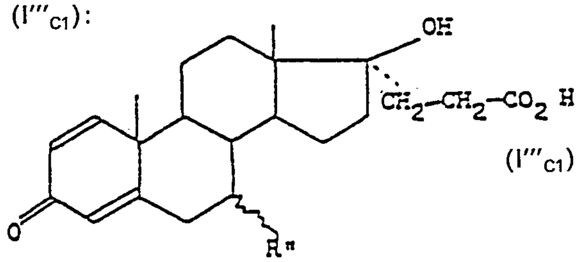

($I'''_{C1}$)

zu erhalten, worin R'' die Bedeutung von R mit Ausnahme der Bedeutung $(CH_2)_n-R_3$ besitzt, worin $R_3$ einen in ein Salz überführten Carboxyrest bedeutet.

3. Verfahren gemäss Anspruch 1, dadurch gekennzeichnet, dass man die Produkte der Formeln ($I''_A$), ($I''_B$), ($I''_C$) und ($I''_D$) oder die Produkte der Formeln ($I''_{A1}$), ($I''_{B1}$), ($I''_{C1}$) und ($I''_{D1}$) oder die Produkte der Formel ($I'''_A$) in Form der 7α- oder 7β-Isomeren oder von deren Gemisch der Einwirkung von Kaliumhydroxid unterzieht, um die entsprechenden Kaliumsalze zu erhalten.

4. Verfahren gemäss Anspruch 1, dadurch gekennzeichnet, dass der Rest R sich in 7α-Stellung befindet.

5. Verfahren gemäss Anspruch 1, dadurch gekennzeichnet, dass man von einem Produkt der Formel ($III_2$), ($III'_2$) oder (IV) ausgeht, worin $R_5$ oder R einen Propyl-, Butyl-, Isobutyl- oder Butenylrest bedeutet, mit der Massgabe, dass Ra nicht einen Methylrest bedeuten kann, wenn die 1(2)-Bindung eine Einfachbindung darstellt.

6. Verfahren gemäss einem der Ansprüche 3, 4 oder 5, dadurch gekennzeichnet, dass man von einem Produkt der Formel (II) oder (IV) ausgeht, das in 10-Stellung mit einem Methylrest substituiert ist.

7. Verfahren gemäss Anspruch 2, dadurch gekennzeichnet, dass man das γ-Lacton der 17β-Hydroxy-7α-propyl-3-oxo-17α-pregna-1,4,-dien-21-carbonsäure und das Kalium-17β-hydroxy-3-oxo-7α-propyl-17α-pregna-1,4-dien-21-carboxylat herstellt.

**Claims** for the Contracting States BE, CH, DE, FR, GB, IT, LI, LU, NL, SE

1. The products with the general Formula (I')

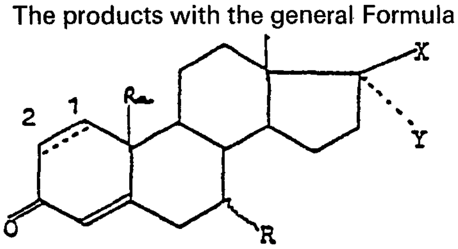

(I')

in which R represents either a radical $R_1$, $R_1$ being an alkyl, alkenyl or alkynyl radical with at most 8 carbon atoms, or a radical $R_2$, $R_2$ being a radical $-(CH_2)_nR_3$ in which n represents an integer 1 to 8 and $R_3$ represents a free or protected hydroxyl radical, an amino, protected amino, mono- or dialkylamino radical, a free, esterified or salified carboxy radical or a halogen atom, X and Y together represent the

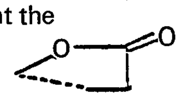

group, or X represents a hydroxyl radical and Y represents the radical $-CH_2-CH_2-CO_2M$ in which M represents a hydrogen atom, an alkali metal atom or an ammonium radical, Ra represents a hydrogen atom or a methyl radical, the dotted line in position 1(2) indicates the possible presence of a second bond between the carbons 1 and 2 and the wavy line signifies that the substituent R may be found in one of the two possible positions α or β, it being understood that R may not represent $R_1$ or $R_2$ in which n is 1 and $R_3$ represents a halogen atom when the bond 1 (2) is a simple bond and when Ra represents a methyl radical, that $R_3$ may not represent a free carboxy radical when Y represents the radical $-CH_2-CH_2-CO_2M$ in which M represents an alkali metal atom or an ammonium radical and that $R_3$ may not represent a salified carboxy radical when Y represents the radical $-CH_2-CH_2-CO_2H$.

2. The products with the general Formula (I') as defined in Claim 1, corresponding to the general Formula (I):

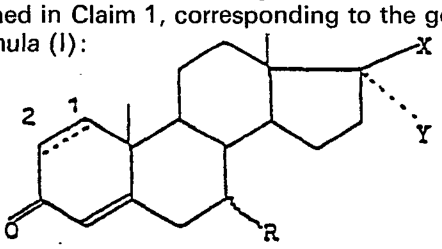

(I)

in which R, X, Y, the dotted line and the wavy line have the significance indicated in Claim 1, it being understood that R may not represent $R_1$ or $R_2$ in which n is 1 and $R_3$ represents a halogen atom, when the bond 1 (2) is a simple bond, that $R_3$ may not represent a free carboxy radical when Y represents the radical $-CH_2-CH_2-CO_2M$ in which M represents an alkali metal atom or an ammonium radical and that $R_3$ may not represent a salified carboxy radical when Y represents the radical $-CH_2-CH_2-CO_2M$.

3. The products with the Formula (I'') corresponding to the products with the Formula (I') as defined in Claim 1, in which Ra represents a methyl radical, R represents $R_2$ and the dotted line in position 1(2) represents a simple bond, it being understood that n may not represent the integer 1 when $R_3$ represents a halogen atom.

4. The products with the Formula (I'') corresponding to the products with the Formula (I') as defined in Claim 1, in which Ra represents a hydrogen atom, R has the significance indicated in Claim 1 and the dotted line in position 1(2) represents a simple bond.

5. The products with the Formula (I''') corresponding to products with the Formula (I') as defined in Claim 1, in which the dotted line in position 1(2) indicates the presence of a double bond.

6. The products with the Formula (I') as defined in any one of Claims 1 to 5 in which X and Y together represent the group

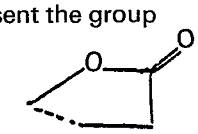

7. The products with the Formula (I') as defined in any one of Claims 1 to 5 in which X represents a hydroxyl radical and Y represents a radical $-CH_2-CH_2-CO_2K$.

8. The products with the Formula (I') as defined in any one of Claims 1 to 7 in which the radical R is in position 7α.

9. The products with the Formula (I') as defined in any one of Claims 1, 2 and 4 to 8, in which R represents a propyl, butyl, isobutyl or butenyl radical, it being understood that Ra does not represent a methyl radical when the bond 1 (2) is a simple bond.

10. The products with the Formula (I') as defined in any one of Claims 1 to 8, in which R represents a chloropropyl, hydroxypropyl, carboxyethyl, methoxycarbonylethyl radical.

11. The γ-lactone of 17β-hydroxy-7α-propyl-3-oxo-17α-pregna-1,4-diene-21-carboxylic acid;
17β-hydroxy-3-oxo-7α-propyl-17α-pregna-1,4-diene-21-carboxylate of potassium.

12. Preparation process for the products with the Formula (I') as defined in Claim ,1, 2 or 3, in which Ra represents a methyl radical, characterized in that a product with the Formula ($II_1$):

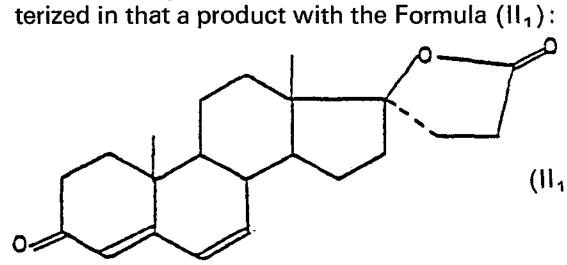

($II_1$)

is submitted
either to the action of a product with the Formula ($III_1$):

$$R_4-(CH_2)_n-MgX' \qquad (III_1)$$

in the presence of a copper salt, in which Formula ($III_1$) X' represents a halogen atom, $R_4$ represents a protected hydroxyl radical, a mono- or dialkylamino or a protected amino radical and n has the significance indicated in Claim 1,
or to the action of a product with the Formula ($III'_1$):

$$[R_4-(CH_2)_n]_2CuLi \qquad (III'_1)$$

in which $R_4$ and n retain the same significance, so as to obtain, after action of an acid, a product with the Formula ($I''_A$):

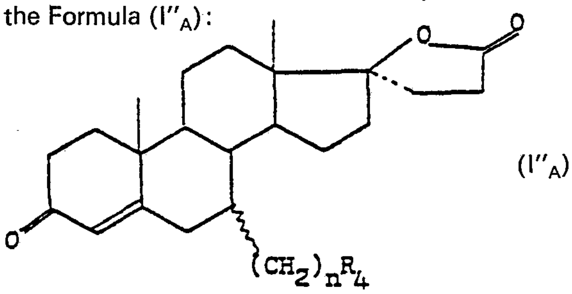

($I''_A$)

possibly in the form of a mixture of 7α and 7β isomers, which mixture, if desired, is separated and, if desired, each isomer or the mixture of the products in which $R_4$ represents a protected hydroxyl radical or a protected amino radical is submitted to the action of a hydrolyzing agent, so as to obtain a product with the Formula ($I''_B$):

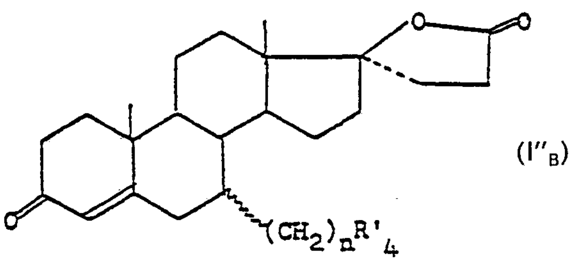

($I''_B$)

in which $R'_4$ represents a hydroxyl or amino radical, and the product with the Formula ($I''_B$) in which $R'_4$ represents a hydroxyl radical is treated, if applicable,
either, when n is an integer greater than 1, with an oxidation reagent, so as to obtain a product with the Formula ($I''_C$):

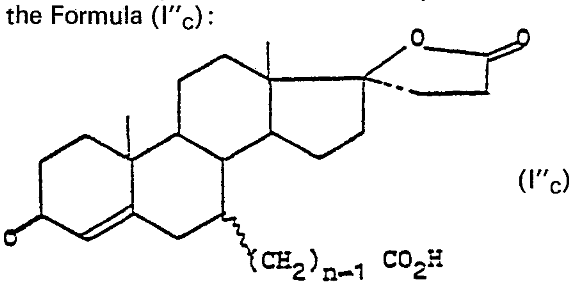

($I''_C$)

which product, if applicable, is esterified or salified according to the usual methods,
or, with the exception of when n is 1, with a halogenation reagent, so as to obtain a product with the Formula ($I''_D$):

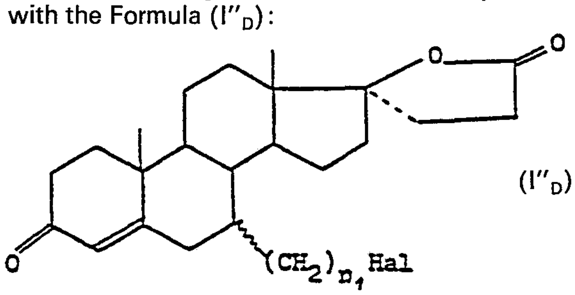

($I''_D$)

in which $n_1$ represents an integer 2 to 8 and Hal represents a halogen atom and, if desired, the products with the Formulae ($I''_A$), ($I''_B$), ($''_C$) and ($I''_D$) in the form of 7α or 7β isomers or their mixture are submitted to the action of an alkaline hydroxide or ammonia, so as to obtain the compounds with the Formula ($I''_T$):

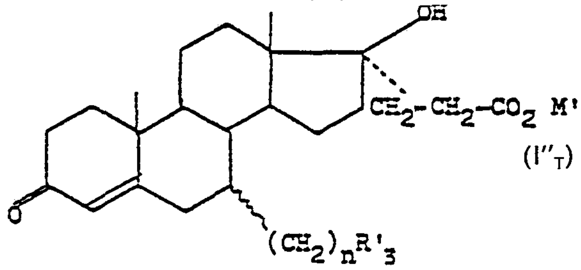

($I''_T$)

in which $R'_3$ has the significance of $R_3$ indicated in Claim 1, with the exception of the free carboxy value and M' represents an alkali metal atom or an ammonium radical, in the form of 7α or 7β isomers or their mixture, which, if desired, is separated into each of its isomers, then if desired, either each of the isomers, or their mixture is submitted to the action of an acid agent, so as to obtain a compound with the Formula ($I''_U$):

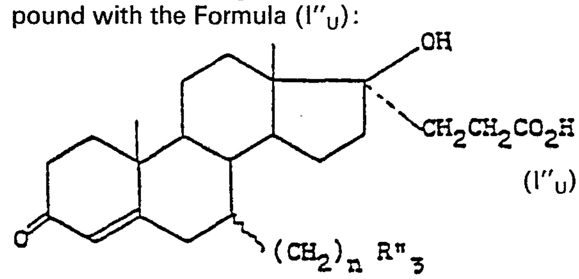

($I''_U$)

in which $R''_3$ has the significance of $R_3$ indicated in Claim 1, with the exception of the salified carboxy value.

13. Preparation process for the products with the Formula (I') as defined in Claim 1 or 4, in which Ra represents a hydrogen atom, characterized in that a product with the Formula ($II_2$):

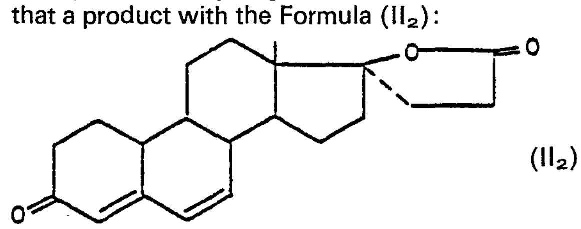

($II_2$)

is submitted
either to the action of a product with the Formula ($III_2$):

$$R_5-MgX' \qquad (III_2)$$

in the presence of a copper salt, in which Formula ($III_2$) $R_5$ represents $R_1$ as defined in Claim 1 or the radical $R_4-(CH_2)_n-$ defined as in Claim 12 and X' represents a halogen atom,
or to the action of a product with the Formula ($III'_2$):

$$(R_5)_2CuLi \qquad (III'_2)$$

in which $R_5$ is defined as above, so as to obtain, after the action of an acid, a product with the Formula ($I''_{A1}$):

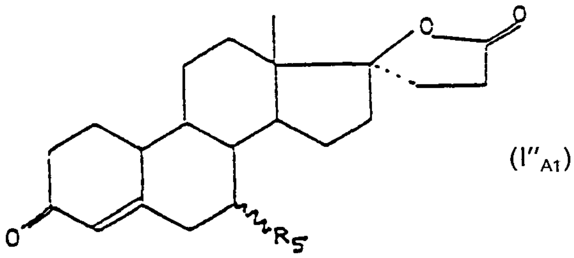

(I"$_{A1}$)

possibly in the form of a mixture of 7α and 7β isomers, which mixture, if desired, is separated and, if desired, each isomer, or the mixture of the products in which $R_5$ represents a radical $-(CH_2)_n-R_4$, in which $R_4$ represents a protected hydroxyl radical or a protected amino radical, is submitted to the action of a hydrolyzing agent, so as to obtain a product with the Formula (I"$_{B1}$):

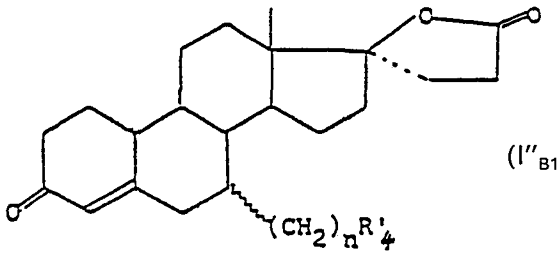

(I"$_{B1}$)

in which $R'_4$ represents a hydroxyl or amino radical and the product with the Formula (I"$_{B1}$) in which $R'_4$ represents a hydroxyl radical is treated, if applicable,
either, when n is an integer greater than 1, with an oxidation reagent, so as to obtain a product with the Formula (I"$_{C1}$):

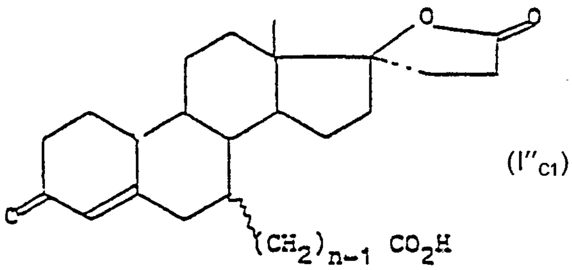

(I"$_{C1}$)

which product, if applicable, is salified or esterified according to the usual methods,
or with a halogenation reagent, so as to obtain a product with the Formula (I"$_{D1}$):

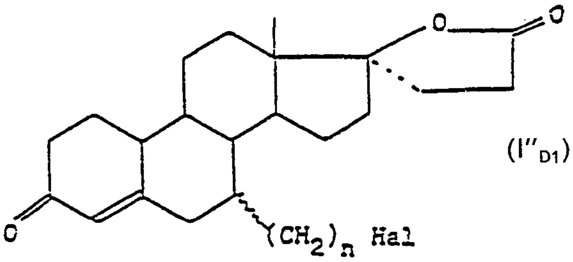

(I"$_{D1}$)

in which Hal represents a halogen atom and, if desired, the products with the Formulae (I"$_{A1}$), (I"$_{B1}$), (I"$_{C1}$) and (I"$_{D1}$) in the form of 7α or 7β isomers or of their mixture are submitted to the action of an alkaline hydroxide or ammonia, so as to obtain compounds with the Formula (I"$_{T1}$):

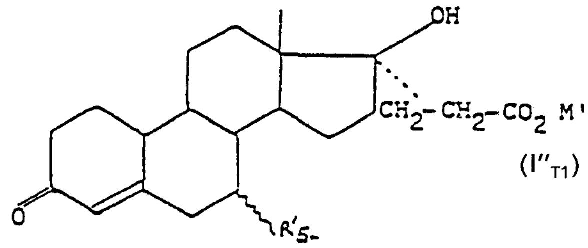

(I"$_{T1}$)

in which $R'_5$ represents $R_1$ which is defined as above, or the radical $-(CH_2)_n-R'_3$, in which $R'_3$ has the significance of $R_3$ indicated above in Claim 1, with the exception of the free carboxy value and M' represents an alkali metal atom or an ammonium radical, in the form of 7α or 7β isomers or of their mixture, which, if desired, is separated into each of its isomers, then, if desired, each of the isomers, or their mixture, is submitted to the action of an acid agent, so as to obtain a compound with the Formula (I"$_{U1}$):

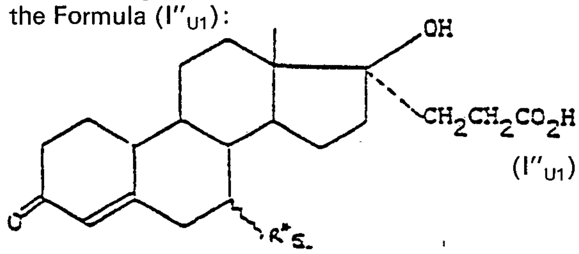

(I"$_{U1}$)

in which $R''_5$ represents $R_1$ which is defined as above, or the radical $-(CH_2)_nR''_3$, in which $R''_3$ has the significance indicated in Claim 1, with the exception of the salified carboxy value.

14. Preparation process for the products with the Formula (I') as defined in Claim 1 or 5, characterized in that a compound with the Formula (IV):

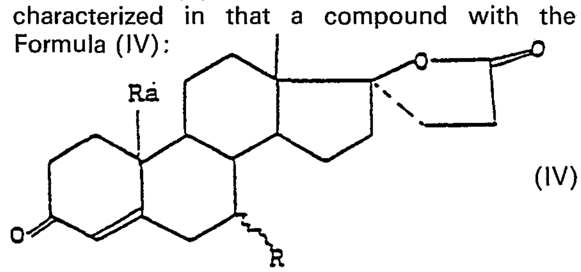

(IV)

in which R represents either a radical $R_1$, $R_1$ being an alkyl, alkenyl or alkynyl radical with at most 8 carbon atoms, or a radical $R_2$, $R_2$ being a radical $-(CH_2)_n-R_3$ in which n represents an integer 1 to 8 and $R_3$ represents a free or protected hydroxyl radical, an amino, protected amino, mono- or dialkylamino radical, a carboxy radical, free, esterified or salified or a halogen atom and Ra has the significance indicated in Claim 1, possibly in the form of a mixture or 7α and 7β isomers, is submitted to the action of a dehydrogenation agent, so as to obtain a product with the Formula (I'''$_A$):

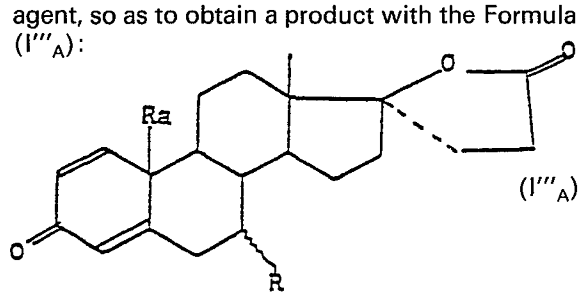

(I'''$_A$)

in which Ra and R have the preceding significance, possibly in the form of a mixture of 7α and 7β isomers, which are separated, if desired, then, if desired, each of the isomers, or their mixture is submitted to the action of an alkaline hydroxide or ammonia, so as to obtain the compounds with the Formula ($I'''_B$):

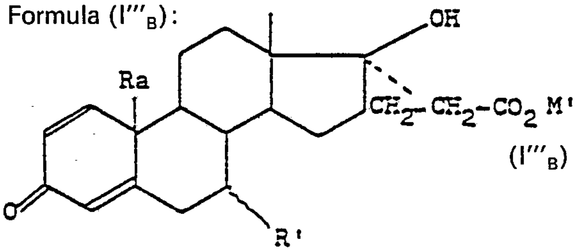

($I'''_B$)

in which Ra has the preceding significance, M' represents an alkali metal atom or the group $NH_4$ and R' has the significance of R with the exception of the $(CH_2)_n-R_3$ value in which $R_3$ represents a free carboxy radical, which product with the Formula ($I'''_B$) in the form of 7α or 7β isomers or their mixture is separated, if desired, into each of the isomers then, if desired, each of the isomers or their mixture is submitted to the action of an acid agent, so as to obtain a product with the Formula ($I'''_C$):

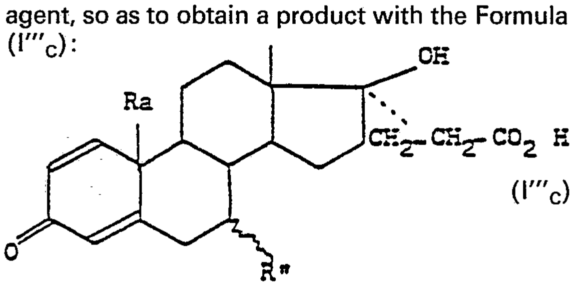

($I'''_C$)

in which Ra has the preceding significance and R" has the significance of R with the exception of the $(CH_2)_n-R_3$ value in which $R_3$ represents a salified carboxy radical.

15. As medicaments, the products with the Formula (I') as defined in Claim 1.

16. As medicaments, the products with the Formula (I) as defined in Claim 2.

17. As medicaments, the products with the Formula (I") according to any one of Claims 3, 4, 6, 7, 8 or 10.

18. As medicaments, the products with the Formula (I''') according to any one of Claims 5 to 10.

19. As medicaments, any one of the products described in Claim 11.

20. Pharmaceutical compositions containing as active principle at least one medicament defined in any one of Claims 15 to 19.

**Claims** for the Contracting State: AT

1. Preparation process for the products with the general Formula (I'):

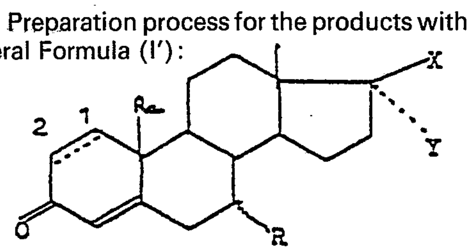

(I')

in which R represents either a radical $R_1$, $R_1$ being an alkyl, alkenyl or alkynyl radical with at most 8 carbon atoms, or a radical $R_2$, $R_2$ being a radical $-(CH_2)_nR_3$ in which n represents an integer 1 to 8 and $R_3$ represents a free or protected hydroxyl radical, an amino, protected amino, mono- or dialkylamino radical, a free, esterified or salified carboxy radical or a halogen atom, X and Y together represent the

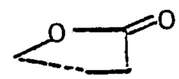

group, or X represents a hydroxyl radical and Y represents the radical $-CH_2-CH_2-CO_2M$ in which M represents a hydrogen atom, an alkali metal atom or an ammonium radical, Ra represents a hydrogen atom or a methyl radical, the dotted line in position 1(2) indicates the possible presence of a second bond between the carbons 1 and 2 and the wavy line signifies that the substituent R may be found in one of the two possible positions α or β, it being understood that R may not represent $R_1$ or $R_2$ in which n is 1 and $R_3$ represents a halogen atom, when the bond 1(2) is a simple bond and when Ra represents a methyl radical, that $R_3$ may not represent a free carboxy radical when Y represents the radical $-CH_2-CH_2-CO_2M$ in which M represents an alkali metal atom or an ammonium radical and that $R_3$ may not represent a salified carboxy radical when Y represents the radical $-CH_2-CH_2-CO_2H$, characterized in that:

(*a*) for the preparation of the products with the Formula (I'), as defined above, in which Ra represents a methyl radical, R represents $R_2$ and the dotted line in position 1(2) represents a simple bond, a product with the Formula ($II_1$):

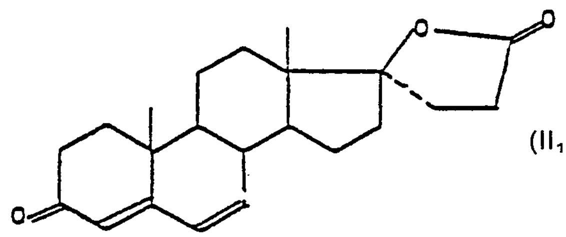

($II_1$)

is submitted either to the action of a product with the Formula ($III_1$):

$$R_4-(CH_2)_n-MgX' \qquad (III_1)$$

in the presence of a copper salt, in which Formula ($III_1$) X' represents a halogen atom, $R_4$ represents a protected hydroxyl radical, a mono- or dialkylamino radical or a protected amino radical and n has the significance previously indicated,

or to the action of a product with the Formula ($III'_1$):

$$[R_4-(CH_2)_n]_2CuLi \qquad (III'_1)$$

in which $R_4$ and n retain the same significance, so as to obtain, after action of an acid, a product with the Formula ($I''_A$);

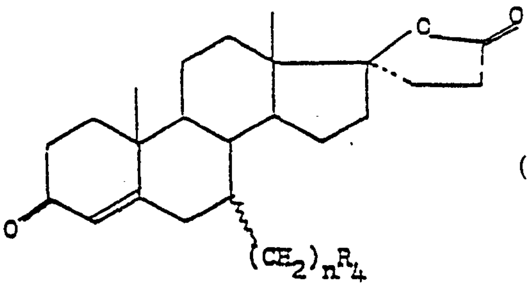

(I''$_A$)

possibly in the form of a mixture of 7α and 7β isomers, which mixture, if desired, is separated, and if desired each isomer or the mixture of the products in which $R_4$ represents a protected hydroxyl radical or a protected amino radical, is submitted to the action of a hydrolyzing agent, so as to obtain a product with the Formula (I''$_B$):

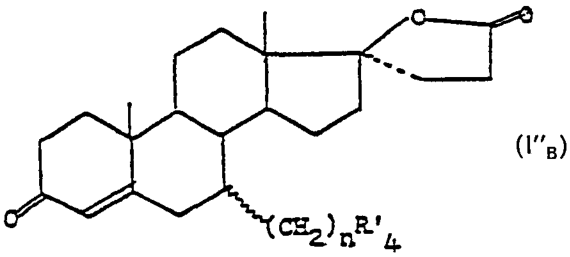

(I''$_B$)

in which $R'_4$ represents a hydroxyl or amino radical, and the product with the Formula (I''$_B$) in which $R'_4$ represents a hydroxyl radical is treated, if applicable,
either, when n is an integer greater than 1 with an oxidation reagent, so as to obtain a product with the Formula (I''$_C$):

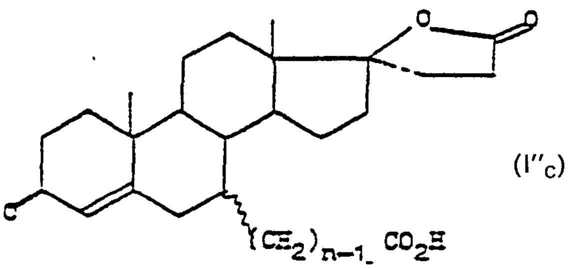

(I''$_C$)

which product, if applicable, is esterified or salified according to the usual methods,
or, with the exception of when n is 1, with a halogenation reagent, so as to obtain a product with the Formula (I''$_D$):

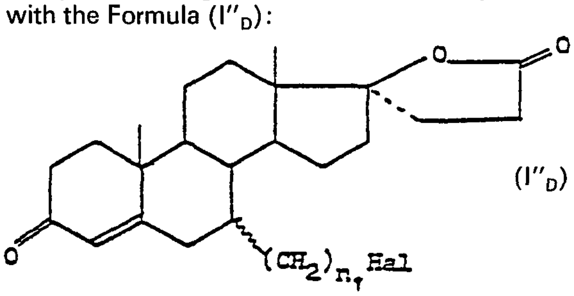

(I''$_D$)

in which $n_1$ represents an integer 2 to 8 and Hal represents a halogen atom and, if desired, the products with the Formulae (I''$_A$), (I''$_B$), (I''$_C$) and (I''$_D$) in the form of 7α or 7β isomers or their mixture are submitted to the action of an alkaline hydroxide or ammonia, so as to obtain the compounds with the Formula (I''$_T$):

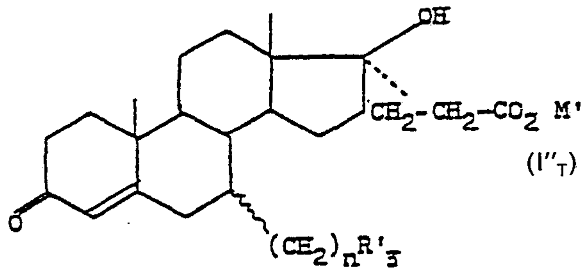

(I''$_T$)

in which $R'_3$ has the significance of $R_3$ indicated previously with the exception of the free carboxy value and M' represents an alkali metal atom or an ammonium radical, in the form of 7α or 7β isomers or of their mixture, which, if desired, is separated into its isomers, then if desired, each of the isomers or their mixture is submitted to the action of an acid agent, so as to obtain a compound with the Formula (I''$_U$):

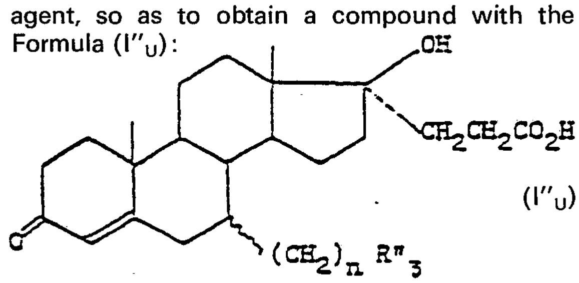

(I''$_U$)

in which $R''_3$ has the significance of $R_3$ indicated previously, with the exception of the salified carboxy value:

(*b*) for the preparation of the products with the Formula (I'), as defined above, in which Ra represents a hydrogen atom and the dotted line in position 1(2) represents a simple bond, a product with the Formula ($II_2$):

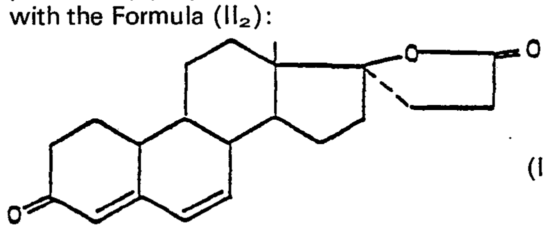

($II_2$)

is submitted
either to the action of a product with the Formula ($III_2$):

$$R_5-MgX' \qquad (III_2)$$

in the presence of a copper salt, in which Formula ($III_2$) $R_5$ represents $R_1$ which is defined as previously or the radical $R_4-(CH_2)_n-$ defined as previously and X' represents a halogen atom,
or to the action of a product with the Formula ($III'_2$):

$$(R_5)_2CuLi \qquad (III'_2)$$

in which $R_5$ is defined as above, so as to obtain, after the action of an acid, a product with the Formula (I''$_{A1}$):

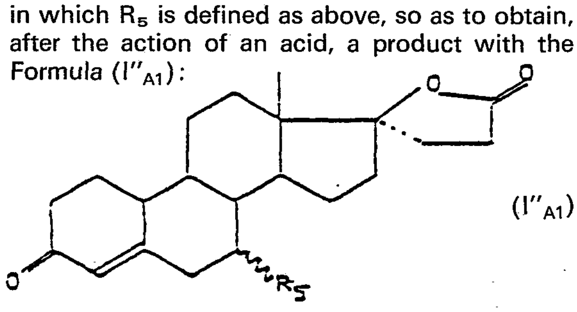

(I''$_{A1}$)

possibly in the form of a mixture of 7α and 7β isomers, which mixture, if desired, is separated and, if desired, each isomer or the mixture of the products in which $R_5$ represents a radical $-(CH_2)_n-R_4$, in which $R_4$ represents a protected hydroxyl radical or a protected amino radical, is submitted to the action of a hydrolizing agent, so as to obtain a product with the Formula ($I''_{B1}$):

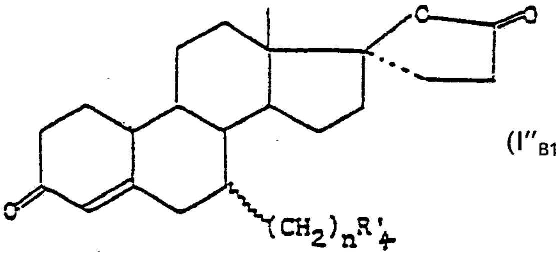

($I''_{B1}$)

in which $R'_4$ represents a hydroxyl or amino radical, and the product with the Formula ($I''_{B1}$) in which $R'_4$ represents a hydroxyl radical is treated, if applicable,
either, when n is an integer greater than 1, with an oxidation reagent, so as to form a product with the Formula ($I''_{C1}$):

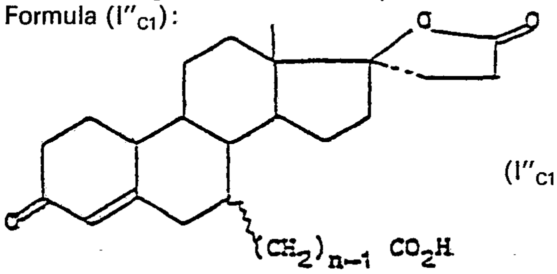

($I''_{C1}$)

which product, if applicable, is esterified or salified according to the usual methods,
or with a halogenation reagent, so as to obtain a product with the Formula ($I''_{D1}$):

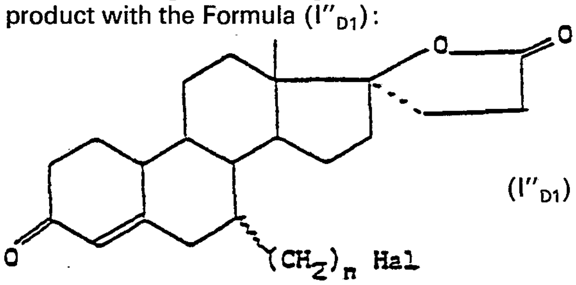

($I''_{D1}$)

in which Hal represents a halogen atom and, if desired, the products with the Formulae ($I''_{A1}$), ($I''_{B1}$), ($I''_{C1}$) and ($I''_{D1}$), in the form of 7α or 7β isomers or of their mixture, are submitted to the action of an alkaline hydroxide or ammonia, so as to obtain the compounds with the Formula ($I''_{T1}$):

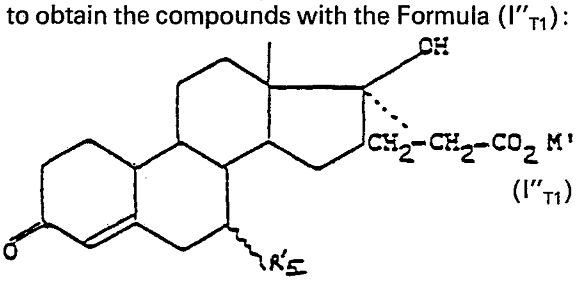

($I''_{T1}$)

in which $R'_5$ represents $R_1$ which is defined as above, or the radical $-(CH_2)_n-R'_3$, in which $R'_3$ has the significance of $R_3$ indicated previously, with the exception of the free carboxy value, and M′ represents an alkali metal atom or an ammonium radical, in the form of 7α or 7β isomers or of their mixture, which is separated, if desired, into each of its isomers, then, if desired, each of the isomers or their mixture is submitted to the action of an acid agent, so as to obtain a compound with the Formula ($I''_{U1}$):

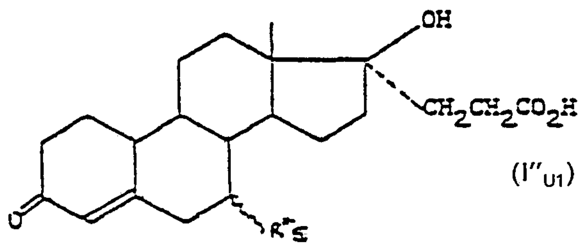

($I''_{U1}$)

in which $R''_5$ represents $R_1$ which is defined as above, or the radical $-(CH_2)_n-R''_3$, in which $R''_3$ has the significance of $R_3$ indicated previously with the exception of the salified carboxy value, and

(*c*) for the preparation of the products with the Formula (I′), as defined above, in which the dotted line in position 1(2) represents a double bond, a compound with the Formula (IV):

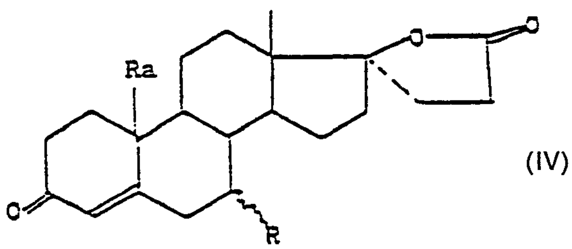

(IV)

in which R represents either a radical $R_1$, $R_1$ being an alkyl, alkenyl or alkynyl radical with at most 8 carbon atoms, or a radical $R_2$, $R_2$ being a radical $-(CH_2)_n-R_3$ in which n represents an integer 1 to 8 and $R_3$ represents a free or protected hydroxyl radical, an amino, protected amino, mono- or dialkylamino radical, a free, esterified or salified carboxy radical or a halogen atom and Ra has the significance indicated previously, possibly in the form of a mixture of 7α and 7β isomers, is submitted to the action of a dehydrogenation agent, so as to obtain a product with the Formula ($I'''_A$):

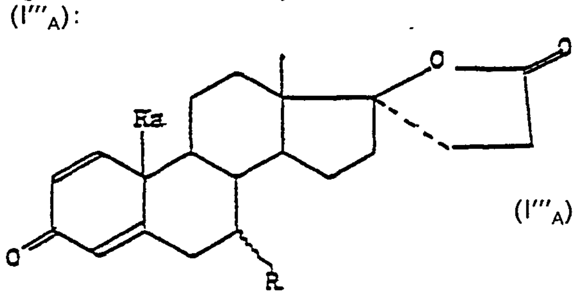

($I'''_A$)

in which Ra and R have the preceding significance, possibly in the form of a mixture of 7α and 7β isomers, which if desired is separated then, if desired, each of the isomers, or their mixture is submitted to the action of an alkaline hydroxide or ammonia, so as to obtain the compounds with the Formula ($I'''_B$):

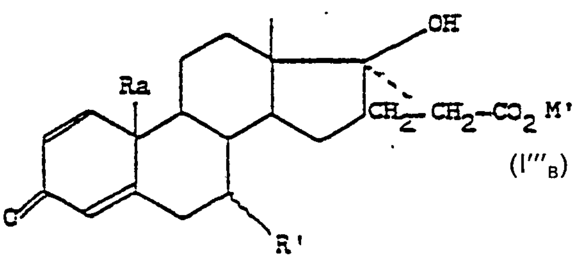

($I'''_B$)

in which Ra has the preceding significance, M' represents an alkali metal atom or the group $NH_4$ and R' has the significance of R with the exception of the $(CH_2)_n-R_3$ value in which $R_3$ represents a free carboxy radical, which product with the Formula ($I'''_B$) in the form of 7α or 7β isomers or their mixture is separated, if desired, into each of the isomers, then, if desired, each of the isomers or their mixture is submitted to the action of an acid agent, so as to obtain a product with the Formula ($I'''_C$):

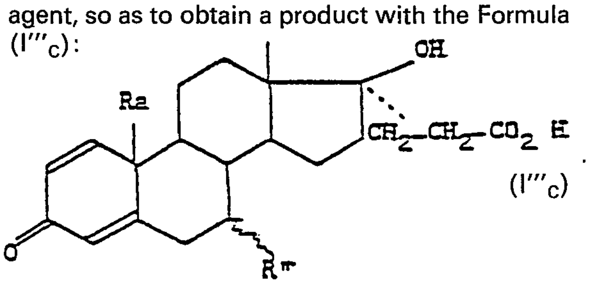

($I'''_C$)

in which Ra has the preceding significance and R" has the significance of R with the exception of the $(CH_2)_n-R_3$ value in which $R_3$ represents a salified carboxy radical.

2. Process for the preparation of products with the Formula (I') as defined in Claim 1, corresponding to Formula (I):

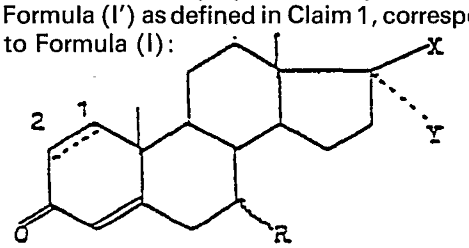

(I)

in which R, X, Y, the dotted line and the wavy line have the significance indicated in Claim 1, it being understood that R may not represent $R_1$ or $R_2$ in which n is 1 and $R_3$ represents a halogen atom, when the bond 1 (2) is a simple bond, that $R_3$ may not represent a free carboxy radical when Y represents the radical $-CH_2-CH_2-CO_2M$ in which M represents an alkali metal atom or an ammonium radical and that $R_3$ may not represent a salified carboxy radical when Y represents the radical $-CH_2-CH_2-CO_2H$, characterized in that:

(*a*) for the preparation of the product with the Formula (I), as defined above, in which R represents $R_2$ and the dotted line in position 1 (2) represents a simple bond, a product with the Formula ($II_1$):

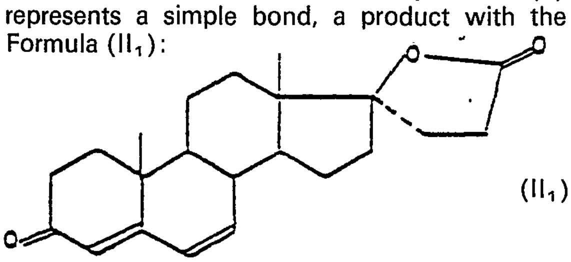

($II_1$)

is submitted,

either to the action of a product with the Formula ($III_1$):

$$R_4-(CH_2)_n-MgX' \qquad (III_1)$$

in the presence of a copper salt, in which Formula ($III_1$) X' represents a halogen atom, $R_4$ represents a protected hydroxyl radical, a mono- or dialkylamino radical or a protected amino radical and n has the significance indicated previously,

or to the action of a product with the Formula ($III'_1$):

$$[R_4-(CH_2)_n]_2CuLi \qquad (III'_1)$$

in which $R_4$ and n retain the same significance, so as to obtain, after action of an acid, a product with the Formula ($I''_A$):

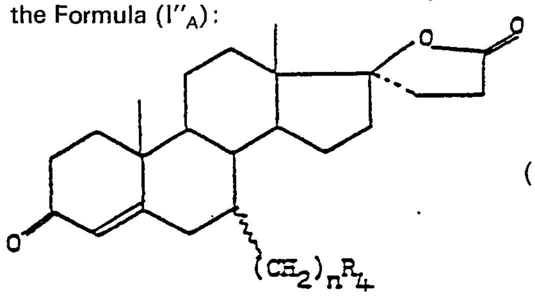

($I''_A$)

possibly in the form of a mixture of 7α and 7β isomers, which mixture, if desired, is separated, and, if desired, each isomer or the mixture of the products in which $R_4$ represents a protected hydroxyl radical or a protected amino radical, is submitted to the action of a hydrolyzing agent, so as to obtain a product with the Formula ($I''_B$):

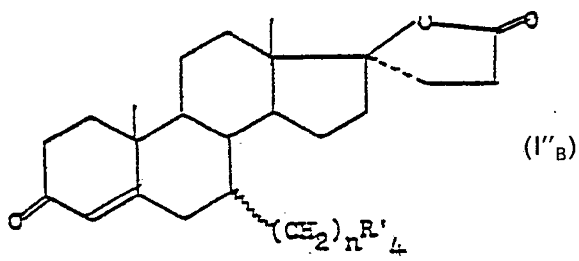

($I''_B$)

in which $R'_4$ represents a hydroxyl or amino radical, and the product with the Formula ($I''_B$) in which $R'_4$ represents a hydroxyl radical is treated, if applicable,

either, when n is an integer greater than 1, with an oxidation agent, so as to obtain a product with the Formula ($I''_C$):

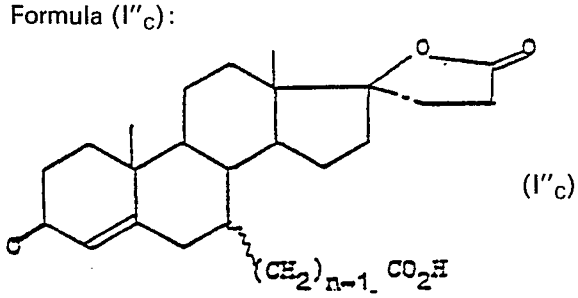

($I''_C$)

which product, if applicable, is esterified or salified according to the usual methods,

or, except when n is 1, with a halogenation agent, so as to obtain a product with the Formula ($I''_D$):

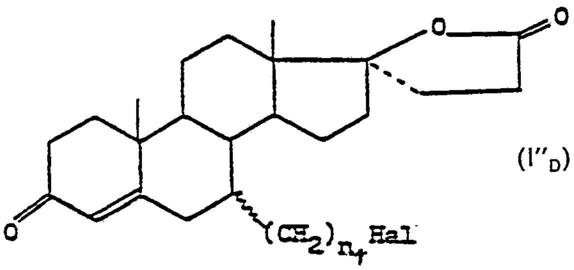

($I''_D$)

in which $n_1$ represents an integer 2 to 8 and Hal represents a halogen atom, and, if desired, the products with the Formulae ($I''_A$), ($I''_B$), ($I''_C$) and ($I''_D$), in the form of 7α or 7β isomers or of their mixture, are submitted to the action of an alkaline hydroxide or ammonia, so as to obtain the compounds with the Formula ($I''_T$):

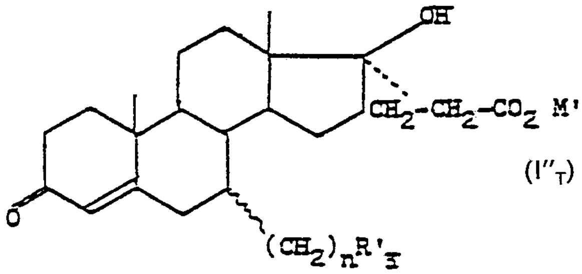

($I''_T$)

in which $R'_3$ has the significance of $R_3$ previously indicated with the exception of the free carboxy value and M' represents an alkali metal atom or an ammonium radical, in the form of 7α or 7β isomers or of their mixture, which, if desired, is separated into each of its isomers, then, if desired, each of the isomers or their mixture is submitted to the action of an acid agent, so as to obtain a compound with the Formula ($I''_U$):

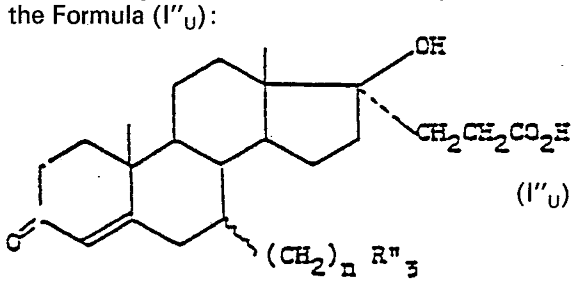

($I''_U$)

in which $R''_3$ has the significance of $R_3$ previously indicated, with the exception of the salified carboxy value, and

(*b*) for the preparation of the products with the Formula (I), as defined above, in which the dotted line in position 1(2) represents a double bond, a compound with the Formula ($IV_1$):

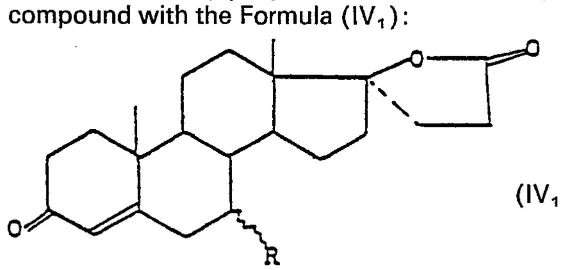

($IV_1$)

in which R has the significance indicated in Claim 1, possibly in the form of a mixture of 7α and 7β isomers, is submitted to the action of a dehydrogenation agent, so as to obtain a product with the Formula ($I'''_{A1}$):

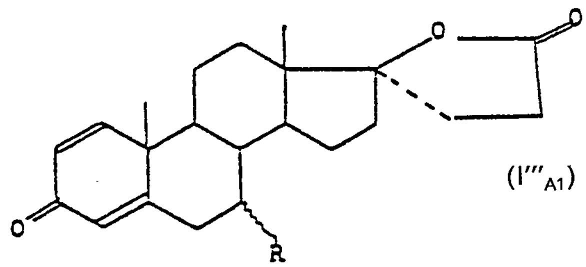

($I'''_{A1}$)

in which R has the preceding significance, possibly in the form of a mixture of 7α and 7β isomers, which, if desired, is separated, then each of the isomers or their mixture is submitted to the action of an alkaline hydroxide or ammonia, so as to obtain the compounds with the Formula ($I'''_{B1}$):

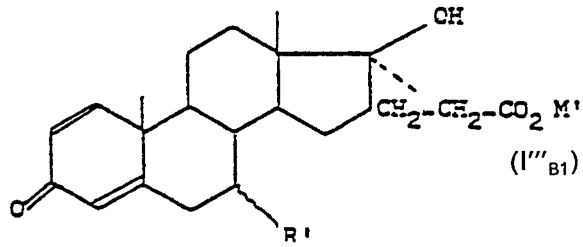

($I'''_{B1}$)

in which M' represents an alkali metal atom or the group $NH_4$ and R' has the significance of R with the exception of the $(CH_2)_nR_3$ value in which $R_3$ represents a free carboxy radical, which product with the Formula ($I'''_{B1}$), in the form of 7α or 7β isomers or of their mixture, is separated, if desired, into each of the isomers, then each of the isomers or their mixture is submitted to the action of an acid agent, so as to obtain a product with the Formula ($I'''_{C1}$):

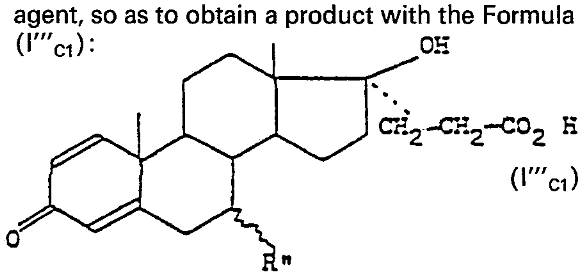

($I'''_{C1}$)

in which R'' has the significance of R with the exception of the $(CH_2)_n-R_3$ value in which $R_3$ represents a salified carboxy radical.

3. Process according to Claim 1, characterized in that the products with the Formulae ($I''_A$), ($I''_B$), ($I''_C$) and ($I''_D$) or the products with the Formulae ($I''_{A1}$), ($I''_{B1}$), ($I''_{C1}$) and ($I''_{D1}$) or the products with the Formula ($I'''_A$) in the form of 7α or 7β isomers or of their mixture are submitted to the action of potassium hydroxide so as to obtain the corresponding salts of potassium.

4. Process according to Claim 1, characterized in that the radical R is in position 7α.

5. Process according to Claim 1, characterized in that a product with the Formula ($III_2$), ($III'_2$) or (IV) is used at the start, in which $R_5$ or R represents a propyl, butyl, isobutyl or butenyl radical, it being understood that Ra does not represent a methyl radical when the bond 1(2) is a simple bond.

6. Process according to Claim 3, 4 or 5, characterized in that a product with the Formula (II) or (IV) substituted in position 10 by a methyl radical is used at the start.

7. Process according to Claim 2, characterized in that:

– γ-lactone of 17β-hydroxy-7α-propyl-3-oxo-17α-pregna-1,4-dien-21-carboxylic acid, and
– 17β-hydroxy-3-oxo-7α-propyl-17α-pregna-1,4-dien-21-carboxylate of potassium
are prepared.